# EUROPEAN PATENT APPLICATION

(11) **EP 3 587 405 A1**
(43) Date of publication of application: **01.01.2020**
(21) Application number: 18756738.3
(22) Date of filing: 26.02.2018
(51) Int. Cl.: C07D 277/36, A01N 43/78, A01P 13/00

(54) **THIAZOLE COMPOUNDS AND HERBICIDE**

(30) Priority: 26.02.2017 JP 2017034231
(71) Applicant: OAT Agrio Co., Ltd., Tokyo 101-0052 (JP)
(72) Inventor: FUKUNAGA Satoshi, Naruto-shi Tokushima 772-0021 (JP); SUMITOMO Tatsuya, Naruto-shi Tokushima 772-0021 (JP); SUMIYOSHI Hayato, Naruto-shi Tokushima 772-0021 (JP); NOYAMA Shinpei, Naruto-shi Tokushima 772-0021 (JP); SHIRAI Yuichi, Naruto-shi Tokushima 772-0021 (JP); NORIMURA Yusuke, Naruto-shi Tokushima 772-0021 (JP); MATSUZAKI Kohei, Naruto-shi Tokushima 772-0021 (JP)
(74) Representative: SSM Sandmair
(86) International application number: PCT/JP2018/006836
(87) International publication number: WO 2018/155661

(57) **Abstract**

An object of the present invention is to provide a thiazole compound and a herbicide containing the thiazole compound.

The present invention relates to a thiazole compound represented by general formula (1): 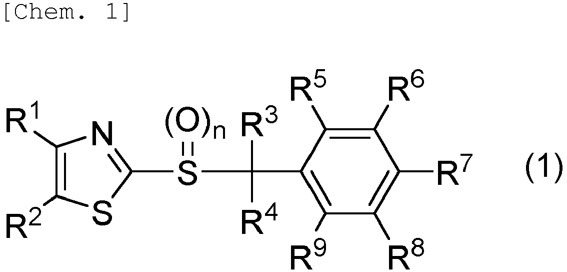 wherein R¹ and R² are identical or different and represent a hydrogen atom, a halogen atom, or an alkyl group which may have a substituent,
R³ and R⁴ are identical or different and represent, for example, a hydrogen atom, a deuterium atom, or a halogen atom,
R⁵, R⁶, R⁷, R⁸, and R⁹ are identical or different and represent, for example, a hydrogen atom, a halogen atom, or a hydroxyl group,
at least one of R⁵, R⁶, R⁷, R⁸ or R⁹ represents a hydroxyl group, an OCOR¹² group, a COR¹³ group, or an OSO₂R¹⁴ group,
R¹⁰, R¹¹, R¹², R¹³, and R¹⁴ represent a hydrogen atom, an alkyl group which may have a substituent, or an aryl group which may have a substituent, and
n represents 0, 1, or 2.

## Description

### BACKGROUND

The present invention relates to a thiazole compound and a herbicide containing the thiazole compound.

A large number of compounds having a thiazole ring have been known. For example, Japanese Unexamined Patent Publication No. 2003-096059 discloses a thiazole compound represented by Formula (A) shown below, and a herbicide composition including the thiazole compound as an active ingredient. 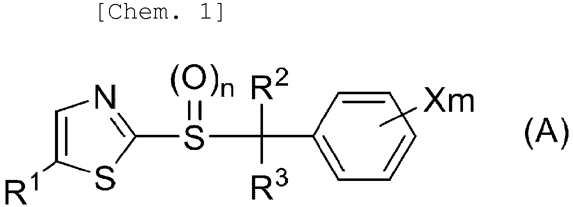 However, while disclosing the thiazole compound including, on a benzene ring, the X group which is a halogen atom, a C₁-C₄ alkyl group, a C₁-C₄ haloalkyl group, a C₁-C₄ alkoxycarbonyl group, or a nitro group, Japanese Unexamined Patent Publication No. 2003-096059 does not at all describe any thiazole compound of which the X group is a group such as a hydroxyl group, an OCOR¹² group, a COR¹³ group, a silyl group, a silyloxy group, a cyano group, an amino group, an alkoxy group, an aryloxy group, a substituted sulfide group, a substituted sulfinyl group, a substituted sulfonyl group, or an OSO₂R¹⁴ group.

### SUMMARY

It is an object of the present invention to provide a low-dose compound which has a wide weed control spectrum for, in particular, paddy field weeds, and which is sufficiently herbicidally active against general annual weeds, such as Echinochloa sp. and Monochoria vaginalis at advanced leaf age, and general perennial weeds such as Schoenoplectus sp. (Hotarui). The present invention also aims to provide a herbicide including the thiazole compound as an active ingredient.

As a result of intensive studies to achieve the above object, the present inventors have made the following findings: a thiazole compound shown below, which has a substituent such as an alkoxy group on a benzene ring (hereinafter also referred to as "the thiazole compound") provides, in a low dose, a wide weed control spectrum. The present invention was made based on these findings.

Specifically, the present invention relates to a thiazole compound shown below and a herbicide containing the thiazole compound.

### Item 1:

A thiazole compound represented by general formula (1): 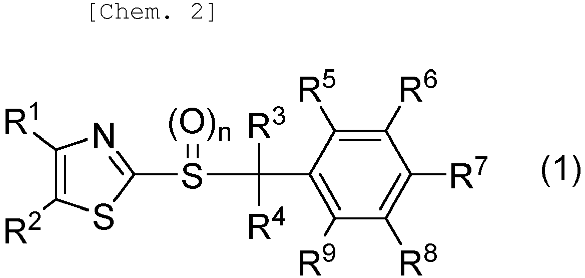 or a salt thereof,
wherein R¹ and R² are identical or different and each represent a hydrogen atom, a halogen atom, or an alkyl group which may have a substituent,
R³ and R⁴ are identical or different and each represent a hydrogen atom, a deuterium atom, a halogen atom, an alkyl group which may have a substituent, a COR¹⁰ group, or a COOR¹¹ group,
R⁵, R⁶, R⁷, R⁸ and R⁹ are identical or different and each represent a hydrogen atom, a halogen atom, a hydroxyl group, an OCOR¹² group, a COR¹³ group, a silyl group which may have a substituent, a silyloxy group which may have a substituent, a nitro group, a cyano group, an amino group which may have a substituent, an alkyl group which may have a substituent, an alkoxy group which may have a substituent, an aryloxy group which may have a substituent, a substituted sulfide group, a substituted sulfinyl group, a substituted sulfonyl group, or an OSO₂R¹⁴ group,
at least one of R⁵, R⁶, R⁷, R⁸ or R⁹ represents a hydroxyl group, an OCOR¹² group, a COR¹³ group, a silyl group which may have a substituent, a silyloxy group which may have a substituent, a cyano group, an amino group which may have a substituent, an alkoxy group which may have a substituent, an aryloxy group which may have a substituent, a substituted sulfide group, a substituted sulfinyl group, a substituted sulfonyl group, or an OSO₂R¹⁴ group,
R¹⁰, R¹¹, R¹², R¹³, and R¹⁴ are identical or different and each represent a hydrogen atom, an alkyl group which may have a substituent, or an aryl group which may have a substituent, and
n represents 0, 1, or 2.

### Item 2:

The thiazole compound or a salt thereof according to Item 1, wherein R¹ is a hydrogen atom, a halogen atom, or an unsubstituted alkyl group.

### Item 3:

The thiazole compound or a salt thereof according to any one of the preceding items, wherein the R² is a halogen atom or a haloalkyl group.

### Item 4:

The thiazole compound or a salt thereof according to any one of the preceding items, wherein R³ and R⁴ are identical or different and are a hydrogen atom, a deuterium atom, a halogen atom, an unsubstituted alkyl group, a COCH₃ group, or a COOCH₃ group.

### Item 5:

The thiazole compound or a salt thereof according to any one of the preceding items, wherein out of R⁵, R⁶, R⁷, R⁸ and R⁹, at least one of R⁵ or R⁹ is a hydroxyl group, an OCOR¹² group, a silyl group which may have a substituent, a silyloxy group which may have a substituent, a cyano group, an amino group which may have a substituent, an alkoxy group which may have a substituent, an aryloxy group which may have a substituent, a substituted sulfonyl group, or an OSO₂R¹⁴ group.

### Item 6:

The thiazole compound or a salt thereof according to any one of the preceding items, wherein at least one of R⁵ or R⁹ is an unsubstituted alkoxy group or a haloalkoxy group.

### Item 7:

The thiazole compound or a salt thereof according to any one of the preceding items, wherein R⁷ is a hydrogen atom, an unsubstituted alkoxy group, or a haloalkoxy group.

### Item 8:

The thiazole compound or a salt thereof according to any one of the preceding items, wherein R⁷ is an unsubstituted alkoxy group or a haloalkoxy group.

### Item 9:

The thiazole compound or a salt thereof according to any one of the preceding items, wherein R⁷ is a hydrogen atom.

### Item 10:

A herbicide containing the thiazole compound or a salt thereof according to any one of Items 1 to 9.

### Item 11:

A paddy field herbicide containing the thiazole compound or a salt thereof according to any one of Items 1 to 9.

### Item 12:

A herbicide containing the thiazole compound or a salt thereof according to any one of Items 1 to 9, and usable for Monochoria vaginalis, Schoenoplectus sp. (Hotarui), Echinochloa sp., and/or Digitaria ciliaris.

The present invention provides a herbicide composition having a high herbicidal effect for general paddy field weeds and upland field weeds.

Non-limiting examples of the paddy field weeds against which the herbicide composition of the present invention is highly herbicidally effective include: annual broad-leaved weeds such as Echinochloa sp. and Monochoria vaginalis; and Schoenoplectus sp. (Hotarui). Non-limiting examples of the upland field weeds against which the herbicide composition of the present invention is highly herbicidally effective include Digitaria ciliaris.

The present invention makes it possible to obtain a low-dose compound which has a wide weed control spectrum for, in particular, paddy field weeds, and which is sufficiently herbicidally active against not only general annual weeds such as Echinochloa sp. and Monochoria vaginalis at advanced leaf age, but also general perennial weeds such as Schoenoplectus sp. (Hotarui). According to the present invention, a herbicide including the thiazole compound as an active ingredient can also obtained.

### BRIEF DESCRIPTION OF THE DRAWINGS

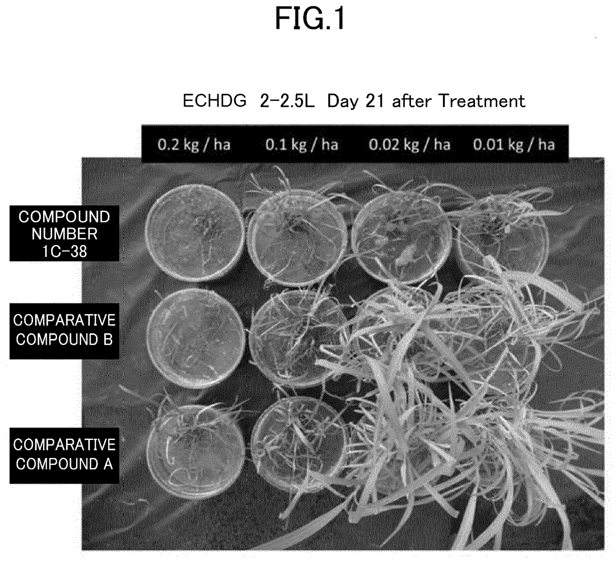

FIG. 1 is a photograph showing the states of plants of Echinochloa cus-galli (L.) P. Beauv. var. formosensis Ohwi (ECHDG) at a leaf stage from 2 to 2.5 which were respectively treated with a compound (1C-38) of an example of the present invention, a comparative compound A, and a comparative compound B, on day 21 after the treatment.

### DETAILED DESCRIPTION

A thiazole compound of the present invention and a herbicide containing the thiazole compound will be described below in detail.

### 1. Thiazole Compound

A thiazole compound or a salt thereof represented by the general formula (1) above (hereinafter also referred to also as "the thiazole compound (1)," "the thiazole compound of the present invention," or "the compound of the present invention") encompasses a sulfide compound (1A) shown below and corresponding to the general formula (1) wherein n is 0, a sulfoxide compound (1B) shown below and corresponding to the general formula (1) wherein n is 1, and a sulfone compound (1C) shown below and corresponding to the general formula (1) wherein n is 2. 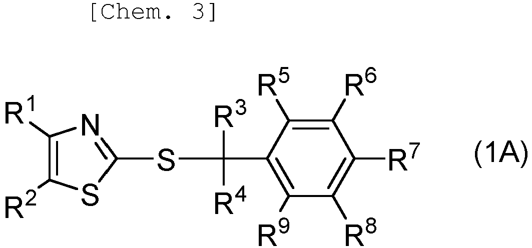 wherein R¹, R², R³, R⁴, R⁵, R⁶, R⁷, R⁸, and R⁹ are the same as those described above. 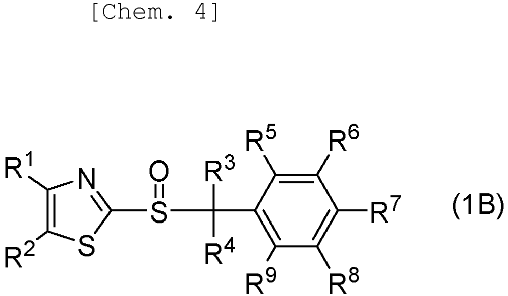 wherein R¹, R², R³, R⁴, R⁵, R⁶, R⁷, R⁸, and R⁹ are the same as those described above. 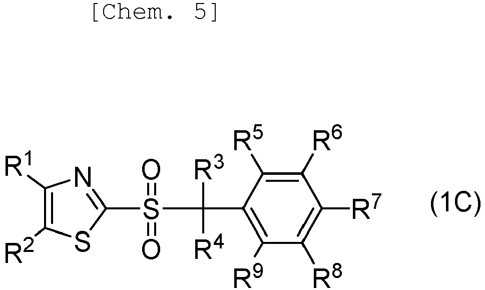 wherein R¹, R², R³, R⁴, R⁵, R⁶, R⁷, R⁸, and R⁹ are the same as those described above.

The sulfoxide compound (1B) includes a sulfoxide compound represented by the general formula (1B-S) shown below, or a sulfoxide compound represented by the general formula (1B-R) shown below. 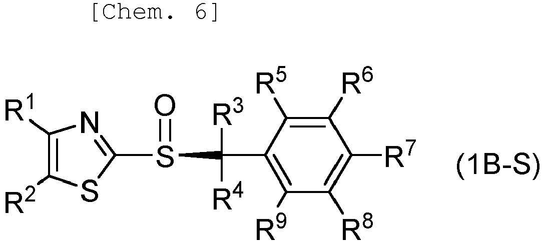 wherein R¹, R², R³, R⁴, R⁵, R⁶, R⁷, R⁸, and R⁹ are the same as those described above. 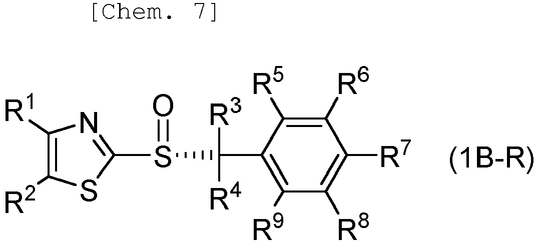 wherein R¹, R², R³, R⁴, R⁵, R⁶, R⁷, R⁸, and R⁹ are the same as those described above.

Specific examples of each of the groups described herein are as follows.

Examples of "halogen atoms" include a fluorine atom, a chlorine atom, a bromine atom, and an iodine atom.

Examples of "alkyl group" include, but are not limited to, straight-chain or branched-chain C₁-C₄ alkyl groups such as a methyl group, an ethyl group, an n-propyl group, an isopropyl group, an n-butyl group, a sec-butyl group, and a tert-butyl group. In this specification, "n-" means normal, "sec-" means secondary, and "t-" means tertiary.

Examples of "alkoxy group" include, but are not limited to, straight-chain or branched-chain C₁-C₄ alkoxy groups such as a methoxy group, an ethoxy group, an n-propoxy group, an isopropoxy group, an n-butoxy group, an isobutoxy group, a sec-butoxy group, and a tert-butoxy group.

Examples of "aryl group" include, but are not limited to, C₆-C₁₄ aryl groups such as a phenyl group, a naphthyl group, and an anthryl group.

Examples of "substituent" or "substitution" for a silyl group which may have a substituent(s), a silyloxy group which may have a substituent (s), an alkyl group which may have a substituent(s), an amino group which may have a substituent(s), an alkoxy group which may have a substituent(s), an aryloxy group which may have a substituent(s), a substituted sulfide group, a substituted sulfinyl group, and a substituted sulfonyl group include, but are not limited to, a halogen atom, an alkyl group, an alkoxy group, a hydroxyl group, a nitro group, a cyano group, and an alkoxycarbonyl group. If the group has the substituent(s), the number of the substituents is not particularly limited, and is usually from 1 to 10, preferably from 1 to 5, and more preferably from 1 to 4.

Examples of "silyl group which may have a substituent(s)" include, but are not limited to, substituted or unsubstituted silyl groups having a carbon number ranging from 0 to 20, such as a trimethylsilyl group, a triethylsilyl group, a triisopropylsilyl group, a dimethylisopropylsilyl group, a diethylisopropylsilyl group, a dimethylhexylsilyl group, a t-butyldimethylsilyl group, a t-butyldiphenylsilyl group, a diphenylmethylsilyl group, a tribenzylsilyl group, tri-p-xylylsilyl group, a triphenylsilyl group, and a t-butylmethoxyphenylsilyl group. Preferable examples thereof include a trimethylsilyl group and a t-butyldimethylsilyl group.

Examples of "silyloxy group which may have a substituent(s)" include, but are not limited to, substituted or unsubstituted silyloxy groups having a carbon number ranging from 0 to 20, such as a silyloxy group, a trimethylsilyloxy group, a t-butyldimethylsilyloxy group, and a diphenylmethylsilyloxy group.

Examples of "alkyl group which may have a substituent(s)" include, but are not limited to: an unsubstituted alkyl group; and a substituted alkyl group such as a haloalkyl group and an alkoxyalkyl group.

Example of "haloalkyl group" include, but are not limited to, C₁-C₄ haloalkyl groups such as a fluoromethyl group, a chloromethyl group, a bromomethyl group, an iodomethyl group, a difluoromethyl group, a trifluoromethyl group, a 2-fluoroethyl group, a 2-chloroethyl group, a 1-fluoroethyl group, a 1-fluoropropyl group, a 2-chloropropyl group, a 3-fluoropropyl group, a 3-chloropropyl group, a 1-fluorobutyl group, a 1-chlorobutyl group, and 4-fluorobutyl group. The number of the halogen atoms is usually from 1 to 5, preferably from 1 to 4, and more preferably from 1 to 3.

Examples of "alkoxyalkyl group" include, but are not limited to, C₁-C₄ alkoxy C₁-C₄ alkyl groups such as a 2-methoxyethyl group, a 2-ethoxyethyl group, a 3-methoxypropyl group, a 3-ethoxypropyl group, a 4-methoxybutyl group, and a 4-ethoxybutyl group. The alkoxyalkoxy group may have a substituent(s) such as a halogen atom.

Examples of "amino group which may have a substituent(s)" include, but are not limited to, an unsubstituted amino group (NH₂), a monoalkylamino group, and a dialkylamino group.

Examples of "monoalkylamino group" include, but are not limited to, mono C₁-C₄ alkylamino groups such as a methylamino group, an ethylamino group, a propylamino group, and an isopropylamino group.

Examples of "dialkylamino group" include, but are not limited to, di C₁-C₄ alkylamino groups such as a dimethylamino group, a diethylamino group, an ethylmethylamino group, a dipropylamino group, and a diisopropylamino group.

Examples of "alkoxy group which may have a substituent(s)" include, but are not limited to, an unsubstituted alkoxy group; and a substituted alkoxy group such as a haloalkoxy group, an alkoxyalkoxy group, an arylalkoxy group, a carboxyalkoxy group, and an alkoxycarbonylalkoxy group.

Examples of "haloalkoxy group" include, but are not limited to, groups in which the alkoxy group is substituted with a halogen atom such as a fluorine atom, a chlorine atom, a bromine atom, and an iodine atom. Specific examples thereof include straight-chain or branched-chain C₁-C₄ haloalkoxy groups such as a monofluoromethoxy group, a difluoromethoxy group, a trifluoromethoxy group, a difluoroethoxy group, a trifluoroethoxy group, a tetrafluoroethoxy group, a pentafluoroethoxy group, a trifluoropropoxyl group, and a nonafluorobutoxy group. The haloalkoxy group may further include a substituent(s).

Examples of "alkoxyalkoxy group" include, but are not limited to, C₁-C₄ alkoxy C₁-C₄ alkoxy groups such as a methoxymethoxy group, a 2-methoxyethoxy group, an ethoxymethoxy group, a 2-ethoxyethoxy group, a 3-methoxypropoxy group, a 3-ethoxypropoxy group, 4-methoxybutoxy group, and a 4-ethoxybutoxy group. The alkoxyalkoxy group may further include a substituent(s) .

Examples of "aryl alkoxy group" include, but are not limited to, C₆-C₁₄ aryl C₁-C₄ alkoxy groups such as a phenylmethoxy group (benzyloxy group), and a naphthyloxy group. The aryl alkoxy group may further include a substituent(s).

Examples of "carboxyalkoxy group" include, but are not limited to, a carboxy C₁-C₄ alkoxy groups such as a carboxymethoxy group (HOOCCH₂O group), a carboxyethoxy group (HOOCCH₂CH₂O group), and a carboxydifluoromethoxy group (HOOCCF₂O group). The carboxyalkoxy group may further include a substituent.

Examples of "alkoxycarbonylalkoxy group" include, but are not limited to, alkoxycarbonyl C₁-C₄ alkoxy groups such as an ethoxycarbonylmethoxy group (EtOOCCH₂O group), an ethoxycarbonylethoxy group (EtOOCCH₂CH₂O group), and an ethoxycarbonyldifluoromethoxy group (EtOOCCF₂O group). The alkoxycarbonylalkoxy group may further have a substituent.

Examples of "substituted sulfide group" include, but are not limited to, an alkylthio group which may have a substituent(s), an arylthio group which may have a substituent (s), and an alkoxysulfide group. Specific examples of the alkylthio group which may have a substituent (s) include a methanesulfide group, an ethanesulfide group, and a trifluoromethanesulfide group. Specific examples of the arylthio group which may have a substituent (s) include substituted or unsubstituted arylthio groups having a carbon number ranging from 6 to 14, such as a benzenesulfide group (phenylthio group), a p-toluenesulfide group (4-methylphenylthio group), a 2-methoxybenzenesulfide group (2-methoxyphenylthio group), a 4-chlorobenzenesulfide group (p-chlorophenylthio group), a 4-nitrobenzenesulfide group (4-nitrophenylthio group). Specific examples of the alkoxysulfide group include a methoxysulfide group and an ethoxysulfide group.

Examples of "substituted sulfinyl group" include, but are not limited to: an alkylsulfinyl group such as a methanesulfinyl group, an ethanesulfinyl group, a trifluoromethanesulfinyl group; an arylsulfinyl group such as a benzenesulfinyl group, a p-toluenesulfinyl group, a methoxybenzenesulfinyl group, and a chlorobenzenesulfinyl group; and an alkoxysulfinyl group such as a methoxysulfinyl group and an ethoxysulfinyl group.

Examples of "substituted sulfonyl group" include, but are not limited to: an alkylsulfonyl group which may have a substituent(s), such as a methanesulfonyl group, an ethanesulfonyl group, and a trifluoromethanesulfonyl group; an arylsulfonyl group which may have a substituent(s), such as a benzenesulfonyl group, a p-toluenesulfonyl group, a methoxybenzenesulfonyl group, and a chlorobenzenesulfonyl group; an alkoxysulfonyl group which may have a substituent(s), such as a methoxysulfonyl group and an ethoxysulfonyl group; a sulfamoyl group which may have a substituent (s), such as a sulfamoyl group, an N,N-dimethylsulfamoyl group, and an N- phenylsulfamoyl group.

Examples of "COR¹⁰ group" and "COR¹³ group" include, but are not limited to, a formyl group; and an acyl group which may have a substituent (s), such as an acetyl group, a pivaloyl group, a methoxyacetyl group, a trifluoroacetyl group, a chloroacetyl group, a benzoyl group, a p-methoxybenzyl group, and a p-nitrobenzoyl group. Examples of R¹⁰ and R¹³ include a hydrogen atom, an alkyl group which may have a substituent (s), and an aryl group which may have a substituent(s), as described above.

Examples of "COOR¹¹ group" include, but are not limited to: an alkylester group which may have a substituent(s), such as a methylester group, an ethylester group, an n-propylester group, an isopropylester group, an n-butylester group, a tert-butylester group, a cyclopentylester group, a cyclohexylester group, and a benzylester group; and an arylester group which may have a substituent(s), such as a phenylester group and a naphthylester group. Examples of R¹¹ include a hydrogen atom, an alkyl group which may have a substituent (s), and an aryl group which may have a substituent(s), as described above.

Examples of "OCOR¹² group" include, but are not limited to: a formyloxy group; an alkylcarbonyloxy group which may have a substituent(s), such as a methylcarbonyloxy group and an ethylcarbonyloxy group; and an arylcarbonyloxy group which may have a substituent(s), such as a phenylcarbonyloxy group and a naphthylcarbonyloxy group. Examples of R¹² include a hydrogen atom, an alkyl group which may have a substituent (s), and an aryl group which may have a substituent(s), as described above.

Examples of "OSO₂R¹⁴ group" include, but are not limited to: an alkylsulfonyloxy group which may have a substituent(s), such as a methanesulfonyloxy group, an ethanesulfonyloxy group, a trifluoromethanesulfonyloxy group, and a pentafluoroethanesulfonyloxy group; and an arylsulfonyloxy group which may have a substituent(s), such as a benzenesulfonyloxy group and a toluenesulfonyloxy group. Examples of R¹⁴ include a hydrogen atom, an alkyl group which may have a substituent(s), and an aryl group which may have a substituent(s), as described above.

In one preferred embodiment, in the thiazole compound (1) of the present invention,
R¹ is a hydrogen atom, a halogen atom, or an unsubstituted alkyl group,
R² is a hydrogen atom, a halogen atom, or an alkyl group which may have a substituent (s),
R³ and R⁴ are identical or different, and are a hydrogen atom, a deuterium atom, a halogen atom, an unsubstituted alkyl group, a COR¹⁰ group, or a COOR¹¹ group,
R⁵, R⁶, R⁷, R⁸ and R⁹ are identical or different, and are a hydrogen atom, a halogen atom, a hydroxyl group, an OCOR¹² group, a COR¹³ group, a silyl group which may have a substituent(s), a silyloxy group which may have a substituent(s), a nitro group, a cyano group, an amino group which may have a substituent(s), an alkyl group which may have a substituent(s), an alkoxy group which may have a substituent (s), an aryloxy group which may have a substituent (s), a substituted sulfide group, a substituted sulfinyl group, a substituted sulfonyl group, or an OSO₂R¹⁴ group,
at least one of R⁵, R⁶, R⁷, R⁸ or R⁹ is a hydroxyl group, an OCOR¹² group, a COR¹³ group, a silyl group which may have a substituent (s), a silyloxy group which may have a substituent(s), a cyano group, an amino group which may have a substituent(s), an alkoxy group which may have a substituent(s), an aryloxy group which may have a substituent(s), a substituted sulfide group, a substituted sulfinyl group, a substituted sulfonyl group, or an OSO₂R¹⁴group,
R¹⁰, R¹¹, R¹², R¹³ and R¹⁴ are identical or different, and are a hydrogen atom, an alkyl group which may have a substituent(s), or an aryl group which may have a substituent(s), and
n is 0, 1, or 2.

In one more preferred embodiment, in the thiazole compound,
R¹ is a hydrogen atom, a halogen atom, or an unsubstituted alkyl group,
R² is a halogen atom or a haloalkyl group,
R³ and R⁴ are identical or different and are a hydrogen atom, a deuterium atom, a halogen atom, or an unsubstituted C₁-C₄ alkyl group,
R⁵ is an unsubstituted alkoxy group, a haloalkoxy group, or an unsubstituted alkyloxy C₁-C₄ alkoxy group, and
at least one of R⁶, R⁷, R⁸ or R⁹ is a hydrogen atom, a halogen atom, an alkyl group, a haloalkyl group, an alkoxy group, or a haloalkoxy group.

In one still more preferred embodiment, in the thiazole compound,
R¹ is a hydrogen atom, a halogen atom, or an unsubstituted C₁-C₄ alkyl group,
R² is a halogen atom or a halo C₁-C₄ alkyl group,
R³ and R⁴ are identical or different and are a hydrogen atom, a halogen atom, or an unsubstituted C₁-C₄ alkyl group,
R⁵ is an unsubstituted C₁-C₄ alkoxy group, a halo C₁-C₄ alkoxy group, or an unsubstituted C₁-C₄ alkyloxy C₁-C₄ alkoxy group, and
at least one of R⁶, R⁷, R⁸ or R⁹ is a hydrogen atom, a halogen atom, an unsubstituted C₁-C₄ alkyl group, a halo C₁-C₄ alkyl group, an unsubstituted C₁-C₄ alkoxy group, or a halo C₁-C₄ alkoxy group.

In one particularly preferred embodiment, in the thiazole compound,
R¹ is a hydrogen atom, a halogen atom, or an unsubstituted C₁-C₄ alkyl group,
R² is a halogen atom or a halo C₁-C₄ alkyl group,
R³ and R⁴ are identical or different and are a hydrogen atom, a halogen atom, or an unsubstituted C₁-C₄ alkyl group,
R⁵ is an unsubstituted C₁-C₄ alkoxy group or a halo C₁-C₄ alkoxy group,
at least one of R⁶, R⁷, or R⁸ is a hydrogen atom, a halogen atom, an unsubstituted C₁-C₄ alkyl group, a halo C₁-C₄ alkyl group, an unsubstituted C₁-C₄ alkoxy group, or a halo C₁-C₄ alkoxy group, and
R⁹ is a hydrogen atom, a halogen atom, an unsubstituted C₁-C₄ alkyl group, a halo C₁-C₄ alkyl group, an unsubstituted C₁-C₄ alkoxy group, or a halo C₁-C₄ alkoxy group.

### Method for Producing Thiazole Compound

The thiazole compound (1) of the present invention can be produced by various methods. For example, a thiazole compound represented by the general formula (1A-1) can be produced by a method represented by the reaction formula-1 shown below. 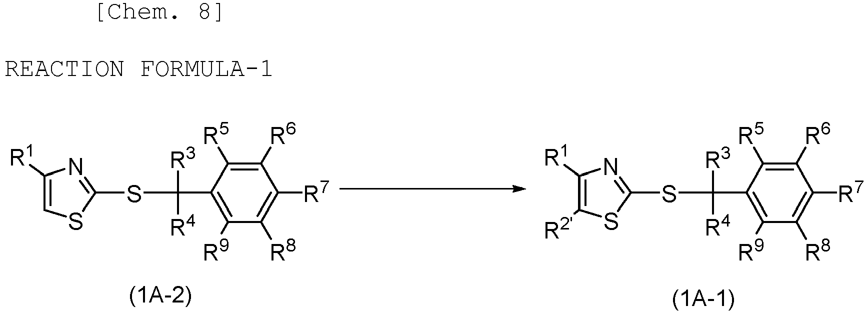 wherein R¹, R³, R⁴, R⁵, R⁶, R⁷, R⁸, and R⁹ are the same as those described above, and R^{2'} represents a halogen atom.

According to the reaction formula-1, a thiazole compound represented by the general formula (1A-2) is allowed to react with a halogenating agent in an appropriate solvent, thereby producing the thiazole compound represented by the general formula (1A-1) of the present invention [the compound 1A: the thiazole compound represented by the general formula (1) wherein n is 0 and R² is a halogen atom].

A solvent which is inert to this reaction can be used as the solvent for this reaction. Examples of the solvent include: hydrocarbons such as hexane, cyclohexane, and heptane; aromatic hydrocarbons such as benzene, chlorobenzene, toluene, and xylene; halogenated hydrocarbons such as methylene chloride, dichloroethane, chloroform, and carbon tetrachloride; esters such as ethyl acetate and methyl acetate; ethers such as diethyl ether, tetrahydrofuran (THF), and dioxane; nitriles such as acetonitrile and propionitrile; alcohols such as methanol, ethanol, and isopropylalcohol; amides such as N,N-dimethylformamide (DMF) and N,N-diethylformamide; and sulfoxides such as dimethylsulfoxide (DMSO). Further, a mixed solvent containing at least two of these substances may be used. Each of these solvents and the mixed solvent is used suitably in an amount of approximately from 0.05 L to 500 L, and preferably in an amount of approximately from 0.5 L to 20 L, with respect to 1 kg of the thiazole compound represented by the general formula (1A-2) .

Examples of the halogenating agent include, but are not limited to, chlorine gas, bromine, sulfuryl chloride, N-chlorosuccinimide (NCS), N-bromosuccinimide (NBS), and N-iodosuccinimide (NIS).

To cause this reaction, the thiazole compound of the general formula (1A-2) and the halogenating agent may be used in any ratio. However, it is preferable to use 0.8-1.5 mol of the halogenating agent with respect to 1 mol of the compound of the general formula (1A-2). This reaction is preferably caused within a temperature range from -20°C to the boiling point of the solvent in use, and usually completed within a period of approximately from 0.5 to 24 hours.

The thiazole compound represented by the general formula (1A-2) and used in the reaction formula-1 can be produced, for example, by a method represented by the reaction formula-2 shown below. 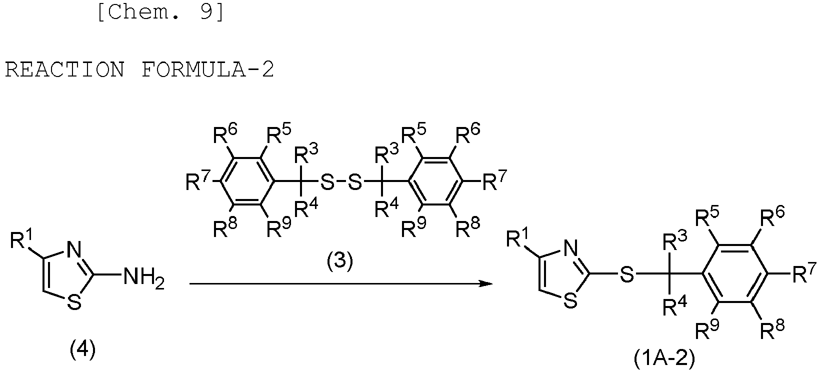 wherein R¹, R³, R⁴, R⁵, R⁶, R⁷, R⁸, and R⁹ are the same as those described above.

According to the reaction formula-2, 2-aminothiazole (4) is allowed to react with a diazotizing agent and a benzyl disulfide compound represented by the general formula (3), thereby producing the thiazole compound represented by the general formula (1A-2).

A solvent which is inert to this reaction can be used as the solvent for this reaction. Examples of the solvent include: hydrocarbons such as hexane, cyclohexane, and heptane; aromatic hydrocarbons such as benzene, chlorobenzene, toluene, and xylene; halogenated hydrocarbons such as methylene chloride, dichloroethane, chloroform, and carbon tetrachloride; esters such as ethyl acetate and methyl acetate; ethers such as diethyl ether, THF, and dioxane; nitriles such as acetonitrile and propionitrile; alcohols such as methanol, ethanol, and isopropylalcohol; amides such as DMF and N,N-diethylformamide; and sulfoxides such as DMSO. Further, a mixed solvent containing at least two of these substances or a mixed solvent containing at least one of these substances and water may be used. Each of these solvents and the mixed solvents is used suitably in an amount of approximately from 0.05 L to 500 L, and preferably in an amount of approximately from 0.5 L to 20 L, with respect to 1 kg of 2-aminothiazole (4).

Examples of the diazotizing agent include, but are not limited to: nitrous esters such as isoamyl nitrite; and nitrites such as sodium nitrite.

To cause this reaction, 2-aminothiazole (4), a benzyl disulfide compound represented by the general formula (3), and the diazotizing agent may be used in any ratio. However, it is preferable to use 0.8-3 mol of the benzyl disulfide compound represented by the general formula (3) and 0.8-4 mol of the diazotizing agent, with respect to 1 mol of 2-aminothiazole (4). This reaction is preferably caused within a temperature range from -20°C to the boiling point of the solvent in use, and usually completed within a period of approximately from 0.1 to 40 hours.

The starting material, i.e., 2-aminothiazole (4), is a known compound and commercially available. The benzyl disulfide compound (3) is commercially available. Alternatively, it is possible to prepare the compound by a method described in a document (e.g., Tetrahedron, 35, 2329 (1979)).

The sulfone compound (1C) shown below can be produced by the method represented by the reaction formula-3 shown below. 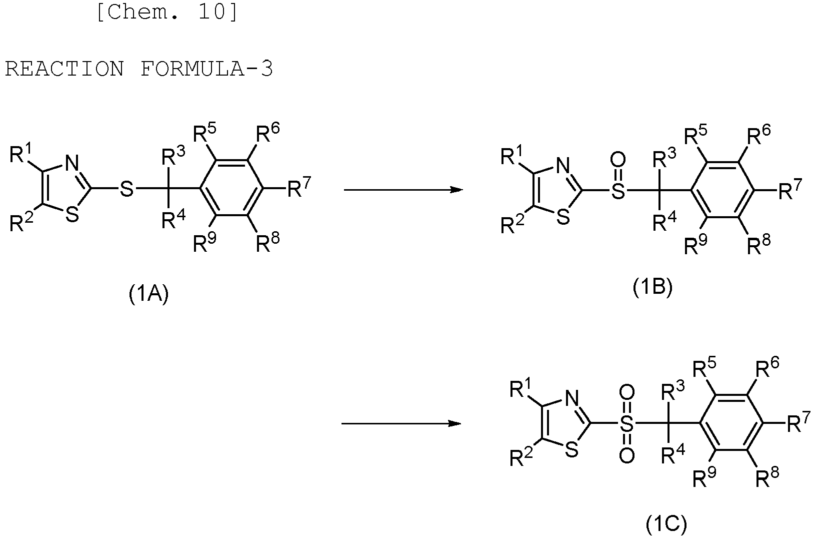 wherein R¹, R², R³, R⁴, R⁵, R⁶, R⁷, R⁸, and R⁹ are the same as those described above.

A thiazole compound represented by the general formula (1A) (hereinafter also referred to as "the sulfide compound (1A)" or "the thiazole compound (1A)") is allowed to react with an oxidant, thereby producing the thiazole compound represented by the general formula (1B) of the present invention [the sulfoxide compound (1B) or "the thiazole compound (1B)": the thiazole compound of the general formula (1) wherein n is 1], and/or the thiazole compound represented by the general formula (1C) of the present invention [the sulfone compound (1C) or "the thiazole compound (1C)": the thiazole compound of the general formula (1) wherein n is 2].

A solvent which is inert to this reaction can be used as the solvent for this reaction. Examples of the solvent include: hydrocarbons such as hexane, cyclohexane, and heptane; aromatic hydrocarbons such as benzene, chlorobenzene, toluene, and xylene; halogenated hydrocarbons such as methylene chloride, dichloroethane, chloroform, and carbon tetrachloride; esters such as ethyl acetate and methyl acetate; ethers such as diethyl ether, THF, and dioxane; nitriles such as acetonitrile and propionitrile; alcohols such as methanol, ethanol, and isopropylalcohol; amides such as DMF and N,N-diethylformamide; sulfoxides such as DMSO; carboxylic acids such as acetic acid; and water. Further, a mixed solvent containing at least two of these substances may be used. Each of these solvents and the mixed solvent is suitably used in an amount of approximately from 0.05 L to 500 L, and preferably in an amount of approximately from 0.5 L to 20 L, with respect to 1 kg of the sulfide compound (1A).

Examples of the oxidant usable in this reaction include, but are not limited to, meta-chloroperbenzoic acid (mCPBA), hydrogen peroxide, peracetic acid, permanganate, hydroperoxide, potassium peroxysulfate, sodium perborate, sodium meta-periodate, osmium oxide, ruthenium oxide, nitric acid, chromic acid, sodium dichromate, halogen, sodium hypochlorite, iodobenzene dichloride, ozone, and singlet oxygen.

The oxidant may be used in a stoichiometric amount or more with respect to the amount of the sulfide compound (1A) or the sulfoxide compound (1B). However, it is preferable to use the oxidant in an amount approximately from 0.8 to 5 times. Usually, this reaction is preferably caused within a temperature range from -20°C to the boiling point of the solvent in use, and usually completed within a period of approximately from 0.5 to 24 hours.

Note that the sulfone compound (1C) can be produced from the sulfide compound (1A) not only in a single step, but also in two steps.

A sulfone compound (1C-3) having the R³ group and the R⁴ group can be produced by the method represented by the reaction formula-4 shown below. 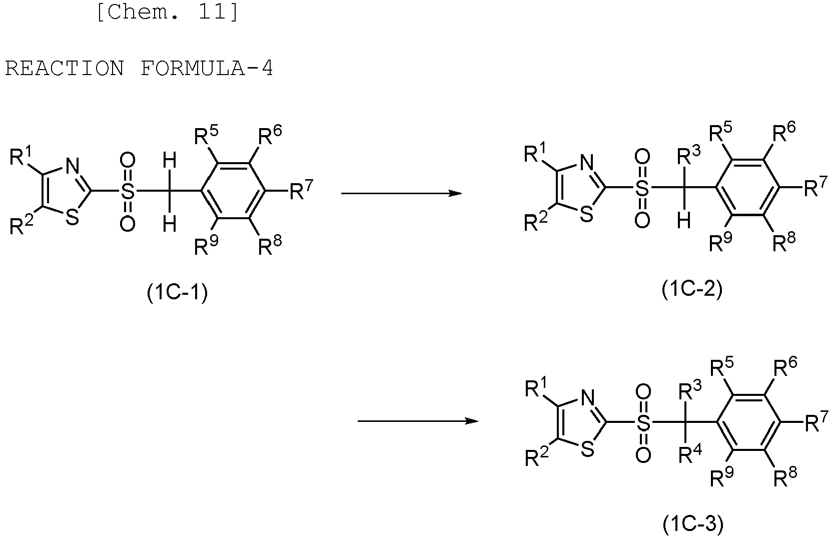 wherein R¹, R², R³, R⁴, R⁵, R⁶, R⁷, R⁸, and R⁹ are the same as those described above.

According to the reaction formula-4, a thiazole compound represented by the general formula (1C-1) of the present invention (hereinafter also referred to as "the thiazole compound (1C-1)") reacts, in a solvent, with an alkylating agent, a benzylating agent, or a deuterating agent in the presence of a base, thereby obtaining a thiazole compound represented by the general formula (1C-2) (hereinafter also referred to as "the thiazole compound (1C-2)") and/or a thiazole compound represented by the general formula (1C-3) (hereinafter also referred to as "the thiazole compound (1C-3)").

In one preferred embodiment, the reaction to produce the thiazole compound (1C-3) of the present invention is caused in the following manner. Examples of the usable solvent include: aliphatic hydrocarbons such as hexane, heptane, and pentane; alicyclic hydrocarbons such as cyclohexane; aromatic hydrocarbons such as benzene, toluene, and xylene; halogenated hydrocarbons such as dichloromethane, chloroform, 1,2-dichloroethane, 1,1,1-trichloroethane, tetrachloroethylene, trichloroethylene, carbon tetrachloride, chlorobenzene, and o-dichlorobenzene; alcohols such as methanol, ethanol, and isopropanol; ethers such as diethyl ether, isopropyl ether, THF, dioxane, and monoglyme; esters such as methyl formate, methyl acetate, ethyl acetate, butyl acetate, and methyl propionate; amides such as DMF, N,N-dimethylacetamide, and 1,3-dimethyl imidazolidinone; nitriles such as acetonitrile; sulfoxides such as DMSO; water; and a mixed solvent containing at least two of the foregoing substances.

Each of these solvents and the mixed solvent is suitably used in an amount of approximately from 0.05 L to 500 L, and preferably in an amount of approximately from 0.5 L to 20 L, with respect to 1 kg of the thiazole compound (C1-1).

Examples of the alkylating agent usable in this reaction include, but are not limited to, alkyl halides such as methyl iodide, methyl bromide, and ethyl bromide; and sulfuric esters such as dimethyl sulfate and ethyl methanesulphonate.

Examples of the benzylating agent usable in this reaction include, but are not limited to, benzyl chloride and benzyl bromide.

Examples of the deuterating agent usable in this reaction include, but are not limited to, protic compounds including heavy hydrogen. Specific examples thereof include heavy water, deuteroalcohols such as deuteromethanol and deuteroethanol, Deuterium chloride, and alkali deuteride. To accelerate the deuteration reaction, an additive such as trimethylamine, triethylamine, metal vanadate apatite (e.g., calcium vanadate apatite) may be added to the reaction solution. The alkylating agent, the benzylating agent, or the deuterating agent is suitably used in an amount of from 0.8 to 1.6 equivalents, and preferably from 1 to 1.1 equivalents, with respect to the thiazole compound (1C-1).

In one preferred embodiment, this reaction takes place in the presence of a base. Examples of the usable base include: alkali metal alkoxides such as sodium methoxide, sodium ethoxide, and potassium tert-butoxide; alkali metal hydroxides such as sodium hydroxide and potassium hydroxide; alkali metal carbonates such as sodium carbonate and potassium carbonate; alkali metal hydrides such as sodium hydride, organic bases such as triethylamine, pyridine, and diazabicycloundecene (DBU); and lithium salts such as butyllithium, lithium diisopropylamide, and lithium bistrimethylsilylamide. One kind of these bases may be used alone, or two or more kinds thereof may be used in combination. The base is suitably used in an amount of from 0.8 to 5 equivalents, and preferably from 1 to 1.1 equivalents, with respect to the thiazole compound (1C-1). A temperature for this reaction may be arbitrarily set within the range from -100°C to the boiling point of the solvent in use. The reaction time is not particularly limited, and is generally in the range approximately from 10 minutes to 24 hours, which is sufficient for the reaction.

For the reaction by which the thiazole compound (1C-3) is produced from the thiazole compound (1C-2) produced by the above reaction, the kind and amount of the solvent to be used, the kind and amount of the alkylating agent to be used, the benzylating agent or deuterating agent to be used, the kind and amount of the base to be used, and the reaction temperature and time are suitably set to be identical as those of the reaction by which the thiazole compound (1C-2) is produced from the thiazole compound (1C-1). Note that the thiazole compound (1C-3) can be produced from the thiazole compound (1C-1) not only in a single step, but also in two steps.

The sulfide compound (1A) can be produced by the reaction represented by the reaction formula-5 shown below. 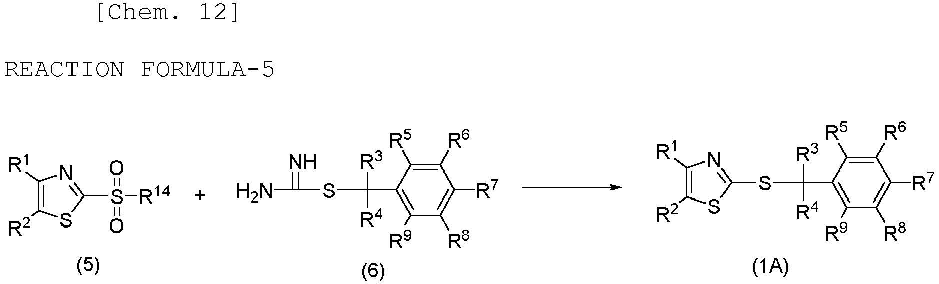 wherein R¹, R², R³, R⁴, R⁵, R⁶, R⁷, R⁸, and R⁹ are the same as those described above, and R¹⁴ represents an alkyl group which may have a substituent(s).

According to the reaction formula-5, a thiazolesulfone compound represented by the general formula (5) is allowed to react, in a solvent, with a carbamimidothioic acid compound represented by the formula (6) or a salt thereof in the presence of a base, thereby obtaining the sulfide compound (1A).

The starting materials, i.e., the thiazolesulfone compound (5) and the carbamimidothioic acid compound (6) or a salt thereof are commercially available. Alternatively, the starting materials can be easily prepared by a method described in a document (e.g., International Publication No. 2006/123088).

Examples of the salts of the carbamimidothioic acid compound include, but are not limited to, hydrochloride and hydrobromic acid.

In one preferred embodiment, the reaction to produce the thiazole compound represented by the general formula (1A) of the present invention is caused in the following manner. Examples of the usable solvent include: aliphatic hydrocarbons such as hexane, heptane, and pentane; alicyclic hydrocarbons such as cyclohexane; aromatic hydrocarbons such as benzene, toluene, and xylene; halogenated hydrocarbons such as dichloromethane, chloroform, 1,2-dichloroethane, 1,1,1-trichloroethane, tetrachloroethylene, trichloroethylene, carbon tetrachloride, chlorobenzene, and o-dichlorobenzene; alcohols such as methanol, ethanol, and isopropanol; ethers such as diethyl ether, isopropyl ether, THF, dioxane, and monoglyme; esters such as methyl formate, methyl acetate, ethyl acetate, butyl acetate, and methyl propionate; amides such as N,N-dimethylformamide, N,N-dimethylacetamide, and 1,3-dimethyl imidazolidinone; nitriles such as acetonitrile; sulfoxides such as DMSO; water; and a mixed solvent containing at least two of the foregoing substances.

Each of these solvents and the mixed solvent is suitably used in an amount of approximately from 0.05 L to 500 L, and preferably in an amount of approximately from 0.5 L to 20 L, with respect to 1 kg of the thiazolesulfone compound represented by the general formula (5).

In one preferred embodiment, this reaction takes place in the presence of a base. Examples of the usable base include, but are not limited to: alkali metal alkoxides such as sodium methoxide, sodium ethoxide, and potassium tert-butoxide; alkali metal hydroxides such as sodium hydroxide and potassium hydroxide; alkali metal carbonates such as sodium carbonate and potassium carbonate; alkali metal hydrides such as sodium hydride, organic bases such as triethylamine, pyridine, and DBU; and lithium salts such as butyllithium, lithium diisopropylamide, lithium bistrimethylsilylamide. One kind of these bases may be used alone, or two or more kinds thereof may be used in combination. The base is suitably used in an amount of from 0.8 to 10 equivalents, and preferably from 2 to 8 equivalents, with respect to the thiazole compound represented by the general formula (5).

For this reaction, a medium including both water and the organic solvent (in particular, a hydrophobic organic solvent) can be used. A phase transfer catalyst may be present to accelerate the reaction between substances of two different phases.

Examples of the phase transfer catalyst include, but are not limited to; quaternary ammonium salts such as tetrabutylammonium bromide (TBAB), trimethylbenzylammonium bromide, triethylbenzylammonium bromide, and trioctylmethylammonium chloride (TOMAC); phosphonium salts such as tetrabutylphosphonium chloride (TBPC); and crown ethers such as 15-crown-5 and 18-crown-6. Additionally, known substances such as alkylammonium salts, carboxylates, and alkyl sulfonic acid salts can be used as the phase transfer catalyst. Among these substances, the quaternary ammonium salts are preferable. For example, TBAB, TOMAC, or Aliquat 336® can be used suitably.

If used, the phase transfer catalyst is added in an amount of from 0.05% to 10% by weight, preferably from 0.08% to 5% by weight, with respect to the weight of the water used as the medium.

A temperature for this reaction may be arbitrarily set within the range from -100°C to the boiling point of the solvent in use. The reaction time is not particularly limited, and is generally in the range approximately from 10 minutes to 24 hours, which is sufficient for the reaction.

The compounds obtained by the reactions described above are easily isolated from the respective reaction mixtures by an ordinary isolation method, such as organic solvent extraction and chromatography, and purified by a commonly-used isolation/purification method such as recrystallization.

### 2. Herbicide

The thiazole compound of the present invention and a herbicide including the thiazole compound as an active ingredient effectively act on weeds which cause various agricultural and horticultural problems, and in particular, are highly herbicidally active against: paddy field weeds such as Cyperus difformis (umbrella sedge), Schoenoplectus sp. (Hotarui) (e.g. Scirpus juncoides), Lindernia procumbens, Monochoria vaginalis, Potamogeton distinctus (pondweed), Rotala indica var. uliginosa, and Echinochloa crus-galli (L.) P.Beauv. var. formosensis Ohwi; and upland field weeds such as Cyperus microiria, Amaranthus viridis, Abutilon theophrasti, Lolium multiflorum Lam. (Italian ryegrass), Setaria viridis, and Digitaria ciliaris.

A herbicide composition including the thiazole compound of the present invention as an active ingredient is effective also when used for a foliage treatment and/or a soil treatment, and provides a particularly high herbicidal effect when used for a flooding treatment. A mechanism of action of the compound of the present invention is unknown. However, it is assumed that the compound of the present invention brings a completely different action as compared to compounds or compositions used in known herbicides because the compound of the present invention neither exhibits quick effects, nor causes browning in portions of plants in contact with the herbicide of the present invention, the quick effects and the browning being unique to light-dependent herbicides.

The compound of the present invention may be used alone as a herbicide, without adding any other components. However, the compound of the present invention is usually mixed with, for example, a liquid, solid, or gaseous carrier. Further, a surfactant and/or other formulation adjuvants are added to the compound as necessary. Thus, the compound can be formulated and used in various forms, such as an emulsion, a wettable powder, a dry flowable formulation, a flowable formulation, a water-soluble formulation, a granule formulation, a fine granule formulation, a parvule formulation, a powder formulation, a coating formulation, a spray formulation, an aerosol formulation, a micro-capsule formulation, a fumigant formulation, and a smoke formulation.

In these formulations, the content of the compound of the present invention that is the active ingredient can be appropriately selected from a wide range in accordance with various conditions such as the form of the formulation and the place where the formulation is used. Usually, the content of the compound of the present invention is approximately from 0.01% to 95% by weight, and preferably from 0.1% to 50% by weight. Examples of the solid carrier usable for the preparation of the formulation include: clay such as kaoline clay, diatomaceous earth, bentonite, Fubasami clay, and Japanese acid clay; talc; ceramics; inorganic minerals such as cerite, quartz, sulfur, activated carbon, silica carbonate, and hydrated silica; fine power such as a chemical fertilizer; and a granular substances.

Examples of the liquid carrier include: water; alcohols; ketones such as acetone and methyl ethyl ketone; hydrocarbons such as hexane, cyclohexane, kerosene, and light oil; aromatic hydrocarbons such as benzene, toluene, xylene, and naphthalene; esters such as ethyl acetate and butyl acetate; nitriles such as acetonitrile and isobutyronitrile; ethers such as diisopropyl ether and dioxane; acid amides such as DMF and N,N-dimethylacetamide; halogenated hydrocarbons such as dichloromethane, trichloroethane, and carbon tetrachloride; DMSO; and vegetable oils such as soybean oil and cottonseed oil.

The gaseous carrier is generally used as a propellant, and examples thereof include butane gas, liquefied petroleum gas, dimethyl ether, and carbonic acid gas.

Specific examples of the surfactant include: nonionic surfactants such as polyoxyethylene alkyl ether, polyoxyethylene alkyl ester, and polyethylene sorbitan alkyl ester; and anionic surfactants such as alkyl benzene sulfonate, alkyl sulfosuccinate, alkyl sulfate, polyoxyethylene alkyl sulfate, allyl sulfonate, and lignin sulfite.

Examples of the formulation adjuvants include a sticking agent, a dispersing agent, and a stabilizer. Examples of the sticking agent and/or the dispersing agent include casein, gelatin, polysaccharides (such as starches, gum arabic, cellulose derivatives, and alginic acid), lignin derivatives, bentonite, saccharides, and synthetic water-soluble polymers (such as polyvinyl alcohol, polyvinyl pyrrolidone, and polyacrylic acids). Examples of the stabilizer include isopropyl acid phosphate (PAP), 2,6-di-tert-butyl-4-methylphenol (BHT), a mixture (BHA) of 2-tert-butyl-4-methoxyphenol and 3-tert-butyl-4-methoxyphenol, vegetable oils, mineral oils, surfactants, fatty acids and esters thereof. These formulations may be colored with an organic or inorganic dye.

The thus-obtained formulations may be used as they are or diluted with, for example, water before use. However, for example, the granule formulation and the powder formulation are generally used as they are without dilution. If the emulsion, the wettable powder, and the flowable formulation are diluted before use, the dilution is carried out such that the active ingredient usually has a concentration approximately from 0.0001% to 100% by weight, and preferably has a concentration approximately from 0.001% to 10% by weight.

Prior to formulating, the thiazole compound of the present invention may be mixed, in advance, with another herbicide, an insecticide, a nematicide, a miticide, a bactericide, a plant growth-regulating agent, a synergist (e.g., piperonyl butoxide), a soil conditioner, or any other chemical. Alternatively, the formulations of the present invention and the chemicals and agents described above may be used in combination.

If the compound of the present invention is used as a herbicide, the application amount of the compound is not particularly limited, but is appropriately selected from a wide range, in accordance with various conditions such as the form of the formulation, the method, timing, and place of application, and the species of a crop planted in the place of application, and the species of a weed to be destroyed. For example, in a case where a foliage treatment, a soil treatment , or a flooding treatment is performed, the application amount is set such that the amount of the active ingredient usually ranges approximately from 1 g to 4000 g, and preferably approximately from 10 g to 1000 g, with respect to an area of 10000 m².

### EXAMPLES

The present invention will now be described in detail with reference to working examples, reference examples, formulation examples, and test examples. Note that the present invention is not limited to the following examples.

### Example 1 (Route 1)

### Synthesis of 5-chloro-2-[(2,5-difluoro-4-ethoxyphenyl)(methanesulfanyl)]-1,3-thiazole (Compound 1A-97)

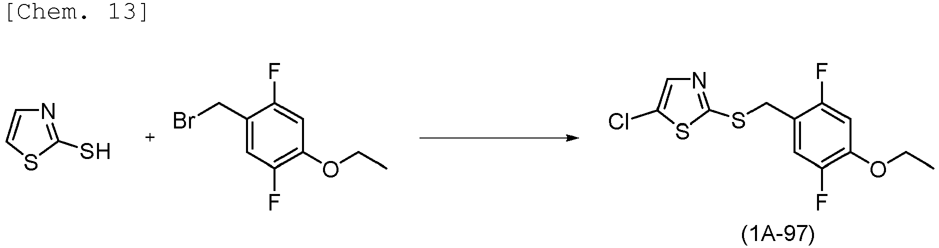

In an eggplant flask, 2-mercaptothiazole (1.44 g, 12.3 mmol) was dissolved in DMF (30 ml). N-chlorosuccinimide (hereinafter also referred to as "NCS") (4.94 g, 37.0 mmol) was added to the solution under ice cooling, and the solution was stirred for 1 hour. Thereafter, K₂CO₃ (5.11 g, 37.0 mmol) was added to the reaction solution, and then, the solution was stirred for 5 minutes. Subsequently, 5-difluoro-4-ethoxybenzyl bromide (3.10 g. 12.3 mmol) was added. After the temperature was raised to room temperature, the solution was stirred for 17 hours and 40 minutes. The reaction was stopped by addition of water to the reaction solution, and extraction was carried out using ethyl acetate. The obtained ethyl acetate solution was washed with water, and washed with a saturated saline solution. An obtained organic phase was dried using anhydrous magnesium sulfate. Following filtration of the obtained solution, the filtrate was concentrated under reduced pressure. A crude product was purified by silica gel column chromatography (ethyl acetate:hexane=20:1), thereby obtaining the subject thiazole compound (1A-97) as a yellow oil (0.93 g, 23%).

### Example 2

### Synthesis of 5-chloro-2-[(2,5-difluoro-4-ethoxyphenyl)(methanesulfonyl)]-1,3-thiazole (Compound 1C-101)

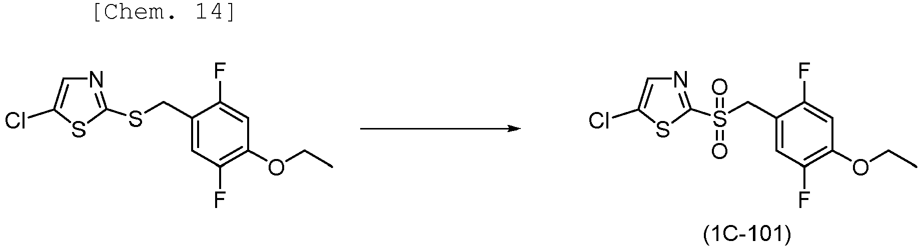

In an eggplant flask, the compound produced in Example 1 described above (1A-97; 0.93 g, 2.89 mmol) was dissolved in dichloromethane (15 ml). Under ice cooling, mCPBA (1.79 g, 7.22 mmol) was added to the obtained solution. Thereafter, the temperature of the solution was raised to room temperature, and then, the solution was stirred for 22 hours. The reaction was stopped by addition of a Na₂SO₃ aqueous solution to the obtained reaction solution, and extraction was carried out using ethyl acetate. An obtained organic phase was washed with a 1M NaOH aqueous solution, water and a saturated saline solution, and dried using anhydrous magnesium sulfate. Following filtration of the solution, the filtrate was concentrated under reduced pressure. A residue was purified by silica gel column chromatography (ethyl acetate:hexane=3:1), thereby obtaining the subject thiazole compound (1C-101) as a white solid (0.65 g, 64%).

### Reference Example 1

### Synthesis of 2-difluoromethoxy-6-fluoro benzyl bromide

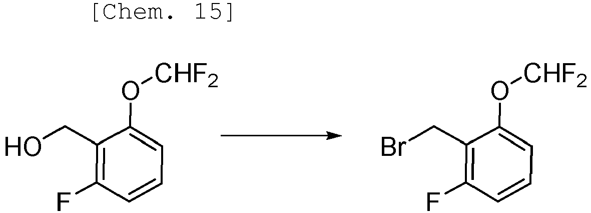

In an eggplant flask, 2-difluoromethoxy-6-fluorobenzyl alcohol (1.87 g, 9.74 mmol) was dissolved in dichloromethane (30 ml). CBr₄ (3.88 g, 11.7 mmol) and PPh₃ (3.07 g, 11.7 mmol) were added to the solution under ice cooling. Thereafter, the temperature was raised to room temperature, and then, stirring was carried out for 1 hour and 15 minutes. Following the completion of the reaction, the reaction solution was concentrated under reduced pressure. A residue was suspended in a solution containing hexane and ethyl acetate at a ratio of 10:1, and then filtered. The obtained filtrate was concentrated under reduced pressure, thereby obtaining 2.98 g of 2-difluoromethoxy-6-fluorobenzyl bromide as a colorless oil (crude).

### Reference Example 2

### Synthesis of carbamimidothioic acid 2-difluoromethoxy-6-fluorophenylmethyl hydrobromide

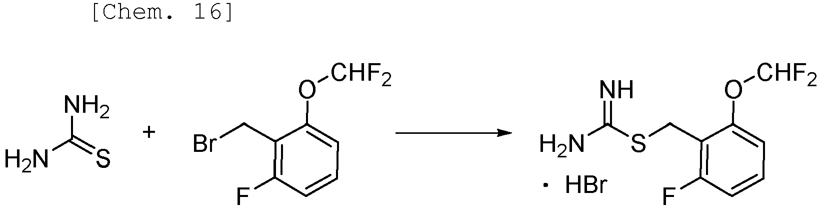

In an eggplant flask, 2.98 g (crude) of 2-difluoromethoxy-6-fluorobenzyl bromide obtained in Reference Example 1 was dissolved in acetone (30 ml), and thiourea (0.82 g, 10.7 mmol) was added. Heat reflux was carried out for 4 hours and 45 minutes. Acetone contained in the solution was evaporated out, thereby obtaining carbamimidothioic acid 2-difluoromethoxy-6-fluorophenylmethyl hydrobromide as a while solid (crude), which was used in the next reaction.

### Example 3 (Route 2)

### Synthesis of 5-chloro-2-{[2-(difluoromethoxy)-6-fluorophenyl][methanesulfanyl]}-1,3-thiazole (Compound 1A-40)

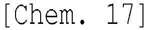

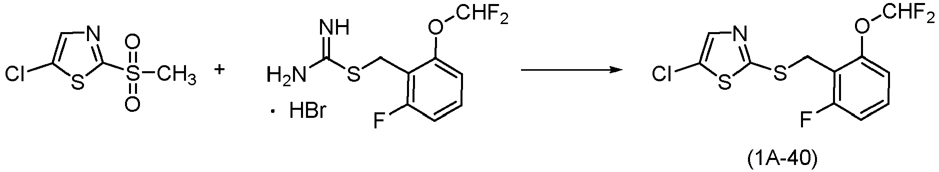

In an eggplant flask, a 1M NaOH aqueous solution (30 ml) was added to carbamimidothioic acid 2-difluoromethoxy-6-fluorophenylmethyl hydrobromide (crude) obtained in Reference Example 2, and the mixture was stirred for 1 hour and 10 minutes at room temperature. Thereafter, toluene (30 ml), tetrabutylammonium bromide (TBAB) (1.57 g, 4.87 mmol), and 2-methylsulfonyl-5-chlorothiazole (1.93 g, 9.74 mmol) were added to the reaction solution, and the solution was stirred for 22 hours at room temperature. After the completion of the reaction, a toluene phase was washed with water and a saturated saline solution, and dried using anhydrous magnesium sulfate. Following filtration of the solution, the filtrate was concentrated under reduced pressure. A residue was purified by silica gel column chromatography (ethyl acetate:hexane=9:1), thereby obtaining 1.67 g the subject thiazole compound (1A-40) as a yellow oil (crude).

### Example 4 (Route 2)

### Synthesis of 5-chloro-2-{[2-(difluoromethoxy)-6-fluorophenyl][methanesulfonyl]}-1,3-thiazole (Compound 1C-41)

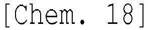

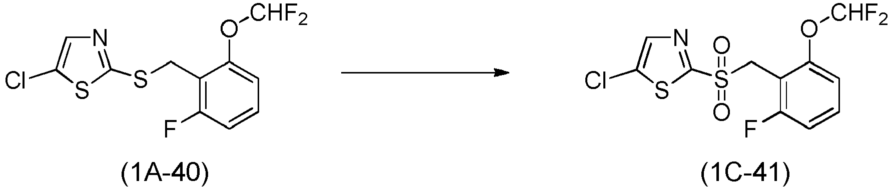

In an eggplant flask, the thiazole compound (1A-40; 1.67g, crude) obtained in Example 3 was dissolved in a dichloromethane (20 ml). Under ice cooling, mCPBA (3.16 g, 12.8 mmol) was added to the solution. Thereafter, the temperature of the reaction solution was raised to room temperature, and then, the solution was stirred for 24 hours and 45 minutes. The reaction was then stopped by addition of a Na₂SO₃ aqueous solution to the reaction solution, and extraction was carried out using ethyl acetate. An organic phase was washed with a 1M NaOH aqueous solution, water, and a saturated saline solution, and dried using anhydrous magnesium sulfate. Following filtration of the resultant solution, the filtrate was concentrated under reduced pressure. A residue was purified by silica gel column chromatography (ethyl acetate:hexane=3:1), thereby obtaining the subject thiazole compound (1C-41) as a while solid (1.57 g, 4 steps, 47%).

### Example 5

### Synthesis of 5-chloro-2-{[3-(trifluoromethoxy)phenyl][methanesulfanyl]}-1,3-thiazole (Compound 1A-81)

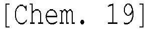

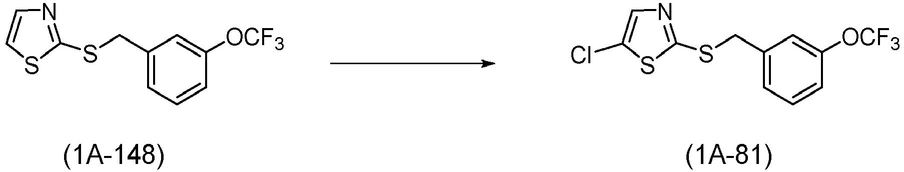

In an eggplant flask, 2-{[3-(trifluoromethoxy)phenyl][methanesulfanyl]}-1,3-thiazole(1A-148; 1.0 g, 3.43 mmol) was dissolved in DMF (10 ml), and NCS (690 mg, 5.15 mmol) was added at room temperature. The solution was further stirred for 1 hour and 50 minutes at room temperature. The reaction was stopped by addition of a sodium sulfate aqueous solution to the reaction solution, and extraction was carried out using ethyl acetate. An organic phase was washed with water and a saturated saline solution, and dried using anhydrous magnesium sulfate. Following filtration of the solution, the filtrate was concentrated under reduced pressure. A residue was purified by silica gel column chromatography (ethyl acetate:hexane=20:1), thereby obtaining the subject thiazole compound (1A-81) as a pale yellow oil (240 mg, 21%).

### Example 6

### Synthesis of 5-chloro-2-{[3-(trifluoromethoxy)phenyl][methanesulfonyl]}-1,3-thiazole (Compound 1C-85)

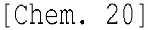

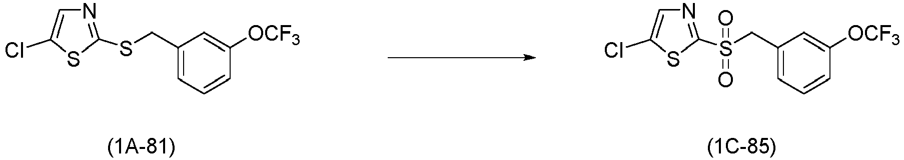

In an eggplant flask, the thiazole compound (1A-81) obtained in Example 5 (240 mg, 0.737 mmol) was dissolved in chloroform (5 ml). At room temperature, mCPBA (450 mg, 1.84 mmol) was added to the solution, and the solution was stirred for 21 hours. The reaction was stopped by addition of a sodium sulfate aqueous solution to the reaction solution.

The obtained reaction solution was extracted using ethyl acetate. An obtained organic phase was washed with a sodium hydroxide aqueous solution, water, and a saturated saline solution, and dried using anhydrous magnesium sulfate. Following filtration of the solution, the filtrate was concentrated under reduced pressure. A residue was suspended in hexane, and the suspension was filtered, thereby obtaining the subject thiazole compound (1C-85) as a white solid (180 mg, 76%)

### Example 7

### Synthesis of 5-bromo-2-{[2-(2,2,2-trifluoroethoxy)-6-chlorophenyl][methanesulfanyl]}-1,3-thiazole(Compound 1A-127)

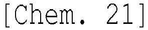

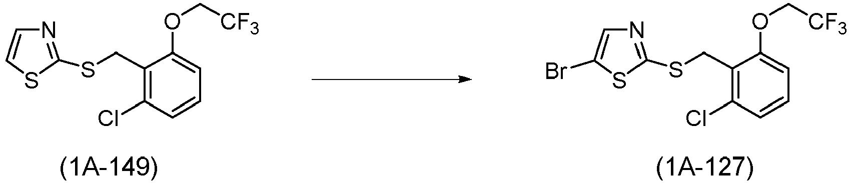

In an eggplant flask, 2-{[2-(2,2,2-trifluoroethoxy)-6-chlorophenyl][methanesulfanyl]}-1,3-thiazole (1A-149; 690 mg, 2.03 mmol) was dissolved in DMF (15 ml). Under ice cooling, NBS (430 mg, 2.44 mmol) was added to the solution. The temperature was raised to room temperature, and then, the solution was stirred for 65 hours. The reaction was stopped by addition of a sodium sulfate aqueous solution to the reaction solution, and extraction was carried out using ethyl acetate. An obtained organic phase was washed with water and a saturated saline solution, and dried using anhydrous magnesium sulfate. Following filtration of the solution, the filtrate was concentrated under reduced pressure. A residue was purified by silica gel column chromatography (ethyl acetate:hexane=7:1), thereby obtaining the subject thiazole compound (1A-127) as a white solid (670 mg, 79%).

### Example 8

### Synthesis of 5-bromo-2-{[2-(2,2,2-trifluoroethoxy)-6-chlorophenyl][methanesulfonyl]}-1,3-thiazole (Compound 1C-161)

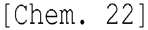

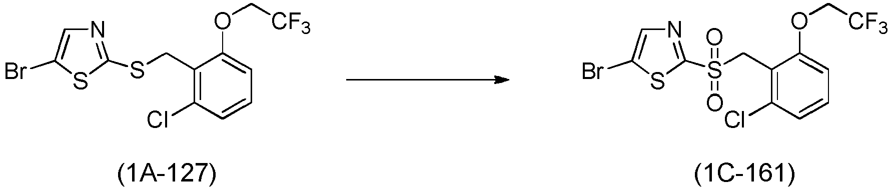

In an eggplant flask, the thiazole compound (1A-127; 210 mg, 0.5 mmol) obtained in Example 7 was dissolved in chloroform (20 ml). At room temperature, mCPBA (310 mg, 1.25 mmol) was added to the solution, and the solution was stirred for 18 hours. The reaction was stopped by addition of a sodium sulfate aqueous solution to the reaction solution, and extraction was carried out using ethyl acetate. An obtained organic phase was washed with a saturated sodium hydrogencarbonate aqueous solution, water, and a saturated saline solution, and dried using anhydrous magnesium sulfate. Following filtration of the solution, the filtrate was concentrated under reduced pressure, thereby obtaining the subject thiazole compound (1C-161) as a white solid (230 mg, quant.).

### Example 9

### 5-chloro-2-{[2-ethoxy-5,6-dichlorophenyl][dideuterium(methanesulfonyl)]}-1,3-thiazole (Compound 1C-130)

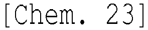

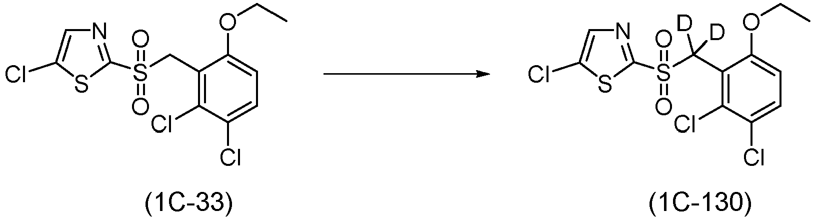

In an eggplant flask, 5-chloro-2-[(2-ethoxy-5,6-dichlorophenyl)(methanesulfonyl)]-1,3-thiazole (1C-33; 3 g, 7.76 mmol) was dissolved in THF (20 ml) and D₂O (20 ml) in a nitrogen atmosphere. Calcium vanadate apatite (80 mg) was added to the solution at room temperature, and then, the solution was stirred at 50°C for 23 hours. After the reaction solution was made to have room temperature again, the reaction was stopped by addition of water, and extraction was carried out using ethyl acetate. An obtained organic phase was washed with a saturated saline solution, and dried using anhydrous magnesium sulfate. Following filtration of the obtained solution, the filtrate was concentrated under reduced pressure, thereby obtaining the subject thiazole compound (1C-130) as a white solid (3.1 g, quant.).

### Example 10

### Synthesis of 5-chloro-2-{[2-(2,2,3,3-tetrafluoropropoxy)-6-fluorophenyl][ethanesulfonyl]-1,3-thiazole (Compound 1C-148)

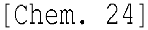

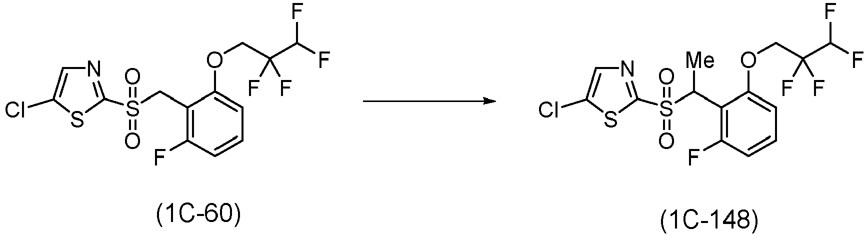

In an eggplant flask, 5-chloro-2-{[2-(2,2,3,3-tetrafluoropropoxy)-6-fluorophenyl][methanesulfonyl]}-1,3-thiazole (1C-60; 250 mg, 0.536 mmol) was dissolved in THF (5.36 ml). NaH (47 mg, 1.18 mmol) and CH₃I (152 mg, 1.07 mmol) were added to the solution under ice cooling, and the solution was stirred for 15 minutes. The reaction was stopped by addition of a saturated ammonium chloride solution to the reaction solution, and extraction was carried out using ethyl acetate. The obtained ethyl acetate solution was washed with water, and washed with a saturated saline solution. An obtained organic phase was dried using anhydrous magnesium sulfate. Following filtration of the obtained solution, the filtrate was concentrated under reduced pressure, and a crude product was purified by silica gel column chromatography (ethyl acetate:hexane=3:1), thereby obtaining the subject thiazole compound (1C-148) as a white oil (200 mg, 78%).

The compounds of the present invention listed in Tables 1-1 to 1-5, Tables 2-1 to 2-4, and Table 3 were produced by any one of the methods of Examples described above.

The symbols appearing in this specification stand for the respective groups, as indicated below.

H: hydrogen, D: heavy hydrogen, F: fluorine, Cl: chlorine, Br: bromine, Me: methyl, OMe: methoxy, OEt: ethoxy, OnPr: normal-propoxy, OiPr: isopropoxy, OnBu: n-butoxy, OCHF₂: difluoromethoxy, OCF₃:trifluoromethoxy, OCH₂CHF₂: difluoroethoxy, OCF₂CHF₂: tetrafluoroethoxy, OCH₂CH₂CF₃: trifluoropropoxy, OCH₂OMe: methoxymethoxy, OBn: benzyloxy, OH: hydroxyl group, OCOMe: acetyloxy, OPh: phenoxy, CN: cyano, CF₃: trifluoromethyl, NMe₂: dimethylamino, O: oxygen atom

**[Table 1-1]**

| | | | | | | | | | |
|---|---|---|---|---|---|---|---|---|---|
| 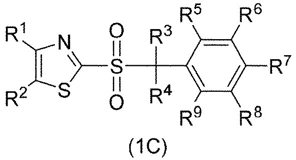 | | | | | | | | | |

| No. | R¹ | R² | R³ | R⁴ | R⁵ | R⁶ | R⁷ | R⁸ | R⁹ |
|---|---|---|---|---|---|---|---|---|---|
| 1C-1 | H | H | H | H | F | H | OMe | H | H |
| 1C-2 | H | Cl | H | H | OMe | H | H | H | H |
| 1C-3 | H | Cl | H | H | OMe | H | F | H | H |
| 1C-4 | H | Cl | H | H | OMe | H | Cl | H | H |
| 1C-5 | H | Cl | H | H | OMe | H | H | H | F |
| 1C-6 | H | Cl | H | H | OMe | H | H | H | Cl |
| 1C-7 | H | Cl | H | H | OMe | H | H | H | CF₃ |
| 1C-8 | H | Cl | H | H | OEt | H | H | H | H |
| 1C-9 | H | Cl | H | H | OEt | F | H | H | H |
| 1C-10 | H | Cl | H | H | OEt | Cl | H | H | H |
| 1C-11 | H | Cl | H | H | OEt | Me | H | H | H |
| 1C-12 | H | Cl | H | H | OEt | H | F | H | H |
| 1C-13 | H | Cl | H | H | OEt | H | Me | H | H |
| 1C-14 | H | Cl | H | H | OEt | H | CF₃ | H | H |
| 1C-15 | H | Cl | H | H | OEt | H | H | F | H |
| 1C-16 | H | Cl | H | H | OEt | H | H | Cl | H |
| 1C-17 | H | Cl | H | H | OEt | H | H | Br | H |
| 1C-18 | H | Cl | H | H | OEt | H | H | Me | H |
| 1C-19 | H | Cl | H | H | OEt | H | H | H | F |
| 1C-20 | H | Cl | H | H | OEt | H | H | H | Cl |
| 1C-21 | H | Cl | H | H | OEt | F | H | F | H |
| 1C-22 | H | Cl | H | H | OEt | Cl | H | F | H |
| 1C-23 | H | Cl | H | H | OEt | Cl | H | Cl | H |
| 1C-24 | H | Cl | H | H | OEt | Br | H | Cl | H |
| 1C-25 | H | Cl | H | H | OEt | Me | H | F | H |
| 1C-26 | H | Cl | H | H | OEt | Me | H | Cl | H |
| 1C-27 | H | Cl | H | H | OEt | F | H | H | F |
| 1C-28 | H | Cl | H | H | OEt | Me | H | H | Me |
| 1C-29 | H | Cl | H | H | OEt | H | F | F | H |
| 1C-30 | H | Cl | H | H | OEt | H | Me | Cl | H |
| 1C-31 | H | Cl | H | H | OEt | H | F | H | F |
| 1C-32 | H | Cl | H | H | OEt | H | H | Cl | F |
| 1C-33 | H | Cl | H | H | OEt | H | H | Cl | Cl |
| 1C-34 | H | Cl | H | H | OEt | Cl | H | Cl | Cl |
| 1C-35 | H | Cl | H | H | OEt | H | Me | Cl | Me |
| 1C-36 | H | Cl | H | H | O*i*-Pr | H | H | H | H |
| 1C-37 | H | Cl | H | H | OCH₂OMe | H | H | H | F |
| 1C-38 | H | Cl | H | H | OCHF₂ | H | H | H | H |

**[Table 1-2]**

| No. | R¹ | R² | R³ | R⁴ | R⁵ | R⁶ | R⁷ | R⁸ | R⁹ |
|---|---|---|---|---|---|---|---|---|---|
| 1C-39 | H | Cl | H | H | OCHF₂ | H | H | Cl | H |
| 1C-40 | H | Cl | H | H | OCHF₂ | H | H | Br | H |
| 1C-41 | H | Cl | H | H | OCHF₂ | H | H | H | F |
| 1C-42 | H | Cl | H | H | OCHF₂ | H | H | H | Cl |
| 1C-43 | H | Cl | H | H | OCHF₂ | F | H | H | F |
| 1C-44 | H | Cl | H | H | OCHF₂ | H | F | H | F |
| 1C-45 | H | Cl | H | H | OCHF₂ | H | H | Cl | F |
| 1C-46 | H | Cl | H | H | OCF₃ | H | H | H | H |
| 1C-47 | H | Cl | H | H | OCF₃ | H | H | Br | H |
| 1C-48 | H | Cl | H | H | OCF₃ | H | H | H | F |
| 1C-49 | H | Cl | H | H | OCF₃ | H | H | H | Cl |
| 1C-50 | H | Cl | H | H | OCH₂CHF₂ | H | H | H | F |
| 1C-51 | H | Cl | H | H | OCH₂CHF₂ | H | H | H | Cl |
| 1C-52 | H | Cl | H | H | OCH₂CHF₂ | H | H | H | CF₃ |
| 1C-53 | H | Cl | H | H | OCH₂CF₃ | H | H | H | H |
| 1C-54 | H | Cl | H | H | OCH₂CF₃ | H | H | H | F |
| 1C-55 | H | Cl | H | H | OCH₂CF₃ | H | H | H | Cl |
| 1C-56 | H | Cl | H | H | OCH₂CF₃ | H | H | H | CF₃ |
| 1C-57 | H | Cl | H | H | OCF₂CHF₂ | H | H | H | H |
| 1C-58 | H | Cl | H | H | OCH₂CH₂CF₃ | H | H | H | F |
| 1C-59 | H | Cl | H | H | OCH₂CH₂CF₃ | H | H | H | Cl |
| 1C-60 | H | Cl | H | H | OCH₂CF₂CHF₂ | H | H | H | F |
| 1C-61 | H | Cl | H | H | OCH₂CF₂CF₃ | H | H | H | F |
| 1C-62 | H | Cl | H | H | OCH₂CF₂CF₃ | H | H | H | Cl |
| 1C-63 | H | Cl | H | H | OCH₂CH₂CH₂CF₃ | H | H | H | F |
| 1C-64 | H | Cl | H | H | OCF₂COOEt | H | H | H | Cl |
| 1C-65 | H | Cl | H | H | H | OMe | H | H | H |
| 1C-66 | H | Cl | H | H | Cl | OMe | H | H | H |
| 1C-67 | H | Cl | H | H | Me | OMe | H | H | H |
| 1C-68 | H | Cl | H | H | H | OMe | Me | H | H |
| 1C-69 | H | Cl | H | H | H | OMe | H | H | F |
| 1C-70 | H | Cl | H | H | H | OMe | H | H | Cl |
| 1C-71 | H | Cl | H | H | F | OMe | H | H | Cl |
| 1C-72 | H | Cl | H | H | Cl | OMe | H | H | F |
| 1C-73 | H | Cl | H | H | Cl | OMe | H | H | Cl |
| 1C-74 | H | Cl | H | H | F | OMe | F | F | H |
| 1C-75 | H | Cl | H | H | F | OEt | H | H | H |
| 1C-76 | H | Cl | H | H | H | OEt | F | H | H |
| 1C-77 | H | Cl | H | H | H | OEt | Cl | H | H |
| 1C-78 | H | Cl | H | H | H | OEt | H | Cl | H |
| 1C-79 | H | Cl | H | H | H | OEt | H | CF₃ | H |
| 1C-80 | H | Cl | H | H | H | OEt | H | H | Cl |

**[Table 1-3]**

| No. | R¹ | R² | R³ | R⁴ | R⁵ | R⁶ | R⁷ | R⁸ | R⁹ |
|---|---|---|---|---|---|---|---|---|---|
| 1C-81 | H | Cl | H | H | F | OEt | F | H | H |
| 1C-82 | H | Cl | H | H | F | OEt | H | H | F |
| 1C-83 | H | Cl | H | H | H | OEt | F | F | H |
| 1C-84 | H | Cl | H | H | H | OCHF₂ | H | H | H |
| 1C-85 | H | Cl | H | H | H | OCF₃ | H | H | H |
| 1C-86 | H | Cl | H | H | H | OPh | F | H | H |
| 1C-87 | H | Cl | H | H | H | H | OMe | H | H |
| 1C-88 | H | Cl | H | H | F | H | OMe | H | H |
| 1C-89 | H | Cl | H | H | H | Cl | OMe | H | H |
| 1C-90 | H | Cl | H | H | F | H | OMe | H | F |
| 1C-91 | H | Cl | H | H | Cl | H | OMe | H | Cl |
| 1C-92 | H | Cl | H | H | Cl | H | OEt | H | H |
| 1C-93 | H | Cl | H | H | Me | H | OEt | H | H |
| 1C-94 | H | Cl | H | H | CF₃ | H | OEt | H | H |
| 1C-95 | H | Cl | H | H | H | F | OEt | H | H |
| 1C-96 | H | Cl | H | H | H | Cl | OEt | H | H |
| 1C-97 | H | Cl | H | H | H | Me | OEt | H | H |
| 1C-98 | H | Cl | H | H | H | CF₃ | OEt | H | H |
| 1C-99 | H | Cl | H | H | F | F | OEt | H | H |
| 1C-100 | H | Cl | H | H | Cl | Cl | OEt | H | H |
| 1C-101 | H | Cl | H | H | F | H | OEt | F | H |
| 1C-102 | H | Cl | H | H | Cl | H | OEt | Cl | H |
| 1C-103 | H | Cl | H | H | F | H | OEt | H | F |
| 1C-104 | H | Cl | H | H | H | F | OEt | F | H |
| 1C-105 | H | Cl | H | H | H | Cl | OEt | F | H |
| 1C-106 | H | Cl | H | H | H | Cl | OEt | Cl | H |
| 1C-107 | H | Cl | H | H | H | Me | OEt | Me | H |
| 1C-108 | H | Cl | H | H | F | F | OEt | F | F |
| 1C-109 | H | Cl | H | H | F | H | O*n*-Pr | H | F |
| 1C-110 | H | Cl | H | H | Cl | H | O*n*-Pr | H | Cl |
| 1C-111 | H | Cl | H | H | F | H | O*i*-Pr | H | F |
| 1C-112 | H | Cl | H | H | F | H | O*n*-Bu | H | F |
| 1C-113 | H | Cl | H | H | Cl | H | O*n*-Bu | H | Cl |
| 1C-114 | H | Cl | H | H | H | H | OCHF₂ | H | H |
| 1C-115 | H | Cl | H | H | H | H | OCF₃ | H | H |
| 1C-116 | H | Cl | H | H | H | H | OCF₂CHF₂ | H | H |
| 1C-117 | H | Cl | H | H | F | H | OCH₂CH₂CF₃ | H | F |
| 1C-118 | H | Cl | H | H | F | H | OCH₂OMe | H | F |
| 1C-119 | H | Cl | H | H | Cl | H | OCH₂OMe | H | Cl |
| 1C-120 | H | Cl | H | H | H | H | OBn | H | H |

**[Table 1-4]**

| No. | R¹ | R² | R³ | R⁴ | R⁵ | R⁶ | R⁷ | R⁸ | R⁹ |
|---|---|---|---|---|---|---|---|---|---|
| 1C-121 | H | Cl | H | H | F | H | OH | H | F |
| 1C-122 | H | Cl | H | H | Cl | H | OH | H | Cl |
| 1C-123 | H | Cl | H | H | F | H | OCOMe | H | F |
| 1C-124 | H | Cl | H | H | OMe | OMe | H | H | H |
| 1C-125 | H | Cl | H | H | OMe | H | OMe | H | H |
| 1C-126 | H | Cl | H | H | OMe | H | H | OMe | H |
| 1C-127 | H | Cl | H | H | OMe | H | H | H | OMe |
| 1C-128 | H | Cl | H | H | H | OMe | OMe | OMe | H |
| 1C-129 | H | Cl | H | H | OCH₂CHF₂ | H | H | H | OCH₂CHF₂ |
| 1C-130 | H | Cl | D | D | OEt | H | H | Cl | Cl |
| 1C-131 | H | Cl | D | D | OCHF₂ | H | H | H | F |
| 1C-132 | H | Cl | D | D | OCHF₂ | H | H | H | Cl |
| 1C-133 | H | Cl | D | D | OCF₃ | H | H | H | Cl |
| 1C-134 | H | Cl | D | D | OCH₂CF₃ | H | H | H | H |
| 1C-135 | H | Cl | D | D | OCH₂CF₃ | H | H | H | Cl |
| 1C-136 | H | Cl | D | D | F | OMe | H | H | Cl |
| 1C-137 | H | Cl | D | D | Cl | OMe | H | H | Cl |
| 1C-138 | H | Cl | F | F | OEt | H | H | Cl | H |
| 1C-139 | F | Cl | F | F | OEt | H | H | Cl | H |
| 1C-140 | H | Cl | Me | H | OCHF₂ | H | H | H | F |
| 1C-141 | H | Cl | Me | Me | OCHF₂ | H | H | H | F |
| 1C-142 | H | Cl | Me | H | OCH₂CHF₂ | H | H | H | F |
| 1C-143 | H | Cl | Me | Me | OCH₂CHF₂ | H | H | H | F |
| 1C-144 | H | Cl | Me | H | OCH₂CHF₂ | H | H | H | OCH₂CHF₂ |
| 1C-145 | H | Cl | COOMe | H | OCHF₂ | H | H | H | F |
| 1C-146 | H | Cl | COMe | H | OCHF₂ | H | H | H | F |
| 1C-147 | H | Cl | COMe | COMe | OCHF₂ | H | H | H | F |
| 1C-148 | H | Cl | Me | H | OCH₂CF₂CHF₂ | H | H | H | F |
| 1C-149 | H | Cl | Me | H | OCH₂CF₂CF₃ | H | H | H | F |
| 1C-150 | H | Cl | Me | H | OCH₂CF₂CF₃ | H | H | H | Cl |
| 1C-151 | H | Br | H | H | OMe | H | H | H | Cl |
| 1C-152 | H | Br | H | H | OCHF₂ | H | H | H | H |
| 1C-153 | H | Br | H | H | OCHF₂ | H | H | H | F |
| 1C-154 | H | Br | H | H | OCHF₂ | H | H | H | Cl |
| 1C-155 | H | Br | H | H | OCHF₂ | F | H | H | F |
| 1C-156 | H | Br | H | H | OCHF₂ | H | F | H | F |
| 1C-157 | H | Br | H | H | OCHF₂ | H | H | Cl | F |
| 1C-158 | H | Br | H | H | OCF₃ | H | H | H | F |
| 1C-159 | H | Br | H | H | OCF₃ | H | H | H | Cl |
| 1C-160 | H | Br | H | H | OCH₂CHF₂ | H | H | H | CF₃ |

**[Table 1-5]**

| No. | R¹ | R² | R³ | R⁴ | R⁵ | R⁶ | R⁷ | R⁸ | R⁹ |
|---|---|---|---|---|---|---|---|---|---|
| 1C-161 | H | Br | H | H | OCH₂CF₃ | H | H | H | Cl |
| 1C-162 | H | Br | H | H | OCH₂CF₃ | H | H | H | CF₃ |
| 1C-163 | H | Br | H | H | OCF₂CHF₂ | H | H | H | H |
| 1C-164 | H | Br | H | H | OCH₂CF₂CHF₂ | H | H | H | F |
| 1C-165 | H | Br | Me | H | OCH₂CF₂CHF₂ | H | H | H | F |
| 1C-166 | Me | Br | H | H | F | H | OMe | H | F |
| 1C-167 | H | CF₃ | H | H | F | OMe | H | H | Cl |
| 1C-168 | H | Cl | H | H | H | H | SO₂Me | H | H |
| 1C-169 | H | Cl | H | H | N(Me)CH₂CCH | H | H | H | F |
| 1C-170 | H | Cl | H | H | Cl | NMe₂ | H | Cl | H |
| 1C-171 | H | Cl | H | H | H | CN | H | H | H |
| 1C-172 | H | Cl | H | H | H | H | CN | H | H |
| 1C-173 | H | Cl | H | H | OCOCH₂Cl | H | H | H | F |
| 1C-174 | H | Cl | H | H | SO₂Et | H | H | H | F |
| 1C-175 | H | Cl | H | H | Cl | H | OCHF₂ | H | Cl |
| 1C-176 | H | Cl | H | H | F | H | OCHF₂ | H | F |
| 1C-177 | H | Cl | H | H | Si(CH₃)₃ | H | H | H | F |
| 1C-178 | H | Cl | H | H | OSi(CH₃)₂C(CH₃)₃ | H | H | H | F |
| 1C-179 | H | Cl | H | H | OCH₂F | H | H | H | F |
| 1C-180 | H | Cl | H | H | F | OCHF₂ | H | H | Cl |
| 1C-181 | H | Cl | H | H | OCH₂COOMe | H | H | H | F |
| 1C-182 | H | Cl | H | H | OCHFCOOEt | H | H | H | F |
| 1C-183 | H | Cl | H | H | OCH₂COOH | H | H | H | F |
| 1C-184 | H | Cl | H | H | OH | H | H | H | F |
| 1C-185 | H | Cl | H | H | OSO₂CF₃ | H | H | H | F |

**[Table 2-1]**

| | | | | | | | | | |
|---|---|---|---|---|---|---|---|---|---|
| 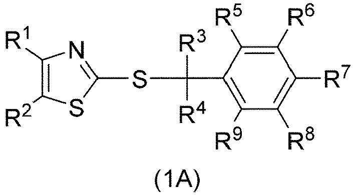 | | | | | | | | | |

| No. | R¹ | R² | R³ | R⁴ | R⁵ | R⁶ | R⁷ | R⁸ | R⁹ |
|---|---|---|---|---|---|---|---|---|---|
| 1A-1 | H | H | H | H | F | H | OMe | H | H |
| 1A-2 | H | Cl | H | H | OMe | H | H | H | H |
| 1A-3 | H | Cl | H | H | OMe | H | F | H | H |
| 1A-4 | H | Cl | H | H | OMe | H | Cl | H | H |
| 1A-5 | H | Cl | H | H | OMe | H | H | H | F |
| 1A-6 | H | Cl | H | H | OMe | H | H | H | Cl |
| 1A-7 | H | Cl | H | H | OMe | H | H | H | CF₃ |
| 1A-8 | H | Cl | H | H | OEt | H | H | H | H |
| 1A-9 | H | Cl | H | H | OEt | F | H | H | H |
| 1A-10 | H | Cl | H | H | OEt | Cl | H | H | H |
| 1A-11 | H | Cl | H | H | OEt | Me | H | H | H |
| 1A-12 | H | Cl | H | H | OEt | H | F | H | H |
| 1A-13 | H | Cl | H | H | OEt | H | Me | H | H |
| 1A-14 | H | Cl | H | H | OEt | H | CF₃ | H | H |
| 1A-15 | H | Cl | H | H | OEt | H | H | F | H |
| 1A-16 | H | Cl | H | H | OEt | H | H | Cl | H |
| 1A-17 | H | Cl | H | H | OEt | H | H | Br | H |
| 1A-18 | H | Cl | H | H | OEt | H | H | Me | H |
| 1A-19 | H | Cl | H | H | OEt | H | H | H | F |
| 1A-20 | H | Cl | H | H | OEt | H | H | H | Cl |
| 1A-21 | H | Cl | H | H | OEt | F | H | F | H |
| 1A-22 | H | Cl | H | H | OEt | Cl | H | F | H |
| 1A-23 | H | Cl | H | H | OEt | Cl | H | Cl | H |
| 1A-24 | H | Cl | H | H | OEt | Br | H | Cl | H |
| 1A-25 | H | Cl | H | H | OEt | Me | H | F | H |
| 1A-26 | H | Cl | H | H | OEt | Me | H | Cl | H |
| 1A-27 | H | Cl | H | H | OEt | F | H | H | F |
| 1A-28 | H | Cl | H | H | OEt | Me | H | H | Me |
| 1A-29 | H | Cl | H | H | OEt | H | F | F | H |
| 1A-30 | H | Cl | H | H | OEt | H | Me | Cl | H |
| 1A-31 | H | Cl | H | H | OEt | H | F | H | F |
| 1A-32 | H | Cl | H | H | OEt | H | H | Cl | F |
| 1A-33 | H | Cl | H | H | OEt | H | H | Cl | Cl |
| 1A-34 | H | Cl | H | H | OEt | Cl | H | Cl | Cl |
| 1A-35 | H | Cl | H | H | OEt | H | Me | Cl | Me |
| 1A-36 | H | Cl | H | H | O*i*-Pr | H | H | H | H |
| 1A-37 | H | Cl | H | H | OCHF₂ | H | H | H | H |
| 1A-38 | H | Cl | H | H | OCHF₂ | H | H | Cl | H |

**[Table 2-2]**

| No. | R¹ | R² | R³ | R⁴ | R⁵ | R⁶ | R⁷ | R⁸ | R⁹ |
|---|---|---|---|---|---|---|---|---|---|
| 1A-39 | H | Cl | H | H | OCHF₂ | H | H | Br | H |
| 1A-40 | H | Cl | H | H | OCHF₂ | H | H | H | F |
| 1A-41 | H | Cl | H | H | OCHF₂ | H | H | H | Cl |
| 1A-42 | H | Cl | H | H | OCHF₂ | F | H | H | F |
| 1A-43 | H | Cl | H | H | OCHF₂ | H | F | H | F |
| 1A-44 | H | Cl | H | H | OCHF₂ | H | H | Cl | F |
| 1A-45 | H | Cl | H | H | OCF₃ | H | H | H | H |
| 1A-46 | H | Cl | H | H | OCF₃ | H | H | Br | H |
| 1A-47 | H | Cl | H | H | OCF₃ | H | H | H | F |
| 1A-48 | H | Cl | H | H | OCF₃ | H | H | H | Cl |
| 1A-49 | H | Cl | H | H | OCH₂CHF₂ | H | H | H | F |
| 1A-50 | H | Cl | H | H | OCH₂CHF₂ | H | H | H | Cl |
| 1A-51 | H | Cl | H | H | OCH₂CHF₂ | H | H | H | CF₃ |
| 1A-52 | H | Cl | H | H | OCH₂CF₃ | H | H | H | H |
| 1A-53 | H | Cl | H | H | OCH₂CF₃ | H | H | H | F |
| 1A-54 | H | Cl | H | H | OCH₂CF₃ | H | H | H | Cl |
| 1A-55 | H | Cl | H | H | OCH₂CF₃ | H | H | H | CF₃ |
| 1A-56 | H | Cl | H | H | OCF₂CHF₂ | H | H | H | H |
| 1A-57 | H | Cl | H | H | OCH₂CF₂CHF₂ | H | H | H | F |
| 1A-58 | H | Cl | H | H | OCH₂CF₂CF₃ | H | H | H | F |
| 1A-59 | H | Cl | H | H | OCH₂CF₂CF₃ | H | H | H | Cl |
| 1A-60 | H | Cl | H | H | OCF₂COOEt | H | H | H | Cl |
| 1A-61 | H | Cl | H | H | H | OMe | H | H | H |
| 1A-62 | H | Cl | H | H | Cl | OMe | H | H | H |
| 1A-63 | H | Cl | H | H | Me | OMe | H | H | H |
| 1A-64 | H | Cl | H | H | H | OMe | Me | H | H |
| 1A-65 | H | Cl | H | H | H | OMe | H | H | F |
| 1A-66 | H | Cl | H | H | H | OMe | H | H | Cl |
| 1A-67 | H | Cl | H | H | F | OMe | H | H | Cl |
| 1A-68 | H | Cl | H | H | Cl | OMe | H | H | F |
| 1A-69 | H | Cl | H | H | Cl | OMe | H | H | Cl |
| 1A-70 | H | Cl | H | H | F | OMe | F | F | H |
| 1A-71 | H | Cl | H | H | F | OEt | H | H | H |
| 1A-72 | H | Cl | H | H | H | OEt | F | H | H |
| 1A-73 | H | Cl | H | H | H | OEt | Cl | H | H |
| 1A-74 | H | Cl | H | H | H | OEt | H | Cl | H |
| 1A-75 | H | Cl | H | H | H | OEt | H | CF₃ | H |
| 1A-76 | H | Cl | H | H | H | OEt | H | H | Cl |
| 1A-77 | H | Cl | H | H | F | OEt | F | H | H |
| 1A-78 | H | Cl | H | H | F | OEt | H | H | F |
| 1A-79 | H | Cl | H | H | H | OEt | F | F | H |
| 1A-80 | H | Cl | H | H | H | OCHF₂ | H | H | H |

**[Table 2-3]**

| No. | R¹ | R² | R³ | R⁴ | R⁵ | R⁶ | R⁷ | R⁸ | R⁹ |
|---|---|---|---|---|---|---|---|---|---|
| 1A-81 | H | Cl | H | H | H | OCF₃ | H | H | H |
| 1A-82 | H | Cl | H | H | H | OPh | F | H | H |
| 1A-83 | H | Cl | H | H | H | H | OMe | H | H |
| 1A-84 | H | Cl | H | H | F | H | OMe | H | H |
| 1A-85 | H | Cl | H | H | H | Cl | OMe | H | H |
| 1A-86 | H | Cl | H | H | F | H | OMe | H | F |
| 1A-87 | H | Cl | H | H | Cl | H | OMe | H | Cl |
| 1A-88 | H | Cl | H | H | Cl | H | OEt | H | H |
| 1A-89 | H | Cl | H | H | Me | H | OEt | H | H |
| 1A-90 | H | Cl | H | H | CF₃ | H | OEt | H | H |
| 1A-91 | H | Cl | H | H | H | F | OEt | H | H |
| 1A-92 | H | Cl | H | H | H | Cl | OEt | H | H |
| 1A-93 | H | Cl | H | H | H | Me | OEt | H | H |
| 1A-94 | H | Cl | H | H | H | CF₃ | OEt | H | H |
| 1A-95 | H | Cl | H | H | F | F | OEt | H | H |
| 1A-96 | H | Cl | H | H | Cl | Cl | OEt | H | H |
| 1A-97 | H | Cl | H | H | F | H | OEt | F | H |
| 1A-98 | H | Cl | H | H | Cl | H | OEt | Cl | H |
| 1A-99 | H | Cl | H | H | F | H | OEt | H | F |
| 1A-100 | H | Cl | H | H | H | F | OEt | F | H |
| 1A-101 | H | Cl | H | H | H | Cl | OEt | F | H |
| 1A-102 | H | Cl | H | H | H | Cl | OEt | Cl | H |
| 1A-103 | H | Cl | H | H | H | Me | OEt | Me | H |
| 1A-104 | H | Cl | H | H | F | F | OEt | F | F |
| 1A-105 | H | Cl | H | H | H | H | OCHF₂ | H | H |
| 1A-106 | H | Cl | H | H | H | H | OCF₃ | H | H |
| 1A-107 | H | Cl | H | H | H | H | OCF₂CHF₂ | H | H |
| 1A-108 | H | Cl | H | H | F | H | OCH₂OMe | H | F |
| 1A-109 | H | Cl | H | H | Cl | H | OCH₂OMe | H | Cl |
| 1A-110 | H | Cl | H | H | H | H | OBn | H | H |
| 1A-111 | H | Cl | H | H | OMe | OMe | H | H | H |
| 1A-112 | H | Cl | H | H | OMe | H | OMe | H | H |
| 1A-113 | H | Cl | H | H | OMe | H | H | OMe | H |
| 1A-114 | H | Cl | H | H | OMe | H | H | H | OMe |
| 1A-115 | H | Cl | H | H | H | OMe | OMe | OMe | H |
| 1A-116 | H | Cl | H | H | OCH₂CHF₂ | H | H | H | OCH₂CHF₂ |
| 1A-117 | H | Cl | Me | H | OCHF₂ | H | H | H | F |
| 1A-118 | H | Br | H | H | OMe | H | H | H | Cl |
| 1A-119 | H | Br | H | H | OCHF₂ | H | H | H | H |
| 1A-120 | H | Br | H | H | OCHF₂ | H | H | H | F |

**[Table 2-4]**

| No. | R¹ | R² | R³ | R⁴ | R⁵ | R⁶ | R⁷ | R⁸ | R⁹ |
|---|---|---|---|---|---|---|---|---|---|
| 1A-121 | H | Br | H | H | OCHF₂ | H | H | H | Cl |
| 1A-122 | H | Br | H | H | OCHF₂ | F | H | H | F |
| 1A-123 | H | Br | H | H | OCHF₂ | H | F | H | F |
| 1A-124 | H | Br | H | H | OCHF₂ | H | H | Cl | F |
| 1A-125 | H | Br | H | H | OCF₃ | H | H | H | F |
| 1A-126 | H | Br | H | H | OCF₃ | H | H | H | Cl |
| 1A-127 | H | Br | H | H | OCH₂CF₃ | H | H | H | Cl |
| 1A-128 | H | Br | H | H | OCH₂CF₃ | H | H | H | CF₃ |
| 1A-129 | H | Br | H | H | OCH₂CHF₂ | H | H | H | CF₃ |
| 1A-130 | H | Br | H | H | OCF₂CHF₂ | H | H | H | H |
| 1A-131 | H | Br | H | H | OCH₂CF₂CHF₂ | H | H | H | F |
| 1A-132 | Me | Br | H | H | F | H | OMe | H | F |
| 1A-133 | H | CF₃ | H | H | F | OMe | H | H | Cl |
| 1A-134 | H | Cl | H | H | H | H | SMe | H | H |
| 1A-135 | H | Cl | H | H | Cl | NMe₂ | H | Cl | H |
| 1A-136 | H | Cl | H | H | N(Me)CH₂CCH | H | H | H | F |
| 1A-137 | H | Cl | H | H | H | CN | H | H | H |
| 1A-138 | H | Cl | H | H | H | H | CN | H | H |
| 1A-139 | H | Cl | H | H | OCOCH₂Cl | H | H | H | F |
| 1A-140 | H | Cl | H | H | SO₂Et | H | H | H | F |
| 1A-141 | H | Cl | H | H | Cl | H | OCHF₂ | H | Cl |
| 1A-142 | H | Cl | H | H | F | H | OCHF₂ | H | F |
| 1A-143 | H | Cl | H | H | Si(CH₃)₃ | H | H | H | F |
| 1A-144 | H | Cl | H | H | OSi(CH₃)₂C(CH₃)₃ | H | H | H | F |
| 1A-145 | H | Cl | H | H | F | OCHF₂ | H | H | Cl |
| 1A-146 | H | Cl | H | H | OH | H | H | H | F |
| 1A-147 | H | Cl | H | H | OSO₂CF₃ | H | H | H | F |
| 1A-148 | H | H | H | H | H | OCF₃ | H | H | H |
| 1A-149 | H | H | H | H | OCH₂CF₃ | H | H | H | Cl |

**[Table 3]**

| | | | | | | | | | |
|---|---|---|---|---|---|---|---|---|---|
| 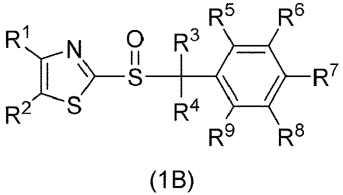 | | | | | | | | | |

| No. | R¹ | R² | R³ | R⁴ | R⁵ | R⁶ | R⁷ | R⁸ | R⁹ |
|---|---|---|---|---|---|---|---|---|---|
| 1B-1 | H | Cl | H | H | OMe | H | H | H | Cl |
| 1B-2 | H | Cl | H | H | OCHF₂ | H | H | H | H |
| 1B-3 | H | Cl | H | H | OCHF₂ | H | H | H | F |
| 1B-4 | H | Cl | H | H | OCHF₂ | H | H | H | Cl |
| 1B-5 | H | Cl | H | H | OCF₃ | H | H | H | F |
| 1B-6 | H | Cl | H | H | OCF₃ | H | H | H | Cl |
| 1B-7 | H | Cl | H | H | OCH₂CHF₂ | H | H | H | F |
| 1B-8 | H | Cl | H | H | OCH₂CHF₂ | H | H | H | CF₃ |
| 1B-9 | H | Cl | H | H | OCF₂CHF₂ | H | H | H | H |
| 1B-10 | H | Cl | H | H | OCH₂CF₃ | H | H | H | Cl |
| 1B-11 | H | Cl | H | H | OCH₂CF₃ | H | H | H | CF₃ |
| 1B-12 | H | Cl | H | H | OCH₂CF₂CHF₂ | H | H | H | F |
| 1B-13 | H | Cl | H | H | OCH₂CF₂CHF₃ | H | H | H | F |
| 1B-14 | H | Cl | H | H | OCH₂CF₂CF₃ | H | H | H | Cl |
| 1B-15 | H | Cl | H | H | OCH₂CHF₂ | H | H | H | OCH₂CHF₂ |
| 1B-16 | H | Cl | Me | H | OCHF₂ | H | H | H | F |
| 1B-17 | H | Br | H | H | OMe | H | H | H | Cl |
| 1B-18 | H | Br | H | H | OCHF₂ | H | H | H | H |
| 1B-19 | H | Br | H | H | OCHF₂ | H | H | H | F |
| 1B-20 | H | Br | H | H | OCHF₂ | H | H | H | Cl |
| 1B-21 | H | Br | H | H | OCF₃ | H | H | H | F |
| 1B-22 | H | Br | H | H | OCF₃ | H | H | H | Cl |
| 1B-23 | H | Br | H | H | OCF₂CHF₂ | H | H | H | H |
| 1B-24 | H | Br | H | H | OCH₂CF₃ | H | H | H | Cl |
| 1B-25 | H | Br | H | H | OCH₂CF₃ | H | H | H | CF₃ |
| 1B-26 | H | Br | H | H | OCH₂CF₂CHF₂ | H | H | H | F |
| 1B-27 | H | Cl | H | H | OCOCH₂Cl | H | H | H | F |
| 1B-28 | H | Cl | H | H | SO₂Et | H | H | H | F |
| 1B-29 | H | Cl | H | H | OSO₂CF₃ | H | H | H | F |

Tables 4-1 to Table 6 show the physical properties and other characteristics of the compounds. The 1H-NMR spectrum was measured using tetramethylsilane (TMS) as the standard.

**[Table 4-1]**

| No. | State | 1H-NMR(400 MHz, CDCl₃, σppm) |
|---|---|---|
| 1C-1 | WHITE SOLID | 8.08 (d, 1H), 7.11 (d, 1H), 7.15 (t, 1H), 6.66 (m, 1H), 6.56 (dd, 1H), 4.65 (s, 2H), 3.78 (s, 3H) |
| 1C-2 | PALE YELLOW SOLID | 7.84 (s, 1H), 7.33 (dt, 1H), 7.23 (dd, 1H), 6.94 (dt, 1H), 6.82 (d, 1H), 4.72 (s, 2H), 3.65 (s, 3H) |
| 1C-3 | WHITE SOLID | 7.85 (s, 1H), 7.20 (dd, 1H), 6.66 (dt, 1H), 6.55 (dd, 1H), 4.67 (s, 2H), 3.66 (s, 3H) |
| 1C-4 | WHITE SOLID | 7.86 (s, 1H), 7.16 (d, 1H), 6.95 (dd, 1H), 6.81 (d, 1H), 4.67 (s, 2H), 3.66 (s, 3H) |
| 1C-5 | PALE YELLOW OIL | 7.86 (s, 1H), 7.31 (dt, 1H), 6.72 (t, 1H), 6.65 (d, 1H), 4.77 (s, 2H), 3.69 (s, 3H) |
| 1C-6 | BEIGE OIL | 7.86 (s, 1H), 7.28 (t, 1H), 7.03 (dd, 1H), 6.78 (d, 1H), 4.96 (s, 2H), 3.69 (s, 3H) |
| 1C-7 | WHITE SOLID | 7.87 (s, 1H), 7.47 (d, 1H), 7.33 (d, 1H), 7.08 (d, 1H), 5.05 (s, 2H), 3.70 (s, 3H) |
| 1C-8 | WHITE SOLID | 7.83 (s, 1H), 7.33-7.22 (m, 2H), 6.93 (t, 1H), 6.79 (d, 1H), 4.73 (s, 2H), 3.87 (dd, 2H), 1.31 (t, 3H) |
| 1C-9 | PALE YELLOW SOLID | 7.87 (s, 1H), 7.10 (m, 1H), 6.99-6.93 (m, 2H), 4.75 (s, 2H), 4.17 (m, 2H), 1.36 (dt, 3H) |
| 1C-10 | PALE YELLOW SOLID | 7.88 (s, 1H), 7.39 (dd, 1H), 7.20 (dd, 1H), 7.04 (t, 1H), 4.73 (s, 2H), 4.09 (q, 2H), 1.40 (t, 3H) |
| 1C-11 | COLORLESS OIL | 7.87 (s, 1H), 7.19 (dd, 1H), 7.07 (dd, 1H), 6.99 (t, 1H), 4.75 (s, 2H), 3.86 (q, 2H), 2.25 (s, 3H), 1.36 (t, 3H) |
| 1C-12 | WHITE SOLID | 7.83 (s, 1H), 7.20 (dd, 1H), 6.65 (dt, 1H), 6.53 (dd, 1H), 4.67 (s, 2H), 3.85 (q, 2H), 1.33 (t, 3H) |
| 1C-13 | WHITE SOLID | 7.82 (s, 1H), 7.09 (d, 1H), 6.74 (d, 1H), 6.60 (s, 1H), 4.68 (s, 2H), 3.84 (q, 2H), 2.33 (s, 3H), 1.30 (t, 3H) |
| 1C-14 | WHITE SOLID | 7.84 (s, 1H), 7.37 (d, 1H), 7.20 (dd, 1H), 7.02 (s, 1H), 4.76 (s, 2H), 3.95 (q, 2H), 1.36 (t, 3H) |
| 1C-15 | WHITE SOLID | 7.83 (s, 1H), 7.04-6.98 (m, 2H), 6.73 (ddd, 1H), 4.70 (s, 2H), 3.85 (q, 2H), 1.31 (t, 3H) |
| 1C-16 | WHITE SOLID | 7.84 (s, 1H), 7.26 (dd, 1H), 7.20 (d, 1H), 6.73 (d, 1H), 4.68 (s, 2H), 3.86 (q, 2H), 1.32 (t, 3H) |
| 1C-17 | WHITE SOLID | 7.85 (s, 1H), 7.40 (dd, 1H), 7.31 (d, 1H), 6.68 (d, 1H), 4.67 (s, 2H), 3.87 (q, 2H), 1.32 (t, 3H) |
| 1C-18 | WHITE SOLID | 7.82 (s, 1H), 7.09 (dd, 1H), 7.05 (d, 1H), 6.67 (d, 1H), 4.67 (s, 2H), 3.81 (q, 2H), 2.26 (s, 3H), 1.28 (t, 3H) |
| 1C-19 | WHITE SOLID | 7.85 (s, 1H), 7.29 (m, 1H), 6.70 (t, 1H), 6.63 (d, 1H), 4.78 (s, 2H), 3.92 (q, 2H), 1.32 (t, 3H) |
| 1C-20 | PALE YELLOW OIL | 7.85 (s, 1H), 7.25 (dd, 1H), 6.99 (dd, 1H), 6.77 (d, 1H), 4.97 (s, 2H), 3.94 (q, 2H), 1.33 (t, 3H) |
| 1C-21 | WHITE SOLID | 7.88 (s, 1H), 6.88 (ddd, 1H), 6.79 (ddd, 1H), 4.72 (s, 2H), 4.11 (dq, 2H), 1.36 (dt, 3H) |
| 1C-22 | WHITE SOLID | 7.89 (s, 1H), 7.16 (dd, 1H), 6.99 (dd, 1H), 4.70 (s, 2H), 4.05 (q, 2H), 1.40 (t, 3H) |

**[Table 4-2]**

| No. | State | 1H-NMR(400 MHz, CDCl₃, σppm) |
|---|---|---|
| 1C-23 | WHITE SOLID | 7.89 (s, 1H), 7.40 (s, 1H), 7.19 (d, 1H), 4.68 (s, 2H), 4.08 (q, 2H), 1.40 (t, 3H) |
| 1C-24 | WHITE SOLID | 7.89 (s, 1H), 7.57 (d, 1H), 7.25 (d, 1H), 4.68 (s, 2H), 4.05 (q, 2H), 1.40 (t, 3H) |
| 1C-25 | WHITE SOLID | 7.88 (s, 1H), 6.91 (dd, 1H), 6.83 (dd, 1H), 4.67 (s, 2H), 3.83 (q, 2H), 2.25 (s, 3H), 1.37 (t, 3H) |
| 1C-26 | WHITE SOLID | 7.88 (s, 1H), 7.17 (d, 1H), 7.06 (d, 1H), 4.65 (s, 2H), 3.85 (q, 2H), 2.24 (s, 3H), 1.37 (t, 3H) |
| 1C-27 | COLORLESS OIL | 7.89 (s, 1H), 7.08 (ddd, 1H), 6.71 (ddd, 1H), 4.81 (s, 2H), 4.26 (m, 2H), 1.37 (dt, 3H) |
| 1C-28 | COLORLESS OIL | 7.84 (s, 1H), 7.08 (d, 1H), 6.92 (d, 1H), 4.74 (s, 2H), 3.71 (q, 2H), 2.42 (s, 3H), 2.15 (s, 3H), 1.28 (t, 3H) |
| 1C-29 | WHITE SOLID | 7.84 (s, 1H), 7.12 (dd, 1H), 6.62 (m, 1H), 4.65 (s, 2H), 3.83 (q, 2H), 1.32 (t, 3H) |
| 1C-30 | WHITE SOLID | 7.84 (s, 1H), 7.17 (s, 1H), 6.66 (s, 1H), 4.64 (s, 2H), 3.84 (q, 2H), 2.34 (s, 3H), 1.30 (t, 3H) |
| 1C-31 | PALE YELLOW OIL | 7.85 (s, 1H), 6.46 (dt, 1H), 6.40 (dt, 1H), 4.71 (s, 2H), 3.90 (q, 2H), 1.33 (t, 3H) |
| 1C-32 | WHITE SOLID | 7.86 (s, 1H), 7.34 (t, 1H), 6.60 (d, 1H), 4.78 (s, 2H), 3.93 (q, 2H), 1.33 (t, 3H) |
| 1C-33 | WHITE SOLID | 7.86 (s, 1H), 7.42 (d, 1H), 6.75 (d, 1H), 5.00 (s, 2H), 3.95 (q, 2H), 1.34 (t, 3H) |
| 1C-34 | WHITE SOLID | 7.88 (s, 1H), 7.55 (s, 1H), 4.99 (s, 2H), 4.13 (q, 2H), 1.38 (t, 3H) |
| 1C-35 | WHITE SOLID | 7.83 (s, 1H), 6.55 (s, 1H), 4.85 (s, 2H), 3.78 (q, 2H), 2.45 (s, 3H), 2.37 (s, 3H), 1.25 (t, 3H) |
| 1C-36 | YELLOW OIL | 7.83 (s, 1H), 7.32-7.22 (m, 2H), 6.90 (dt, 1H), 6.80 (d, 1H), 4.71 (s, 2H), 4.46 (m, 1H), 1.22 (m, 6H) |
| 1C-37 | WHITE SOLID | 7.87 (s, 1H), 7.30 (m, 1H), 6.96 (d, 1H), 6.73 (t, 1H), 5.12 (s, 2H), 4.81 (s, 2H), 3.48 (s, 3H) |
| 1C-38 | WHITE SOLID | 7.87 (s, 1H), 7.41 (m, 2H), 7.32 (m, 2H), 6.47 (t, 1H), 4.75 (s, 2H) |
| 1C-39 | WHITE SOLID | 7.89 (s, 1H), 7.39-7.32 (m, 2H), 7.12 (d, 1H), 6.45 (t, 1H), 4.70 (s, 2H) |
| 1C-40 | WHITE SOLID | 7.89 (s, 1H), 7.52 (dd, 1H), 7.44 (d, 1H), 7.06 (d, 1H), 6.45 (t, 1H), 4.69 (s, 2H) |
| 1C-41 | WHITE SOLID | 7.87 (s, 1H), 7.40 (dd, 1H), 6.99 (m, 2H), 6.51 (t, 1H), 4.80 (s, 2H) |
| 1C-42 | COLORLESS OIL | 7.88 (s, 1H), 7.36 (t, 1H), 7.28 (t, 1H), 7.14 (d, 1H), 6.52 (t, 1H), 4.98 (s, 2H) |
| 1C-43 | WHITE OIL | 7.89 (s, 1H), 7.23 (m, 1H), 7.02 (m, 1H), 6.62 (t, 1H), 4.82 (s, 2H) |
| 1C-44 | COLORLESS OIL | 7.88 (s, 1H), 6.76 (m, 2H), 6.50 (t, 1H), 4.73 (s, 2H) |
| 1C-45 | WHITE SOLID | 7.89 (s, 1H), 7.46 (t, 1H), 6.99 (d, 1H), 6.50 (t, 1H), 4.80 (s, 2H) |
| 1C-46 | WHITE SOLID | 7.86 (s, 1H), 7.42 (m, 2H), 7.29 (m, 1H), 7.23 (m, 1H), 4.73 (s, 2H) |
| 1C-47 | WHITE SOLID | 7.88 (s, 1H), 7.54 (m, 2H), 7.13 (d, 1H), 4.67 (s, 2H) |
| 1C-48 | WHITE SOLID | 7.88 (s, 1H), 7.44 (m, 1H), 7.08 (m, 2H), 4.79 (d, 2H) |
| 1C-49 | WHITE SOLID | 7.88 (s, 1H), 7.38 (m, 2H), 7.22 (m, 1H), 4.97 (s, 2H) |
| 1C-50 | COLORLESS OIL | 7.86 (s, 1H), 7.35 (dd, 1H), 6.78 (t, 1H), 6.71 (d, 1H), 6.09 (tt, 1H), 4.79 (s, 2H), 4.18 (dt, 2H) |
| 1C-51 | WHITE SOLID | 7.86 (s, 1H), 7.31 (t, 1H), 7.08 (d, 1H), 6.84 (d, 1H), 6.13 (tt, 1H), 4.97 (s, 2H), 4.21 (dt, 2H) |

**[Table 4-3]**

| No. | State | 1H-NMR(400 MHz, CDCl₃, σppm) |
|---|---|---|
| 1C-52 | WHITE SOLID | 7.86 (s, 1H), 7.53 (t, 1H), 7.41 (d, 1H), 7.17 (d, 1H), 6.18 (tt, 1H), 5.07 (s, 2H), 4.27 (dt, 2H) |
| 1C-53 | WHITE SOLID | 7.84 (s, 1H), 7.38 (m, 1H), 7.29 (m, 1H), 7.06 (t, 1H), 6.87 (d, 1H), 4.75 (s, 2H), 4.32 (q, 2H) |
| 1C-54 | WHITE SOLID | 7.85 (s, 1H), 7.37 (dd, 1H), 6.84 (t, 1H), 6.73 (d, 1H), 4.80 (s, 2H), 4.36 (q, 2H) |
| 1C-55 | WHITE SOLID | 7.85 (s, 1H), 7.33 (t, 1H), 7.14 (dd, 1H), 6.87 (d, 1H), 4.98 (s, 2H), 4.40 (q, 2H) |
| 1C-56 | WHITE SOLID | 7.85 (s, 1H), 7.55 (t, 1H), 7.46 (d, 1H), 7.19 (d, 1H), 5.09 (s, 2H), 4.46 (q, 2H) |
| 1C-57 | WHITE SOLID | 7.85 (s, 1H), 7.45-7.37 (m, 2H), 7.33-7.28 (m, 2H), 5.95 (tt, 1H), 4.69 (s, 2H) |
| 1C-58 | PALE YELLOW OIL | 7.86 (s, 1H), 7.33 (dd, 1H), 6.72 (m, 2H), 4.77 (s, 2H), 4.19 (t, 2H), 2.64 (m, 2H) |
| 1C-59 | YELLOW OIL | 7.86 (s, 1H), 7.30 (m, 1H), 7.05 (t, 1H), 6.83 (d, 1H), 4.95 (s, 2H), 4.21 (t, 2H), 2.67 (m, 2H) |
| 1C-60 | WHITE SOLID | 7.86 (s, 1H), 7.41-7.35 (m, 1H), 6.81 (t, 1H), 6.74 (d, 1H), 6.25 (tt, 1H), 4.77 (d, 2H), 4.41-4.35 (m, 2H) |
| 1C-61 | WHITE SOLID | 7.85 (s, 1H), 7.37 (dt, 1H), 6.83(t, 1H), 6.74 (d, 1H), 4.78 (d, 2H), 4.49-4.42 (m, 2H) |
| 1C-62 | WHITE SOLID | 7.85 (s, 1H), 7.34 (t, 1H), 7.14-7.12 (m, 1H), 6.88 (d, 1H), 4.96 (s, 2H), 4.52-4.45 (m, 2H) |
| 1C-63 | WHITE SOLID | 7.85 (s, 1H), 7.31 (m, 1H), 6.70 (m, 2H), 4.78 (s, 2H), 4.02 (t, 2H), 2.38 (m, 2H), 2.04 (m, 2H) |
| 1C-64 | MILK WHITE OIL | 7.83 (s, 1H), 7.34 (m, 2H), 7.26 (m, 1H), 4.98 (s, 2H), 4.42 (q, 2H), 1.41 (t, 3H) |
| 1C-65 | PALE YELLOW OIL | 7.86 (s, 1H), 7.23 (m, 1H), 6.88 (m, 1H), 6.75 (m, 2H), 4.59 (s, 2H), 3.76 (s, 3H) |
| 1C-66 | WHITE SOLID | 7.85 (s, 1H), 7.23 (dd, 1H), 7.00-6.95 (m, 2H), 4.87 (s, 2H), 3.89 (s, 3H) |
| 1C-67 | ORANGE OIL | 7.87 (s, 1H), 7.08 (t, 1H), 6.84 (d, 1H), 6.68 (d, 1H), 4.72 (s, 2H), 3.82 (s, 3H), 2.21 (s, 3H) |
| 1C-68 | YELLOW OIL | 7.87 (s, 1H), 7.04 (d, 1H), 6.63 (m, 2H), 4.59 (s, 2H), 3.77 (s, 3H), 2.18 (s, 3H) |
| 1C-69 | WHITE SOLID | 7.87 (s, 1H), 6.96 (t, 1H), 6.87 (ddd, 1H), 6.78 (ddd, 1H), 4.65 (m, 2H), 3.74 (s, 3H) |
| 1C-70 | WHITE SOLID | 7.87 (s, 1H), 7.25 (d, 1H), 6.92 (d, 1H), 6.87 (dd, 1H), 4.79 (s, 2H), 3.78 (s, 3H) |
| 1C-71 | WHITE SOLID | 7.87 (s, 1H), 7.16 (dd, 1H), 6.95 (t, 1H), 4.89 (d, 2H), 3.80 (s, 3H) |
| 1C-72 | COLORLESS OIL | 7.87 (s, 1H), 7.02 (t, 1H), 6.94 (dd, 1H), 4.93 (s, 2H), 3.88 (s, 3H) |
| 1C-73 | WHITE SOLID | 7.88 (s, 1H), 7.32 (d, 1H), 6.93 (d, 1H), 5.13 (s, 2H), 3.90 (s, 3H) |
| 1C-74 | WHITE SOLID | 7.89 (s, 1H), 6.87 (ddd, 1H), 4.62 (s, 2H), 4.00 (s, 3H) |
| 1C-75 | WHITE SOLID | 7.87 (s, 1H), 7.04 (m, 1H), 6.97 (dt, 1H), 6.82 (ddd, 1H), 4.70 (s, 2H), 4.07 (q, 2H), 1.43 (t, 3H) |
| 1C-76 | WHITE SOLID | 7.87 (s, 1H), 6.99 (dd, 1H), 6.85 (dd, 1H), 6.68 (ddd, 1H), 4.56 (s, 2H), 4.05 (q, 2H), 1.44 (t, 3H) |

**[Table 4-4]**

| No. | State | 1H-NMR(400 MHz, CDCl₃, σppm) |
|---|---|---|
| 1C-77 | WHITE SOLID | 7.87 (s, 1H), 7.28 (d, 1H), 6.80 (d, 1H), 6.67 (dd, 1H), 4.58 (s, 2H), 4.05 (q, 2H), 1.46 (t, 3H) |
| 1C-78 | WHITE SOLID | 7.89 (s, 1H), 6.87 (t, 1H), 6.74 (t, 1H), 6.66 (t, 1H), 4.54 (s, 2H), 3.97 (q, 2H), 1.39 (t, 3H) |
| 1C-79 | WHITE SOLID | 7.89 (s, 1H), 7.10 (s, 1H), 7.00 (s, 1H), 6.88 (s, 1H), 4.62 (s, 2H), 4.03 (q, 2H), 1.43 (t, 3H) |
| 1C-80 | WHITE SOLID | 7.66 (s, 1H), 7.24 (d, 1H), 6.91 (d, 1H), 6.84 (t, 1H), 4.77 (s, 2H), 3.99 (dd, 2H), 1.43 (t, 3H) |
| 1C-81 | WHITE SOLID | 7.87 (s, 1H), 6.94 (m, 2H), 4.64 (s, 2H), 4.15 (q, 2H), 1.35 (t, 3H) |
| 1C-82 | WHITE SOLID | 7.88 (s, 1H), 6.96 (m, 1H), 6.82 (m, 1H), 4.75 (s, 2H), 4.06 (q, 2H), 1.42 (t, 3H) |
| 1C-83 | WHITE SOLID | 7.88 (s, 1H), 6.64 (m, 2H), 4.53 (s, 2H), 4.06 (dd, 2H), 1.44 (t, 3H) |
| 1C-84 | WHITE SOLID | 7.87 (s, 1H), 7.34 (t, 1H), 7.13 (m, 2H), 6.96 (s, 1H), 6.47 (t, 1H), 4.62 (s, 2H) |
| 1C-85 | WHITE SOLID | 7.87 (s, 1H), 7.39 (t, 1H), 7.23 (m, 2H), 7.02 (s, 1H), 4.64 (s, 2H) |
| 1C-86 | WHITE SOLID | 7.78 (s, 1H), 7.36 (m, 2H), 7.15 (m, 2H), 7.02 (m, 1H), 6.91 (m, 2H), 6.80 (dd, 1H), 4.53 (s, 2H) |
| 1C-87 | WHITE SOLID | 7.86 (s, 1H), 7.12 (dt, 2H), 6.83 (dt, 2H), 4.57 (s, 2H), 3.79 (s, 3H) |
| 1C-88 | WHITE SOLID | 7.86 (s, 1H), 7.18 (t, 1H), 6.69 (dt, 1H), 6.59 (dd, 1H), 4.62 (s, 2H), 3.80 (s, 3H) |
| 1C-89 | WHITE SOLID | 7.88 (s, 1H), 7.21 (d, 1H), 7.12 (dd, 1H), 6.87 (d, 1H), 4.54 (s, 2H), 3.90 (s, 3H) |
| 1C-90 | WHITE SOLID | 7.88 (s, 1H), 6.47 (d, 2H), 4.66 (s, 2H), 3.81 (s, 3H) |
| 1C-91 | WHITE SOLID | 7.88 (s, 1H), 6.91 (s, 2H), 5.02 (s, 2H), 3.82 (s, 3H) |
| 1C-92 | WHITE SOLID | 7.86 (s, 1H), 7.29 (d, 1H), 6.89 (d, 1H), 6.80 (dd, 1H), 4.75 (s, 2H), 4.01 (q, 2H), 1.42 (t, 3H) |
| 1C-93 | WHITE SOLID | 7.87 (s, 1H), 6.97 (d, 1H), 6.73 (d, 1H), 6.65 (dd, 1H), 4.63 (s, 2H), 4.00 (q, 2H), 2.33 (s, 3H), 1.40 (t, 3H) |
| 1C-94 | WHITE SOLID | 7.85 (s, 1H), 7.52 (t, 1H), 7.18 (d, 1H), 7.07 (dd, 1H), 4.76 (s, 2H), 4.08 (q, 2H), 1.44 (t, 3H) |
| 1C-95 | WHITE SOLID | 7.87 (s, 1H), 6.93 (m, 3H), 4.55 (s, 2H), 4.09 (q, 2H), 1.46 (t, 3H) |
| 1C-96 | WHITE SOLID | 7.88 (s, 1H), 7.19 (d, 1H), 7.08 (dd, 1H), 6.84 (d, 1H), 4.53 (s, 2H), 4.11 (dt, 2H), 1.47 (t, 3H) |
| 1C-97 | WHITE SOLID | 7.86 (s, 1H), 6.94 (m, 2H), 6.71 (d, 1H), 4.52 (s, 2H), 4.01 (dd, 2H), 2.16 (s, 3H), 1. 42 (t, 3H) |
| 1C-98 | WHITE SOLID | 7.88 (s, 1H), 7.40 (dd, 1H), 7.29 (d, 1H), 6.95 (d, 1H), 4.58 (s, 2H), 4.12 (q, 2H), 1.46 (t, 3H) |
| 1C-99 | WHITE SOLID | 7.87 (s, 1H), 6.99 (dt, 1H), 6.73 (ddd, 1H), 4.64 (s, 2H), 4.12 (q, 2H), 1.46 (t, 3H) |
| 1C-100 | WHITE SOLID | 7.86 (s, 1H), 7.29 (d, 1H), 6.86 (d, 1H), 4.81 (s, 2H), 4.13 (q, 2H), 1.49 (t, 3H) |
| 1C-101 | WHITE SOLID | 7.88 (s, 1H), 7.00 (dd, 1H), 6.65 (dd, 1H), 4.59 (s, 2H), 4.07 (q, 2H), 1,47 (t, 3H) |
| 1C-102 | WHITE SOLID | 7.88 (s, 1H), 7.34 (s, 1H), 6.90 (s, 1H), 4.72 (s, 2H), 4.09 (q, 2H), 1.48 (t, 3H) |
| 1C-103 | WHITE SOLID | 7.88 (s, 1H), 6.45 (d, 2H), 4.65 (s, 2H), 4.00 (q, 2H), 1.42 (t, 3H) |

**[Table 4-5]**

| No. | State | 1H-NMR(400 MHz, CDCl₃, σppm) |
|---|---|---|
| 1C-104 | WHITE SOLID | 7.89 (s, 1H), 6.82 (m, 2H), 4.53 (s, 2H), 4.23 (q, 2H), 1.38 (t, 3H) |
| 1C-105 | WHITE SOLID | 7.89 (s, 1H), 7.04 (t, 1H), 6.96 (dd, 1H), 4.52 (s, 2H), 4.19 (dq, 2H), 1.41 (dt, 3H) |
| 1C-106 | WHITE SOLID | 7.90 (s, 1H), 7.19 (s, 2H), 4.52 (s, 2H), 4.10 (q, 2H), 1.45 (t, 3H) |
| 1C-107 | WHITE SOLID | 7.87 (s, 1H), 6.84 (s, 2H), 4.49 (s, 2H), 3.81 (q, 2H), 2.21 (s, 6H), 1.40 (t, 3H) |
| 1C-108 | WHITE SOLID | 7.89 (s, 1H), 4.63 (s, 2H), 4.29 (q, 2H), 1.41 (m, 3H) |
| 1C-109 | WHITE SOLID | 7.86 (s, 1H), 6.46 (d, 2H), 4.65 (s, 2H), 3.89 (t, 2H), 1.04 (t, 3H) |
| 1C-110 | WHITE SOLID | 7.87 (s, 1H), 6.90 (s, 2H), 5.01 (s, 2H), 3.91 (t, 2H), 1.80 (dt, 2H), 1.03 (t, 3H) |
| 1C-111 | WHITE SOLID | 7.88 (s, 1H), 6.43 (d, 2H), 4.65 (s, 2H), 4.49 (m, 1H), 1.34 (d, 6H) |
| 1C-112 | WHITE SOLID | 7.88 (s, 1H), 6.45 (d, 2H), 4.65 (s, 2H), 3.93 (t, 2H), 1.76 (ddd, 2H), 1.45 (m, 2H), 0.98 (t, 3H) |
| 1C-113 | WHITE SOLID | 7.87 (s, 1H), 6.90 (s, 2H), 5.03 (s, 2H), 3.95 (t, 2H), 1.76 (m, 2H), 1.48 (m, 2H), 0.98 (m, 3H) |
| 1C-114 | WHITE SOLID | 7.87 (s, 1H), 7.24 (d, 2H), 7.08 (d, 2H), 6.52 (t, 1H), 4.61 (s, 2H) |
| 1C-115 | WHITE SOLID | 7.87 (s, 1H), 7.28 (d, 2H), 7.18 (d, 2H), 4.63 (s, 2H) |
| 1C-116 | WHITE SOLID | 7.87 (s, 1H), 7.27 (m, 2H), 7.18 (m, 2H), 5.91 (tt, 1H), 4.63 (s, 2H) |
| 1C-117 | WHITE SOLID | 7.88 (s, 1H), 6.49 (d, 2H), 4.66 (s, 2H), 4.17 (t, 2H), 2.64 (m, 2H) |
| 1C-118 | PALE YELLOW SOLID | 7.88 (s, 1H), 6.64 (d, 2H), 5.17 (s, 2H), 4.66 (s, 2H), 3.47 (s, 3H) |
| 1C-119 | WHITE SOLID | 7.88 (s, 1H), 7.07 (s, 2H), 5.17 (s, 2H), 5.03 (s, 2H), 3.48 (s, 3H) |
| 1C-120 | WHITE SOLID | 7.85 (s, 1H), 7.42-7.32 (m, 5H), 7.12 (dt, 2H), 6.91 (dt, 2H), 5.05 (s, 2H), 4.57 (s, 2H) |
| 1C-121 | WHITE SOLID | 7.88 (s, 1H), 6.41 (d, 2H), 6.23 (s, 1H), 4.65 (s, 2H) |
| 1C-122 | WHITE SOLID | 7.88 (s, 1H), 6.86 (s, 2H), 5.01 (s, 2H) |
| 1C-123 | WHITE SOLID | 7.88 (s, 1H), 6.79 (d, 2H), 4.71 (s, 2H), 2.31 (s, 3H) |
| 1C-124 | YELLOW OIL | 7.87(s, 1H), 7.00 (t, 1H), 6.92 (dd, 1H), 6.77 (dd, 1H), 4.75 (s, 2H), 3.86 (s, 3H), 3.85 (s, 3H) |
| 1C-125 | WHITE SOLID | 7.87 (s, 1H), 7.03 (s, 1H), 6.93 (m, 2H), 4.66 (s, 2H), 2.33 (s, 3H), 2.30 (s, 3H) |
| 1C-126 | WHITE SOLID | 7.84 (s, 1H), 6.87 (dd, 1H), 6.78 (d, 1H), 6.74 (d, 1H), 4.69 (s, 2H), 3.74 (s, 3H), 3.61 (s, 3H) |
| 1C-127 | WHITE SOLID | 7.84 (s, 1H), 7.29 (t, 1H), 6.50 (d, 2H), 4.82 (s, 2H), 3.67 (s, 6H) |
| 1C-128 | BEIGE SOLID | 7.88 (s, 1H), 6.40 (s, 2H), 4.55 (s, 2H), 3.83 (s, 3H), 3.79 (s, 6H) |
| 1C-129 | PALE YELLOW SOLID | 7.82 (s, 1H), 7.34 (t, 1H), 6.59 (d, 2H), 6.06 (tt, 2H), 4.85 (s, 2H), 4.14 (dt, 4H) |
| 1C-130 | WHITE SOLID | 7.86 (s, 1H), 7.42 (d, 1H), 6.75 (d, 1H), 3.94 (q, 2H), 1.34 (t, 3H) |
| 1C-131 | WHITE SOLID | 7.88 (s, 1H), 7.39 (q, 1H), 6.99 (m, 2H), 6.51 (t, 1H) |
| 1C-132 | WHITE SOLID | 7.88 (s, 1H), 7.36 (t, 1H), 7.29 (d, 1H), 7.14 (d, 1H), 6.52 (t, 1H) |

**[Table 4-6]**

| No. | State | 1H-NMR(400 MHz, CDCl₃, σppm) |
|---|---|---|
| 1C-133 | WHITE SOLID | 7.88 (s, 1H), 7.39 (m, 2H), 7.22 (brs, 1H) |
| 1C-134 | WHITE SOLID | 7.84 (s, 1H), 7.38 (m, 1H), 7.27 (m, 1H), 7.07 (t, 1H), 6.87 (d, 1H), 4.32 (q, 2H) |
| 1C-135 | WHITE SOLID | 7.85 (s, 1H), 7.33 (t, 1H), 7.14 (dd, 1H), 6.87 (d, 1H), 4.39 (q, 2H) |
| 1C-136 | WHITE SOLID | 7.88 (s, 1H), 7.16 (dd, 1H), 6.95 (t, 1H), 3.88 (s, 3H) |
| 1C-137 | WHITE SOLID | 7.87 (s, 1H), 7.32 (d, 1H), 6.93 (d, 1H), 3.90 (s, 3H) |
| 1C-138 | WHITE SOLID | 7.95 (s, 1H), 7.47 (d, 1H), 7.42 (dd, 1H), 6.89 (d, 1H), 4.11 (m, 2H), 1.45 (m, 3H) |
| 1C-139 | PALE YELLOW SOLID | 7.97 (d, 1H), 7.50 (m, 1H), 6.91 (m, 1H), 4.11 (m, 2H), 1.43 (m, 3H) |
| 1C-140 | WHITE OIL | 7.86 (s, 1H), 7.37 (q, 1H), 6.98 (m, 2H), 6.51 (t, 1H), 5.24 (m, 1H), 1.93 (d, 3H) |
| 1C-141 | WHITE OIL | 7.83 (s, 1H), 7.36 (q, 1H), 6.98-6.88 (m, 2H), 6.45 (t, 1H), 2.15 (s, 3H), 2.14 (s, 3H) |
| 1C-142 | PALE YELLOW SOLID | 7.83 (s, 1H), 7.35-7.29 (m, 1H), 6.78 (brt, 1H), 6.72-6.64 (m, 1H), 6.13 (brt, 1H), 5.44-5.16 (m, 1H), 4.29-4.05 (m, 2H), 1.93-1.90 (m, 3H) |
| 1C-143 | LIGHT BROWN OIL | 7.80 (s, 1H), 7.33-7.28 (m, 1H), 6.73-6.65 (m, 2H), 6.15 (tt, 1H), 4.13 (dt, 2H), 2.14 (s, 6H) |
| 1C-144 | PALE YELLOW SOLID | 7.80 (s, 1H), 7.31 (t, 1H), 6.61 (d, 1H), 6.55 (d, 1H), 6.38-5.95 (m. 2H), 5.49 (q, 1H), 4.25-4.05 (m, 4H), 1.87 (d, 3H) |
| 1C-145 | ORANGE VISCOU S OIL | 7.83 (s, 1H), 7.48 (dt, 1H), 7.10-7.05 (m, 2H), 6.54 (t, 1H), 6.12 (s, 1H), 3.76 (s, 3H) |
| 1C-146 | ORANGE VISCOU S OIL | 7.79 (s, 1H), 7.51 (dt, 1H), 7.14-7.06 (m, 1H), 6.55 (t, 1H), 5.98 (s, 1H), 2.28 (s, 3H) |
| 1C-147 | WHITE SOLID | 7.79 (s, 1H), 7.47 (dt, 1H), 7.12-7.04 (m, 2H), 6.57 (dd, 1H), 2.35 (s, 3H), 2.00 (s, 3H) |
| 1C-148 | PALE YELLOW SOLID | 7.84 (s, 1H), 7.38-7.32 (m, 1H), 6.81 (brt, 1H), 6.70 (brd, 1H), 6.29 (brt, 1H), 5.30 (brs, 1H), 4.47-4.24 (m, 2H), 1.90-1.87 (m, 3H) |
| 1C-149 | YELLOW OIL | 7.83 (s, 1H), 7.35 (dt, 1H), 6.83 (t, 1H), 6.73-6.71 (m, 1H), 5.27 (brs, 1H), 4.47-4.41 (m, 2H), 1.92 (dd, 3H) |
| 1C-150 | YELLOW OIL | 7.83 (s, 1H), 7.30 (t, 1H), 7.13-7.11 (m, 1H), 6.94 (d, 1H), 5.57 (q, 1H), 4.66 (dt, 1H), 4.50 (dt, 1H), 1.91 (d, 3H) |
| 1C-151 | WHITE SOLID | 7.95 (s, 1H), 7.28 (t, 1H), 7.03 (dd, 1H), 6.78 (d, 1H), 4.96 (s, 2H), 3.67 (s, 3H) |
| 1C-152 | WHITE SOLID | 7.97 (s, 1H), 7.41 (t, 1H), 7.33 (d, 1H), 7.21 (t, 1H), 7.15 (d, 1H), 6.46 (t, 1H), 4.74 (s, 2H) |
| 1C-153 | WHITE SOLID | 7.97 (s, 1H), 7.40 (q, 1H), 6.99 (m, 2H), 6.50 (t, 1H), 4.80 (s, 2H) |
| 1C-154 | WHITE SOLID | 7.97 (s, 1H), 7.36 (t, 1H), 7.29 (d, 1H), 7.13 (d, 1H), 6.52 (t, 1H), 4.97 (s, 2H) |
| 1C-155 | WHITE SOLID | 7.98 (s, 1H), 7.23 (m, 1H), 7.02 (m, 1H), 6.62 (t, 1H), 4.81 (s, 2H) |
| 1C-156 | COLORL ESS OIL | 7.97 (s, 1H), 6.76 (m, 2H), 6.50 (t, 1H), 4.73 (s, 2H) |
| 1C-157 | WHITE SOLID | 7.98 (s, 1H), 7.46 (t, 1H), 6.99 (d, 1H), 6.50 (t, 1H), 4.79 (s, 2H) |
| 1C-158 | WHITE SOLID | 7.97 (s, 1H), 7.44 (m, 1H), 7.08 (m, 2H), 4.79 (d, 2H) |
| 1C-159 | WHITE SOLID | 7.97 (s, 1H), 7.39 (m, 2H), 7.22 (m, 1H), 4.96 (s, 2H) |
| 1C-160 | WHITE SOLID | 7.95 (s, 1H), 7.53 (t, 1H), 7.41 (d, 1H), 7.16 (d, 1H), 6.17 (tt, 1H), 5.06 (s, 2H), 4.26 (dt, 2H) |
| 1C-161 | WHITE SOLID | 7.94 (s, 1H), 7.33 (t, 1H), 7.14 (dd, 1H), 6.87 (d, 1H), 4.98 (s, 2H), 4.39 (q, 2H) |

**[Table 4-7]**

| No. | State | 1H-NMR(400 MHz, CDC1₃, σppm) |
|---|---|---|
| 1C-162 | WHITE SOLID | 7.95 (s, 1H), 7.55 (t, 1H), 7.46 (d, 1H), 7.19 (d, 1H), 5.09 (s, 2H), 4.46 (q, 2H) |
| 1C-163 | WHITE SOLID | 7.91 (s, 1H), 7.40 (m, 2H), 7.29 (m, 2H), 5.96 (tt, 1H), 4.68 (s, 2H) |
| 1C-164 | WHITE SOLID | 7.95 (s, 1H), 7.40-7.35 (m, 1H), 6.81 (t, 1H), 6.73 (d, 1H), 6.25 (tt, 1H), 4.77 (s, 2H), 4.41-4.35 (m, 2H) |
| 1C-165 | PALE YELLOW SOLID | 7.93 (s, 1H), 7.38-7.32 (m, 1H), 6.81 (brt, 1H), 6.70 (brd, 1H), 6.30 (brt, 1H), 5.30 (brs, 1H), 4.47-4.24 (m, 2H), 1.89-1.87 (m, 3H) |
| 1C-166 | WHITE SOLID | 6.47 (d, 2H), 4.64 (s, 2H), 3.81 (s, 3H), 2.51 (s, 3H) |
| 1C-167 | PALE YELLOW SOLID | 8.34 (s, 1H), 7.17 (d, 1H), 6.97 (t, 1H), 4.96 (s, 2H), 3.88 (s, 3H) |
| 1C-168 | WHITE SOLID | 7.94 (d, 2H), 7.89 (s, 1H), 7.50 (d, 2H), 4.73 (s, 2H), 3.06 (s, 3H) |
| 1C-169 | YELLOW OIL | 7.86 (s, 1H), 7.32 (m, 1H), 7.24 (m, 1H), 6.91 (m, 1H), 4.96 (s, 2H), 3.65 (s, 2H), 2.68 (s, 3H), 2.27 (s, 1H) |
| 1C-170 | WHITE SOLID | 7.75 (s, 1H), 7.05 (d, 1H), 6.92 (d, 1H), 4.64 (d, 1H), 4.34 (d, 1H), 2.79 (s, 6H) |
| 1C-171 | WHITE SOLID | 7.89 (s, 1H), 7.67 (t, 1H), 7.55-7.49 (m, 3H), 4.66 (s, 2H) |
| 1C-172 | WHITE SOLID | 7.88 (s, 1H), 7.64 (d, 2H), 7.39 (d, 2H), 4.69 (s, 2H) |
| 1C-173 | YELLOW OIL | 7.87 (s, 1H), 7.46-7.41 (m, 1H), 7.17-7.15 (m, 1H), 7.03-6.98 (m, 1H), 4.76 (s, 2H), 4.37 (s, 2H) |
| 1C-174 | COLORLESS OIL | 7.93-7.95 (m, 1H), 7.89 (s, 1H), 7.61-7.66 (m, 1H), 7.45-7.40 (m, 1H), 5.53 (d, 2H), 3.54 (q, 2H), 1.39 (t, 3H) |
| 1C-175 | WHITE SOLID | 7.89 (s, 1H), 7.18 (s, 2H), 6.55 (t, 1H), 5.06 (s, 2H) |
| 1C-176 | WHITE SOLID | 7.89 (s, 1H), 6.75 (d, 2H), 6.54 (t, 1H), 4.70 (s, 2H) |
| 1C-177 | - | - |
| 1C-178 | PALE YELLOW OIL | 7.85 (s, 1H), 7.20 (m, 1H), 6.66 (m, 2H), 4.75 (s, 2H), 0.99 (m, 9H), 0.25 (m, 6H) |
| 1C-179 | ORANGE VISCOUS OIL | 7.87 (s, 1H), 7.40-7.34 (m, 1H), 6.98 (d, 1H), 6.87 (t, 1H), 5.63 (d, 2H), 4.79 (d, 2H) |
| 1C-180 | ORANGE OIL | 7.89 (s, 1H), 7.27 (m, 2H), 6.53 (t, 1H), 4.90 (s, 2H) |
| 1C-181 | COLORLESS OIL | 7.84 (s, 1H), 7.34-7.28 (m, 1H), 6.78 (t, 1H), 6.57 (d, 1H), 4.82 (s, 2H), 4.58 (s, 2H), 3.79 (s, 3H) |
| 1C-182 | ORANGE OIL | 7.84 (s, 1H), 7.43-7.37 (m, 1H), 7.03 (d, 1H), 6.94 (t, 1H), 5.88 (d, 1H), 4.86-4.78 (m, 2H), 4.42-4.36 (m, 2H), 1.40 (t, 3H) |
| 1C-183 | PALE YELLOW SOLID | 7.87 (s, 1H), 7.36 (q, 1H), 6.80 (t, 1H), 6.66 (d, 1H), 4.88 (s, 2H), 4.68 (s, 2H) |
| 1C-184 | WHITE SOLID | 10.31 (brs, 1H), 8.30 (s, 1H), 7.25-7.19 (m, 1H), 6.69-6.63 (m, 2H), 4.76 (s, 2H) |
| 1C-185 | COLORLESS OIL | 7.89 (s, 1H), 7.55-7.49 (m, 1H), 7.26-7.19 (m, 2H), 4.80 (s, 2H) |

**[Table 5-1]**

| No. | State | 1H-NMR(400 MHz, CDC1₃, σppm) |
|---|---|---|
| 1A-1 | - | 7.71 (s, 1H), 7.28 (m, 1H), 7.22 (m, 1H), 6.61 (m, 2H), 4.42 (s, 2H), 3.78 (s, 3H) |
| 1A-3 | WHITE SOLID | - |
| 1A-4 | - | 7.47 (s, 1H), 7.19 (dd, 1H), 6.86 (m, 2H), 4.35 (s, 2H), 3.84 (s, 3H) |
| 1A-5 | - | 7.51 (s, 1H), 7.22 (dt, 1H), 6.68 (m, 2H), 4.42 (d, 2H), 3.82 (s, 3H) |
| 1A-6 | YELLOW OIL | 7.52 (s, 1H), 7.20 (t, 1H), 7.00 (dd, 1H), 6.78 (d, 1H), 4.56 (s, 2H), 3.80 (s, 3H) |
| 1A-7 | - | 7.52 (s, 1H), 7.37 (m, 1H), 7.26 (m, 1H), 7.08 (d, 1H), 4.58 (s, 2H), 3.83 (s, 3H) |
| 1A-8 | PALE YELLOW OIL | 7.47 (s, 1H), 7.31-7.22 (m, 2H), 6.87 (m, 2H), 4.41 (s, 2H), 4.08 (m, 2H), 1.42 (m, 3H) |
| 1A-9 | BROWN OIL | - |
| 1A-10 | YELLOW OIL | 7.49 (s, 1H), 7.30 (d, 1H), 7.24 (d, 1H), 6.97 (t, 1H), 4.45 (s, 2H), 4.12 (q, 2H), 1.46 (t, 3H) |
| 1A-11 | - | 7.48 (s, 1H), 7.12 (m, 2H), 6.96 (dt, 1H), 4.45 (s, 2H), 3.93 (m, 2H), 1.43 (t, 3H) |
| 1A-12 | YELLOW OIL | 7.47 (s, 1H), 7.22 (m, 1H), 6.57 (m, 2H), 4.36 (s, 2H), 4.04 (q, 2H), 1.43 (t, 3H) |
| 1A-13 | PALE YELLOW OIL | - |
| 1A-14 | YELLOW OIL | 7.48 (s, 1H), 7.39 (d, 1H), 7.13 (d, 1H), 7.05 (s, 1H), 4.42 (s, 2H), 4.12 (q, 2H), 1.45 (t, 3H) |
| 1A-15 | PALE YELLOW OIL | 7.47 (s, 1H), 7.04 (dd, 1H), 6.91 (dt, 1H), 6.77 (dd, 1H), 4.37 (s, 2H), 4.03 (q, 2H), 1.41 (t, 3H) |
| 1A-16 | - | 7.48 (s, 1H), 7.26 (d, 1H), 7.17 (dd, 1H), 6.77 (d, 1H), 4.36 (s, 2H), 4.04 (q, 2H), 1.41 (t, 3H) |
| 1A-18 | - | 7.47 (s, 1H), 7.08 (d, 1H), 7.02 (dd, 1H), 6.75 (d, 1H), 4.38 (s, 2H), 4.03 (q, 2H), 1.39 (t, 3H) |
| 1A-19 | PALE YELLOW OIL | 7.51 (s, 1H), 7.19 (dt, 1H), 6.69-6.63 (m, 2H), 4.42 (d, 2H), 4.03 (m, 2H), 1.39 (t, 3H) |
| 1A-20 | - | 7.51 (s, 1H), 7.17 (t, 1H), 6.98 (dd, 1H), 6.75 (d, 1H), 4.56 (s, 2H), 4.01 (q, 2H), 1.37 (t, 3H) |
| 1A-21 | BROWN OIL | 7.49 (s, 1H), 6.87 (m, 1H), 6.77 (m, 1H), 4.39 (s, 2H), 4.16 (m, 2H), 1.40 (dt, 3H) |
| 1A-22 | BROWN OIL | 7.75 (s, 1H), 7.19-7.02 (m, 2H), 4.42 (s, 2H), 4.09 (m, 2H), 1.46 (m, 3H) |
| 1A-24 | PALE YELLOW OIL | - |
| 1A-25 | - | 7.49 (s, 1H), 6.91 (dd, 1H), 6.80 (dd, 1H), 4.41 (s, 2H), 3.90 (m, 2H), 1.44 (m, 3H) |
| 1A-27 | PALE YELLOW OIL | 7.52 (s, 1H), 6.98 (ddd, 1H), 6.71 (dt, 1H), 4.43 (d, 2H), 4.23 (m, 2H), 1.38 (dt, 3H) |
| 1A-28 | - | 7.50 (s, 1H), 7.03 (d, 1H), 6.86 (d, 1H), 4.53 (s, 2H), 3.93 (q, 2H), 2.36 (s, 1H), 2.26 (s, 3H), 1.42 (t, 3H) |
| 1A-29 | PALE YELLOW OIL | 7.75 (s, 1H), 7.14 (dd, 1H), 6.67 (m, 1H), 4.33 (s, 2H), 4.01 (q, 2H), 1.42 (dt, 3H) |
| 1A-30 | PALE YELLOW OIL | 7.48 (s, 1H), 7.25 (s, 1H), 6.71 (s, 1H) 4.33 (s, 2H), 4.03 (q, 2H), 2.33 (s, 3H), 1.40 (t, 3H) |
| 1A-31 | PALE YELLOW OIL | 7.75 (s, 1H), 6.45-6.38 (m, 2H), 4.36 (d, 2H), 4.01 (m, 2H), 1.39 (t, 3H) |

**[Table 5-2]**

| No. | State | 1H-NMR(400 MHz, CDC1₃, σppm) |
|---|---|---|
| 1A-32 | COLORLESS OIL | 7.52 (s, 1H), 7.25 (m, 1H), 6.59 (dd, 1H), 4.42 (d, 2H), 4.02 (q, 2H), 1.38 (t, 3H) |
| 1A-33 | PALE YELLOW OIL | 7.52 (s, 1H), 7.33 (d, 1H), 6.72 (d, 1H), 4.58 (s, 2H), 3.99 (q, 2H), 1.36 (t, 3H) |
| 1A-34 | BROWN OIL | 7.53 (s, 1H), 7.48 (s, 1H), 4.64 (s, 2H), 4.10 (m, 2H), 1.42 (m, 3H) |
| 1A-35 | BROWN OIL | 7.49 (s, 1H), 6.62 (s, 1H), 4.50 (s, 2H), 3.99 (q, 2H), 2.41 (s, 3H), 2.37 (s, 3H), 1.38 (t, 3H) |
| 1A-36 | PALE YELLOW OIL | 7.47 (s, 1H), 7.30-7.21 (m, 2H), 6.90-6.82 (m, 2H), 4.60 (m, 1H), 4.39 (s, 2H), 1.35 (m, 6H) |
| 1A-37 | PALE YELLOW OIL | 7.48 (s, 1H), 7.39 (d, 1H), 7.30 (t, 1H), 7.14 (m, 2H), 6.55 (t, 1H), 4.43 (s, 2H) |
| 1A-38 | YELLOW OIL | - |
| 1A-39 | COLORLESS OIL | 7.55 (d, 1H), 7.49 (s, 1H), 7.40 (dd, 1H), 7.02 (d, 1H), 6.53 (t, 1H), 4.38 (s, 2H) |
| 1A-40 | PALE YELLOW OIL | 7.52 (s, 1H), 7.26 (m, 1H), 6.95 (m, 2H), 6.52 (t, 1H), 4.44 (s, 2H) |
| 1A-41 | PALE YELLOW OIL | 7.52 (s, 1H), 7.25 (m, 2H), 7.07 (d, 1H), 6.50 (t, 1H), 4.57 (s, 2H) |
| 1A-42 | YELLOW OIL | 7.53 (s, 1H), 7.12 (m, 1H), 6.94 (m, 1H), 6.64 (t, 1H), 4.47 (s, 2H) |
| 1A-43 | COLORLESS OIL | 7.51 (s, 1H), 6.73 (m, 2H), 6.52 (t, 1H), 4.38 (s, 2H) |
| 1A-44 | PALE YELLOW OIL | 7.52 (s, 1H), 7.33 (m, 1H), 6.93 (m, 1H), 6.50 (t, 1H), 4.44 (s, 2H) |
| 1A-45 | - | 7.49 (s, 1H), 7.44 (d, 1H), 7.38-7.20 (m, 3H), 4.45 (s, 2H) |
| 1A-46 | BROWN OIL | - |
| 1A-47 | COLORLESS OIL | 7.53 (s, 1H), 7.32 (m, 1H), 7.08 (d, 1H), 7.03 (m, 1H) , 4.45 (d, 2H) |
| 1A-48 | YELLOW OIL | 7.53 (s, 1H), 7.35 (d, 1H), 7.27 (m, 1H), 7.20 (t, 1H), 4.58 (s, 2H) |
| 1A-49 | - | 7.51 (s, 1H), 7.22 (m, 1H), 6.77 (t, 1H), 6.65 (d, 1H), 6.06 (tt, 1H), 4.43 (s, 2H), 4.21 (m, 2H) |
| 1A-50 | COLORLESS OIL | 7.52 (s, 1H), 7.21 (t, 1H), 7.08 (d, 1H), 6.76 (d, 1H), 6.05 (tt, 1H), 4.57 (s, 2H), 4.17 (dt, 2H) |
| 1A-51 | COLORLESS OIL | 7.52 (s, 1H), 7.41 (t, 1H), 7.34 (d, 1H), 7.07 (d, 1H), 6.02 (tt, 1H), 4.63 (s, 2H), 4.23 (dt, 2H) |
| 1A-52 | COLORLESS OIL | 7.47 (s, 1H), 7.34 (d, 1H), 7.28 (m, 1H), 6.98 (t, 1H), 6.85 (d, 1H), 4.42 (m, 4H) |
| 1A-53 | - | 7.51 (s, 1H), 7.25 (m, 1H), 6.80 (t, 1H), 6.65 (d, 1H), 4.44 (s, 2H), 4.38 (m, 2H) |
| 1A-54 | WHITE SOLID | 7.51 (s, 1H), 7.24 (m, 1H), 7.11 (dd, 1H), 6.76 (d, 1H), 4.57 (s, 2H), 4.36 (m, 2H) |
| 1A-55 | YELLOW OIL | 7.51 (s, 1H), 7.39 (m, 2H), 7.08 (d, 1H), 4.64 (s, 2H), 4.42 (q, 2H) |
| 1A-56 | YELLOW OIL | 7.48 (s, 1H), 7.44 (d, 1H), 7.34-7.20 (m, 3H), 5.97 (t, 1H), 4.41 (s, 2H) |
| 1A-57 | COLORLESS OIL | 7.51 (s, 1H), 7.29-7.24 (m, 1H), 6.80 (t, 1H), 6.66 (d, 1H), 6.16 (tt, 1H), 4.44 (d, 2H), 4.41-4.35 (m, 2H) |
| 1A-58 | WHITE SOLID | 7.50 (s, 1H), 7.29-7.23 (m, 1H), 6.82-6.78 (m, 1H), 6.66 (d, 1H), 4.48-4.42 (m, 2H), 4.41 (d, 2H) |
| 1A-59 | WHITE SOLID | 7.51 (s, 1H), 7.23 (t, 1H), 7.13-7.11 (m, 1H), 6.77 (d, 1H), 4.55 (s, 2H), 4.46-4.40 (m, 2H) |

**[Table 5-3]**

| No. | State | 1H-NMR(400 MHz, CDC1₃, σppm) |
|---|---|---|
| 1A-60 | YELLOW OIL | 7.50 (s, 1H), 7.33-7.23 (m, 3H), 4.79 (s, 2H), 4.40 (m, 2H), 1.38 (m, 3H) |
| 1A-61 | COLORLESS OIL | 7.47 (s, 1H), 7.23 (t, 1H), 6.93 (d, 1H), 6.89 (t, 1H), 6.81 (dd, 1H), 4.35 (s, 2H), 3.79 (s, 3H) |
| 1A-62 | - | 7.49 (s, 1H), 7.16 (t, 1H), 7.00 (dd, 1H), 6.87 (dd, 1H), 4.52 (s, 2H), 3.90 (s, 3H) |
| 1A-64 | YELLOW OIL | 7.47 (s, 1H), 7.06 (d, 1H), 6.83 (m, 2H), 4.35 (s, 2H), 3.81 (s, 3H), 2.19 (s, 3H) |
| 1A-66 | PALE YELLOW OIL | 7.49 (s, 1H), 7.27 (d, 1H), 6.94 (d, 1H), 6.77 (dd, 1H), 4.46 (s, 2H), 3.75 (s, 3H) |
| 1A-67 | YELLOW OIL | 7.53 (s, 1H), 7.13 (dd, 1H), 6.85 (t, 1H), 4.51 (d, 2H), 3.87 (s, 3H) |
| 1A-69 | COLORLESS OIL | 7.54 (s, 1H), 7.28 (d, 1H), 6.84 (d, 1H), 4.69 (s, 2H), 3.90 (s, 3H) |
| 1A-70 | YELLOW OIL | 7.49 (s, 1H), 6.93 (m, 1H), 4.35 (d, 2H), 4.03 (s, 3H) |
| 1A-72 | PALE YELLOW OIL | 7.48 (s, 1H), 7.02-6.94 (m, 2H), 6.85 (m, 1H), 4.32 (s, 2H), 4.08 (q, 2H), 1.44 (t, 3H) |
| 1A-73 | YELLOW OIL | 7.48 (s, 1H), 7.28 (d, 1H), 6.91 (d, 1H), 6.85 (dd, 1H), 4.33 (s, 2H), 4.08 (q, 2H), 1.46 (t, 3H) |
| 1A-74 | YELLOW OIL | 7.48 (s, 1H), 6.92 (m, 1H), 6.81-6.77 (m, 2H), 4.30 (s, 2H), 4.00 (m, 2H), 1.41 (m, 3H) |
| 1A-75 | YELLOW OIL | 7.48 (s, 1H), 7.17 (s, 1H), 7.06 (m, 2H), 4.37 (s, 2H), 4.05 (m, 2H), 1.43 (t, 3H) |
| 1A-76 | - | 7.49 (s, 1H), 7.26 (d, 1H), 6.93 (d, 1H), 6.75 (dd, 1H), 4.45 (s, 2H), 3.96 (q, 2H), 1.39 (t, 3H) |
| 1A-77 | PALE YELLOW OIL | 7.49 (s, 1H), 6.98 (m, 1H), 6.81 (dt, 1H), 4.36 (s, 2H), 4.19 (q, 2H), 1.39 (t, 3H) |
| 1A-78 | COLORLESS OIL | 7.64 (s, 1H), 6.89-6.76 (m, 2H), 4.42 (t, 2H), 4.05 (q, 2H), 1.42 (t, 3H) |
| 1A-79 | PALE YELLOW OIL | 7.48 (s, 1H), 6.79-6.72 (m, 2H), 4.29 (s, 2H), 4.09 (m, 2H), 1.44 (t, 3H) |
| 1A-80 | PALE YELLOW OIL | 7.48 (s, 1H), 7.31 (m, 1H), 7.20 (t, 1H), 7.11 (d, 1H), 7.02 (dd, 1H), 6.49 (t, 1H), 4.37 (s, 2H) |
| 1A-81 | PALE YELLOW OIL | 7.48 (s, 1H), 7.34 (t, 1H), 7.28 (m, 1H), 7.21 (m, 1H), 7.12 (m, 1H), 4.38 (s, 2H) |
| 1A-82 | COLORLESS OIL | 7.42 (s, 1H), 7.34 (m, 2H), 7.11 (m, 3H), 7.01 (dd, 2H), 6.95 (m, 1H), 4.28 (s, 2H) |
| 1A-83 | PALE YELLOW OIL | 7.47 (s, 1H), 7.26 (m, 2H), 6.84 (m, 2H), 4.34 (s, 2H), 3.79 (s, 3H) |
| 1A-84 | COLORLESS OIL | - |
| 1A-85 | - | 7.48 (s, 1H), 7.37 (d, 1H), 7.21 (dd, 1H), 6.86 (d, 1H), 4.31 (s, 2H), 3.89 (s, 3H) |
| 1A-86 | WHITE SOLID | 7.52 (s, 1H), 6.46 (m, 2H), 4.35 (s, 2H), 3.78 (s, 3H) |
| 1A-87 | PALE YELLOW OIL | - |

**[Table 5-4]**

| No. | State | 1H-NMR(400 MHz, CDC1₃, σppm) |
|---|---|---|
| 1A-88 | BROWN OIL | 7.49 (s, 1H), 7.26 (d, 1H), 6.92 (d, 1H), 6.71 (dd, 1H), 4.45 (s, 2H), 3.99 (q, 2H), 1.40 (t, 3H) |
| 1A-89 | PALE YELLOW OIL | 7.48 (s, 1H), 7.16 (d, 1H), 6.72 (m, 1H), 6.65 (dd, 1H), 4.36 (s, 2H), 4.00 (m, 2H), 2.37 (s, 3H), 1.40 (m, 3H) |
| 1A-92 | - | 7.47 (s, 1H), 7.36 (d, 1H), 7.17 (dd, 1H), 6.83 (d, 1H), 4.30 (s, 2H), 4.09 (q, 2H), 1.46 (t, 3H) |
| 1A-93 | YELLOW OIL | - |
| 1A-94 | PALE YELLOW OIL | - |
| 1A-95 | PALE YELLOW OIL | - |
| 1A-96 | PALE YELLOW SOLID | - |
| 1A-97 | WHITE SOLID | 7.49 (s, 1H), 7.08 (dd, 1H), 6.66 (dd, 1H), 4.32 (d, 2H), 4.06 (q, 2H), 1.45 (t, 3H) |
| 1A-100 | PALE YELLOW OIL | 7.75 (s, 1H), 6.94-6.88 (m, 2H), 4.29 (s, 2H), 4.18 (q, 2H), 1.37 (t, 3H) |
| 1A-101 | PALE YELLOW OIL | 7.48 (s, 1H), 7.15 (dd, 1H), 7.03 (dd, 1H), 4.29 (s, 2H), 4.17 (m, 2H), 1.42 (m, 3H) |
| 1A-102 | - | 7.48 (s, 1H), 7.28 (s, 2H), 4.28 (s, 2H), 4.08 (q, 2H), 1.45 (t, 3H) |
| 1A-104 | PALE YELLOW OIL | - |
| 1A-105 | PALE YELLOW OIL | 7.47 (s, 1H), 7.34 (m, 2H), 7.06 (d, 2H), 6.49 (t, 1H), 4.36 (s, 2H) |
| 1A-106 | PALE YELLOW OIL | 7.48 (s, 1H), 7.37 (dt, 2H), 7.15 (d, 2H), 4.38 (s, 2H) |
| 1A-107 | YELLOW OIL | - |
| 1A-108 | COLORLESS OIL | 7.52 (s, 1H), 6.60 (d, 2H), 5.14 (s, 2H), 4.36 (s, 2H), 3.47 (s, 3H) |
| 1A-109 | COLORLESS OIL | 7.53 (s, 1H), 7.04 (s, 2H), 5.15 (s, 2H), 4.62 (s, 2H), 3.47 (s, 3H) |
| 1A-110 | - | 7.46 (s, 1H), 7.43-7.31 (m, 5H), 7.27 (m, 2H), 6.91 (dt, 2H), 5.05 (s, 2H), 4.33 (s, 2H) |
| 1A-111 | YELLOW OIL | 7.47 (s, 1H), 6.98 (t, 1H), 6.91 (dd, 1H), 6.86 (dd, 1H), 4.42 (s, 2H), 3.90 (s, 3H), 3.87 (s, 3H) |
| 1A-112 | YELLOW OIL | 7.48 (s, 1H), 7.15 (d, 1H), 7.00 (s, 1H), 6.94 (d, 1H), 4.38 (s, 2H), 2.37 (s, 3H), 2.30 (s, 3H) |
| 1A-113 | YELLOW OIL | 7.48 (s, 1H), 6.88 (d, 1H), 6.77 (m, 2H), 4.38 (s, 2H), 3.81 (s, 3H), 3.73 (s, 3H) |
| 1A-115 | - | 7.49 (s, 1H), 6.57 (s, 2H), 4.33 (s, 2H), 3.84 (s, 6H), 3.83 (s, 3H) |
| 1A-116 | WHITE SOLID | 7.49 (s, 1H), 7.24 (t, 1H), 6.56 (d, 2H), 6.05 (tt, 2H), 4.46 (s, 2H), 4.18 (dt, 4H) |
| 1A-117 | YELLOW OIL | 7.47 (s, 1H), 7.27-7.22 (m, 1H), 6.97-6.92 (m, 2H), 6.52 (t, 1H), 5.32 (dq, 1H), 1.81-1.79(m, 3H) |
| 1A-118 | YELLOW OIL | 7.61 (s, 1H), 7.20 (t, 1H), 7.00 (dd, 1H), 6.78 (d, 1H), 4.57 (s, 2H), 3.79 (s, 3H) |
| 1A-119 | YELLOW OIL | 7.57 (s, 1H), 7.40 (d, 1H), 7.29 (m, 1H), 7.16-7.12 (m, 2H), 6.55 (t, 1H), 4.45 (s, 2H) |
| 1A-120 | PALE YELLOW OIL | 7.61 (s, 1H), 7.27 (m, 1H), 6.95 (m, 2H), 6.52 (t, 1H), 4.45 (s, 2H) |
| 1A-121 | PALE YELLOW OIL | 7.62 (s, 1H), 7.25 (m, 2H), 7.07 (d, 1H), 6.49 (t, 1H), 4.58 (s, 2H) |
| 1A-122 | PALE YELLOW OIL | 7.62 (s, 1H), 7.11 (dt, 1H), 6.95 (dt, 1H), 6.64 (t, 1H), 4.48 (s, 2H) |

**[Table 5-5]**

| No. | State | 1H-NMR(400 MHz, CDC1₃, σppm) |
|---|---|---|
| 1A-123 | COLORLESS OIL | 7.61 (s, 1H), 6.73 (m, 2H), 6.52 (t, 1H), 4.39 (s, 2H) |
| 1A-124 | PALE YELLOW OIL | 7.61 (s, 1H), 7.34 (t, 1H), 6.93 (d, 1H), 6.50 (t, 1H), 4.45 (s, 2H) |
| 1A-125 | YELLOW OIL | 7.62 (s, 1H), 7.32 (m, 1H), 7.08 (d, 1H), 7.03 (t, 1H), 4.46 (d, 2H) |
| 1A-126 | COLORLESS OIL | 7.62 (s, 1H), 7.35 (dd, 1H), 7.29 (d, 1H), 7.19 (m, 1H), 4.59 (s, 2H) |
| 1A-127 | WHITE SOLID | 7.61 (s, 1H), 7.22 (t, 1H), 7.11 (dd, 1H), 6.77 (d, 1H), 4.59 (s, 2H), 4.36 (q, 2H) |
| 1A-128 | COLORLESS OIL | 7.60 (s, 1H), 7.40 (m, 2H), 7.08 (d, 1H), 4.65 (s, 2H), 4.42 (q, 2H) |
| 1A-129 | PALE YELLOW OIL | 7.61 (s, 1H), 7.41 (t , 1H), 7.34 (d, 1H), 7.07 (d, 1H), 6.01 (tt, 1H), 4.64 (s, 2H), 4.22 (m, 2H) |
| 1A-130 | YELLOW OIL | 7.57 (s, 1H), 7.44 (d, 1H), 7.31 (m, 2H), 7.21 (m, 1H), 5.97 (tt, 1H), 4.42 (s, 2H) |
| 1A-131 | COLORLESS OIL | 7.60 (s, 1H), 7.29-7.24 (m, 1H), 6.80 (t, 1H), 6.66 (d, 1H), 6.16 (tt, 1H), 4.45 (d, 2H), 4.35-4.41 (m, 2H) |
| 1A-132 | COLORLESS OIL | - |
| 1A-133 | COLORLESS OIL | 7.97 (s, 1H), 7.15 (dd, 1H), 6.88 (t, 1H), 4.66 (t, 1H), 3.88 (s, 3H) |
| 1A-134 | YELLOW OIL | 7.47 (s, 1H), 7.27 (d, 2H), 7.18 (d, 2H), 4.34 (s, 2H), 2.47 (s, 3H) |
| 1A-135 | - | 7.50 (s, 1H), 7.09 (d, 1H), 6.97 (d, 1H), 4.48 (s, 2H), 2.80 (s, 6H) |
| 1A-136 | PALE YELLOW OIL | 7.50 (s, 1H), 7.24 (m, 1H), 7.10 (d, 1H), 6.86 (t, 1H), 4.55 (s, 2H), 3.77 (s, 2H), 2.79 (s, 3H), 2.05 (s , 1H) |
| 1A-137 | COLORLESS OIL | 7.48-7.40 (m, 2H), 7.67-7.55 (m, 3H), 4.40 (s, 2H) |
| 1A-138 | WHITE SOLID | 7.60 (m, 2H), 7.45 (m, 3H), 4.41 (s, 2H) |
| 1A-139 | YELLOW OIL | 7.49 (s, 1H), 7.35-7.29 (m, 1H), 7.03-6.99 (m, 2H), 4.40 (d, 2H), 4.34 (s, 2H) |
| 1A-140 | COLORLESS OIL | 7.88-7.84 (m, 1H), 7.53 (s, 1H), 7.53-7.48 (m, 1H), 7.36 (t, 1H), 4.94 (s, 2H), 3.37 (q, 2H), 1.32 (t, 3H) |
| 1A-141 | YELLOW OIL | 7.54 (s, 1H), 7.14 (s, 2H), 6.51 (t, 1H), 4.64 (s, 2H) |
| 1A-142 | YELLOW OIL | 7.70 (s, 2H), 6.70 (dd, 2H), 6.50 (dt, 1H), 4.37 (s, 2H) |
| 1A-143 | - | - |
| 1A-144 | YELLOW OIL | 7.49 (s, 1H), 7.11 (m, 1H), 6.65 (m, 2H), 4.42 (s, 2H), 0.95 (m, 9H), 0.24 (m, 6H) |
| 1A-145 | YELLOW OIL | 7.54 (s, 1H), 7.26 (m, 2H), 6.51 (t, 1H), 4.51 (s, 2H) |
| 1A-146 | - | - |
| 1A-147 | COLORLESS OIL | 7.53 (s, 1H), 7.41-7.35 (m, 1H), 7.17-7.11 (m, 2H), 4.49 (d, 2H) |
| 1A-148 | - | 7.70 (d, 1H), 7.30 (m, 2H), 7.23 (d, 2H), 7.10 (d, 1H), 4.43 (s, 2H) |
| 1A-149 | - | 7.73 (d, 1H), 7.28-7.19 (m, 2H), 7.11 (dd, 1H), 6.77 (d, 1H), 4.64 (s, 2H), 4.36 (q, 2H) |

**[Table 6]**

| No. | State | 1H-NMR(400 MHz, CDC1₃, σppm) |
|---|---|---|
| 1B-1 | WHITE SOLID | 7.62 (s, 1H), 7.26 (t, 1H), 7.02 (dd, 1H), 6.78 (d, 1H), 4.65 (m, 2H), 3.76 (s, 3H) |
| 1B-2 | WHITE SOLID | 7.75 (s, 1H), 7.38 (t, 1H), 7.20-7.12 (m, 3H), 6.50 (t, 1H), 4.61-4.29 (m, 2H) |
| 1B-3 | WHITE SOLID | 7.71 (s, 1H), 7.36 (dd, 1H), 7.00-6.95 (m, 2H), 6.52 (t, 1H), 4.59-4.45 (m, 2H) |
| 1B-4 | WHITE SOLID | 7.69 (s, 1H), 7.35-7.30 (m, 2H), 7.11 (d, 1H), 6.52 (t, 1H), 4.67 (s, 2H) |
| 1B-5 | WHITE SOLID | 7.69 (s, 1H), 7.40 (m, 1H), 7.08 (m, 2H), 4.51 (m, 2H) |
| 1B-6 | WHITE SOLID | 7.67 (s, 1H), 7.40-7.34 (m, 2H), 7.21 (d, 1H), 4.66 (s, 2H) |
| 1B-7 | WHITE SOLID | 7.66 (s, 1H), 7.35-7.29 (m, 1H), 6.79 (t, 1H), 6.69 (d, 1H), 6.08 (tt, 1H), 4.53-4.46 (m, 2H), 4.22-4.14 (m, 2H) |
| 1B-8 | WHITE SOLID | 7.68 (s, 1H), 7.49 (t, 1H), 7.41 (d, 1H), 7.12 (d, 1H), 6.16 (tt, 1H), 4.73-4.56 (m, 2H), 4.30-4.21 (m, 2H) |
| 1B-9 | WHITE SOLID | 7.72 (s, 1H), 7.42-7.39 (m, 1H), 7.34-7.29 (m, 3H), 5.96 (t, 1H), 4.50 (d, 1H), 4.27 (d, 1H) |
| 1B-10 | WHITE SOLID | 7.64 (s, 1H), 7.30 (t, 1H), 7.15 (dd, 1H), 6.82 (d, 1H), 4.67 (q, 2H), 4.36 (m, 2H) |
| 1B-11 | WHITE SOLID | 7.86 (s, 1H), 7.49 (m, 2H), 7.15 (d, 1H), 4.74 (d, 1H), 4.57 (d, 1H), 4.44 (m, 2H) |
| 1B-12 | WHITE SOLID | 7.70 (s, 1H), 7.38-7.32 (m, 1H), 6.82 (t, 1H), 6.72 (d, 1H), 6.19 (tt, 1H), 4.52-4.42 (m, 2H), 4.41-4.35 (m, 2H) |
| 1B-13 | WHITE SOLID | 7.66 (s, 1H), 7.35 (dt, 1H), 6.83 (t, 1H), 6.71 (d, 1H), 4.51-4.41 (m, 4H) |
| 1B-14 | WHITE SOLID | 7.64 (s, 1H), 7.31 (t, 1H), 7.16-7.14 (m, 1H), 6.83 (d, 1H), 4.68 (d, 1H), 4.61 (d, 1H), 4.52-4.35 (m, 2H) |
| 1B-15 | WHITE SOLID | 7.61 (s, 1H), 7.31 (t, 1H), 6.58 (d, 2H), 6.04 (tt, 2H), 4.56 (d, 1H), 4.52 (d, 1H), 4.21-4.06 (m, 4H) |
| 1B-16 | COLORLESS OIL | 7.49 (s, 1H), 7.35-7.29 (m, 1H), 6.97-6.89 (m, 2H), 6.44 (t, 1H), 4.66 (m, 1H), 1.88-1.85 (m, 3H) |
| 1B-17 | WHITE SOLID | 7.72 (s, 1H), 7.26 (t, 1H), 7.02 (dd, 1H), 6.78 (d, 1H), 4.65 (m, 2H), 3.74 (s, 3H) |
| 1B-18 | WHITE SOLID | 7.84 (s, 1H), 7.38 (t, 1H), 7.19-7.11 (m, 3H), 6.50 (t, 1H), 4.61-4.29 (m, 2H) |
| 1B-19 | WHITE SOLID | 7.81 (s, 1H), 7.35 (m, 1H), 6.98 (m, 2H), 6.51 (t, 1H), 4.52 (m, 2H) |
| 1B-20 | WHITE SOLID | 7.80 (s, 1H), 7.32 (m, 2H), 7.12 (m, 1H), 6.51 (t, 1H), 4.67 (s, 2H) |
| 1B-21 | WHITE SOLID | 7.79 (s, 1H), 7.40 (m, 1H), 7.11-7.04 (m, 2H), 4.50 (m, 2H) |
| 1B-22 | WHITE SOLID | 7.76 (s, 1H), 7.38 (m, 2H), 7.21 (m, 1H), 4.65 (m, 2H) |
| 1B-23 | WHITE SOLID | 7.82 (s, 1H), 7.43-7.39 (m, 1H), 7.34-7.24 (m, 3H), 5.96 (tt, 1H), 4.49 (d, 1H), 4.26 (d, 1H) |
| 1B-24 | WHITE SOLID | 7.73 (s, 1H), 7.30 (t, 1H), 7.15 (dd, 1H), 6.81 (d, 1H), 4.67 (q, 2H), 4.35 (m, 2H) |
| 1B-25 | WHITE SOLID | 7.76 (s, 1H), 7.49 (m, 2H), 7.15 (d, 1H), 4.73 (d, 1H), 4.58 (d, 1H), 4.44 (m, 2H) |
| 1B-26 | WHITE SOLID | 7.79 (s, 1H), 7.38-7.32 (m, 1H), 6.82 (t, 1H), 6.72 (d, 1H), 6.20 (tt, 1H), 4.51-4.42 (m, 2H), 4.41-4.35 (m, 2H) |
| 1B-27 | YELLOW OIL | 7.73 (s, 1H), 7.43-7.37 (m, 1H), 7.13-7.11 (m, 1H), 7.02 (m, 1H), 4.52 (m, 1H), 4.38 (m, 1H), 4.32 (s, 2H) |
| 1B-28 | COLORLESS OIL | 7.93 (d, 1H), 7.77 (s, 1H), 7.63-7.57 (m, 1H), 7.44 (t, 1H), 5.21 (d, 1H), 4.93 (m, 1H), 3.56-3.39 (m, 2H), 1.34 (t, 3H) |
| 1B-29 | COLORLESS OIL | 7.73 (s, 1H), 7.50-7.45 (m, 1H), 7.21-7.17 (m, 2H), 4.58 (m, 1H), 4.48 (m, 1H) |

Formulation examples will be described next.

### Formulation Example 1 (Emulsion)

Ten parts of each of the compounds of the present invention was dissolved in 45 parts of SOLVESSO 150 and 35 parts of N-methyl pyrrolidone. Ten parts of an emulsifier (Trade name: Sorpol 3005X, Manufacturer: TOHO Chemical Industry Co., Ltd.) was added to the resultant mixture. The mixture was stirred and mixed together, thereby obtaining a 10% emulsion for each compound.

### Formulation Example 2 (Wettable Powder)

Twenty parts of each of the compounds of the present invention was added to a mixture containing 2 parts of sodium lauryl sulfate, 4 parts of sodium ligninsulfonate, 20 parts of fine power of synthetic hydrous silicon oxide, and 54 parts of clay. The mixture was then stirred and mixed together using a juice-mixer, thereby obtaining a 20% wettable powder.

### Formulation Example 3 (Granule Formulation)

Two parts of sodium dodecylbenzene sulfonate, 10 parts of bentonite, and 83 parts of clay were added to 5 parts of each of the compounds of the present invention, and the resultant mixture was stirred and mixed together sufficiently. Following addition of an appropriate amount of water, the mixture was stirred further. The mixture was then formed into granules using a granulator, and the obtained granules were forced-air dried, thereby obtaining a 5% granule formulation.

### Formulation Example 4 (Powder Formulation)

One part of each of the compounds of the present invention was dissolved in acetone. To this product, added were 5 parts of fine power of synthetic hydrous silicon oxide, 0.3 parts of isopropyl acid phosphate (PAP), and 93.7 parts of clay. The resultant mixture was stirred and mixed together using a juice-mixer, and the acetone was evaporated out, thereby obtaining a 1% powder formulation.

### Formulation Example 5 (Flowable Formulation)

Twenty parts of each of the compounds of the present invention, 3 parts of polyoxyethylene tristyrylphenyl ether phosphate triethanolamine, and 20 parts of water containing 0.2 parts of a silicone-based antifoaming agent (Trade name: RHODORSIL®426R, Manufacturer: RHODIA CHIMIE) were mixed together. The resultant mixture was wet-ground by a mill (Dyno Mill, Manufacturer: Willy A Bachofen AG). The wet-ground mixture was mixed with 60 parts of water containing 8 parts of propylene glycol and 0.32 parts of xanthan gum, thereby obtaining a 20% suspension in water.

Next, it will be described below the usefulness of the compounds of the present invention as an active ingredient of a herbicide. The compounds of the present invention are denoted by compound numbers listed in Tables 1 to 3.

### Test Example 1

(#1) Test for Herbicidal Effect in Flooding Treatment: Styrol cups each having an opening diameter of 8 cm (about 52 cm²) were respectively filled with soil (45 ml) which had been passed through a sieve (2 mm). Water (about 115 ml) was mixed with the soil such that the soil was flooded. After the soil stood still for 24 hours, Monochoria vaginalis, Echinochloa crus-galli (L.) P.Beauv. var. formosensis Ohwi, Cyperus difformis (umbrella sedge), and Scirpus juncoides were seeded. Each of the compounds of the present invention was dissolved in acetone, and the obtained product was then diluted with water containing Tween 80®· Each of the resultant chemical solutions was dropped on the entire surface of an associated one of treatment sections, so that each section was treated with 1 ml of the respective chemical solution (a dose of 2 kg/ha). After the treatment, the plants were grown in a glasshouse. On day 21, a survey was conducted on the herbicidal activity by visual observation, and evaluation was made according to the following criteria.
(#2) Test for Herbicidal Effect in Flooding Treatment: Styrol cups each having an opening diameter of 13 cm (about 130 cm²) were respectively filled with muddy soil (500 ml). Water (about 300 ml) was mixed with the soil such that the soil was flooded. After the soil stood still for 24 hours, Echinochloa crus-galli (L.) P.Beauv. var. formosensis Ohwi was seeded. When the Echinochloa crus-galli (L.) P.Beauv. var. formosensis Ohwi grew to reach 2-2.5 leaf stage, chemical solutions were dropped on the entire surface of treatment sections, so that each section is treated with 1 ml of the respective chemical solution (a dose of 0.2 kg/ha). Each of the chemical solutions was prepared by dissolving associated one of the compounds of the present invention in acetone, followed by dilution with water containing Tween 80®. After the treatment, the plants were grown in a glasshouse. On day 21, a survey was conducted on the herbicidal activity by visual observation, and evaluation was made according to the following criteria.

Tables 7-1 to 7-5 and Table 8 show the results.

### [Criteria]

0: Growth inhibition effect is less than 10%.
1: Growth inhibition effect is equal to or greater than 10%, and less than 30%.
2: Growth inhibition effect is equal to or greater than 30%, and less than 50%.
3: Growth inhibition effect equal to or greater than 50%, and less than 70%.
4: Growth inhibition effect is equal to or greater than 70%, and less than 90%.
5: Growth inhibition effect is equal to or greater than 90%, or complete withering is achieved.

### Test plants

ECHDG=Echinochloa crus-galli (L.) P.Beauv. var. formosensis Ohwi, MONVA=Monochoria vaginalis, SCIJU=Scirpus juncoides, CYMIC=Cyperus microiria.

**[Table 7-1]**

| No. | ECHDG #1 | ECHDG (2-2.5L) #2 | MONVA #1 | SCIJU #1 | CYMIC #1 |
|---|---|---|---|---|---|
| 1C-1 | 5 | - | - | 4 | - |
| 1C-2 | 5 | 5 | 5 | 5 | 5 |
| 1C-3 | 5 | 4 | - | 5 | 5 |
| 1C-4 | 5 | 5 | - | 5 | 5 |
| 1C-5 | 5 | 5 | 5 | 5 | 5 |
| 1C-6 | 5 | 5 | 5 | 5 | - |
| 1C-7 | 5 | 5 | 5 | 5 | - |
| 1C-8 | 5 | 5 | 5 | 5 | 5 |
| 1C-9 | 5 | 5 | - | 5 | 5 |
| 1C-10 | 5 | 5 | - | 5 | 5 |
| 1C-11 | 5 | 5 | 5 | 5 | 5 |
| 1C-12 | 5 | 4 | - | 5 | 5 |
| 1C-13 | 5 | 5 | - | 5 | 5 |
| 1C-14 | 5 | 4 | - | 5 | 5 |
| 1C-15 | 5 | 5 | - | 5 | 5 |
| 1C-16 | 5 | 5 | - | 5 | 5 |
| 1C-17 | 5 | 5 | - | 5 | 5 |
| 1C-18 | 5 | 5 | - | 5 | 5 |
| 1C-19 | 5 | 5 | 5 | 5 | 5 |
| 1C-20 | 5 | 5 | - | 5 | 5 |
| 1C-21 | 5 | 5 | 5 | 5 | 5 |
| 1C-22 | 5 | 5 | 5 | 5 | 5 |
| 1C-23 | 5 | 3 | 5 | 5 | 5 |
| 1C-24 | 5 | 5 | - | 5 | 5 |
| 1C-25 | 5 | 5 | 5 | 5 | 5 |
| 1C-26 | 5 | 5 | 5 | 5 | 5 |
| 1C-27 | 5 | 5 | 5 | 5 | 5 |
| 1C-28 | 5 | 5 | 5 | 5 | 5 |
| 1C-29 | 5 | 5 | 5 | 5 | 5 |
| 1C-30 | 5 | 5 | 5 | 5 | 5 |
| 1C-31 | 5 | 5 | 5 | 5 | 5 |
| 1C-32 | 5 | 5 | 5 | 5 | - |
| 1C-33 | 5 | 5 | 5 | 5 | - |
| 1C-34 | 5 | 4 | - | 5 | 5 |
| 1C-35 | 5 | 5 | 5 | 5 | 5 |
| 1C-36 | 5 | 4 | 5 | 5 | 5 |
| 1C-37 | 5 | 5 | - | 5 | - |
| 1C-38 | 5 | 5 | 5 | 5 | 5 |

**[Table 7-2]**

| No. | ECHDG #1 | ECHDG (2-2.5L) #2 | MONVA #1 | SCIJU #1 | CYMIC #1 |
|---|---|---|---|---|---|
| 1C-39 | 5 | 5 | 5 | 5 | 5 |
| 1C-40 | 5 | 5 | 5 | 5 | 5 |
| 1C-41 | 5 | 5 | 5 | 5 | - |
| 1C-42 | 5 | 5 | 5 | 5 | - |
| 1C-43 | 5 | 5 | 5 | 5 | - |
| 1C-44 | 5 | 5 | 5 | 5 | - |
| 1C-45 | 5 | 5 | 5 | 5 | - |
| 1C-46 | 5 | 5 | - | 5 | 5 |
| 1C-47 | 5 | 4 | 5 | 5 | 5 |
| 1C-48 | 5 | 5 | - | 5 | - |
| 1C-49 | 5 | 5 | 5 | 5 | - |
| 1C-50 | 5 | 5 | 5 | 5 | - |
| 1C-51 | 5 | 5 | 5 | 5 | - |
| 1C-52 | 5 | 5 | 5 | 5 | - |
| 1C-53 | 5 | 5 | 5 | 5 | - |
| 1C-54 | 5 | 5 | 5 | 5 | - |
| 1C-55 | 5 | 5 | 5 | 5 | - |
| 1C-56 | 5 | 5 | - | 5 | - |
| 1C-57 | 5 | 5 | - | 5 | - |
| 1C-58 | 5 | 5 | 5 | 5 | - |
| 1C-59 | 5 | 4 | 5 | 5 | - |
| 1C-60 | 5 | 5 | 5 | 5 | - |
| 1C-61 | 5 | 5 | 5 | 5 | - |
| 1C-62 | 5 | 4 | 5 | 5 | - |
| 1C-63 | 5 | 3 | 5 | 5 | - |
| 1C-64 | 5 | - | 5 | 5 | - |
| 1C-65 | 5 | 5 | 5 | 5 | 5 |
| 1C-66 | 5 | 4 | 5 | 5 | 5 |
| 1C-67 | 5 | 5 | - | 5 | 5 |
| 1C-68 | 5 | 4 | - | 5 | 5 |
| 1C-69 | 5 | 4 | 5 | 5 | 5 |
| 1C-70 | 5 | 4 | 5 | 5 | 5 |
| 1C-71 | 5 | 5 | - | 5 | 5 |
| 1C-72 | 5 | 5 | - | 5 | 5 |
| 1C-73 | 5 | 5 | 5 | 5 | - |
| 1C-74 | 5 | 3 | - | 5 | 5 |
| 1C-75 | 5 | 4 | 5 | 5 | 5 |
| 1C-76 | 5 | 4 | - | 5 | 5 |

**[Table 7-3]**

| No. | ECHDG #1 | ECHDG (2-2. 5L) #2 | MONVA #1 | SCIJU #1 | CYMIC #1 |
|---|---|---|---|---|---|
| 1C-77 | 5 | 3 | 5 | 5 | 5 |
| 1C-78 | 5 | 5 | 5 | 5 | 5 |
| 1C-79 | 5 | 2 | 5 | 5 | 5 |
| 1C-80 | 5 | 5 | - | 5 | 5 |
| 1C-81 | 5 | 5 | 5 | 5 | 5 |
| 1C-82 | 5 | 5 | 5 | 5 | 5 |
| 1C-83 | 5 | 4 | 5 | 5 | 5 |
| 1C-84 | 5 | 3 | - | 5 | 5 |
| 1C-85 | 5 | 1 | - | 5 | 5 |
| 1C-86 | 5 | 1 | - | - | - |
| 1C-87 | 5 | 1 | - | 5 | 5 |
| 1C-88 | 5 | 4 | 5 | 5 | 5 |
| 1C-89 | 5 | 3 | - | 5 | 5 |
| 1C-90 | 5 | 5 | - | 5 | 5 |
| 1C-91 | 5 | 5 | 5 | 5 | 5 |
| 1C-92 | 5 | 5 | - | 5 | 5 |
| 1C-93 | 5 | 5 | 5 | 5 | 5 |
| 1C-94 | 5 | 5 | 5 | 5 | 5 |
| 1C-95 | 5 | 3 | - | 5 | 5 |
| 1C-96 | 5 | 4 | - | 5 | 5 |
| 1C-97 | 5 | 3 | - | 5 | 5 |
| 1C-98 | 5 | 1 | 5 | 5 | 5 |
| 1C-99 | 5 | 5 | 5 | 5 | 5 |
| 1C-100 | 5 | 5 | 5 | 5 | 5 |
| 1C-101 | 5 | 5 | 5 | 5 | 5 |
| 1C-102 | 5 | 4 | - | 5 | 5 |
| 1C-103 | 5 | 5 | - | 5 | 5 |
| 1C-104 | 5 | 1 | 5 | 5 | 5 |
| 1C-105 | 5 | 2 | 5 | 5 | 5 |
| 1C-106 | 5 | 3 | - | 5 | 5 |
| 1C-107 | 5 | 4 | - | 5 | 5 |
| 1C-108 | 5 | 5 | - | 5 | 5 |
| 1C-109 | 5 | 4 | - | 5 | 5 |
| 1C-110 | 5 | 5 | 5 | 5 | 5 |
| 1C-111 | 5 | 5 | - | 5 | 5 |
| 1C-112 | 5 | 3 | - | 5 | 5 |
| 1C-113 | 5 | 5 | - | 5 | 5 |
| 1C-114 | 5 | 1 | - | 5 | 5 |

**[Table 7-4]**

| No. | ECHDG #1 | ECHDG (2-2. 5L) #2 | MONVA #1 | SCIJU #1 | CYMIC #1 |
|---|---|---|---|---|---|
| 1C-115 | 5 | 1 | - | 5 | 5 |
| 1C-116 | 5 | 3 | - | 5 | 5 |
| 1C-117 | 5 | 4 | 5 | 5 | 5 |
| 1C-118 | 5 | 4 | - | 5 | 5 |
| 1C-119 | 5 | 4 | 5 | 5 | 5 |
| 1C-120 | 5 | 2 | - | 4 | - |
| 1C-121 | - | - | - | - | - |
| 1C-122 | - | - | - | - | - |
| 1C-123 | - | - | - | - | - |
| 1C-124 | 5 | 4 | - | 5 | 5 |
| 1C-125 | 5 | 5 | - | 5 | 5 |
| 1C-126 | 5 | 3 | - | 5 | 5 |
| 1C-127 | 5 | - | - | 5 | 5 |
| 1C-128 | 5 | 1 | - | 4 | 5 |
| 1C-129 | 5 | - | 5 | 4 | - |
| 1C-130 | 5 | 5 | 5 | 5 | - |
| 1C-131 | 5 | 5 | 5 | 5 | - |
| 1C-132 | 5 | 5 | 5 | 5 | - |
| 1C-133 | 5 | 5 | 5 | 5 | - |
| 1C-134 | 5 | 5 | 5 | 5 | - |
| 1C-135 | 5 | 5 | 5 | 5 | - |
| 1C-136 | 5 | 5 | 5 | 5 | - |
| 1C-137 | 5 | 5 | 5 | 5 | - |
| 1C-138 | 5 | 3 | 5 | 5 | - |
| 1C-139 | - | 1 | 5 | 5 | - |
| 1C-140 | 5 | 5 | - | 5 | - |
| 1C-141 | 5 | 4 | - | 5 | - |
| 1C-142 | 5 | 5 | 5 | 5 | - |
| 1C-143 | 5 | 4 | 5 | 5 | - |
| 1C-144 | - | - | - | - | - |
| 1C-145 | 5 | 5 | 5 | 5 | - |
| 1C-146 | 5 | 5 | 5 | 5 | - |
| 1C-147 | 5 | 5 | 5 | 5 | - |
| 1C-148 | 5 | 5 | 5 | 5 | - |
| 1C-149 | 5 | 4 | 5 | 5 | - |
| 1C-150 | 5 | - | 5 | 5 | - |

**[Table 7-5]**

| No. | ECHDG #1 | ECHDG (2-2. 5L) #2 | MONVA #1 | SCIJU #1 | CYMIC #1 |
|---|---|---|---|---|---|
| 1C-151 | 5 | 5 | - | 5 | - |
| 1C-152 | 5 | 5 | - | 5 | - |
| 1C-153 | 5 | 5 | 5 | 5 | - |
| 1C-154 | 5 | 5 | 5 | 5 | - |
| 1C-155 | 5 | 5 | 5 | 5 | - |
| 1C-156 | 5 | 5 | 5 | 5 | - |
| 1C-157 | 5 | 5 | 5 | 5 | - |
| 1C-158 | 5 | 5 | - | 5 | - |
| 1C-159 | 5 | 5 | - | 5 | - |
| 1C-160 | 5 | 5 | 5 | 5 | - |
| 1C-161 | 5 | 5 | - | 5 | - |
| 1C-162 | 5 | 5 | 5 | 5 | - |
| 1C-163 | 5 | - | - | 5 | - |
| 1C-164 | 5 | 5 | 5 | 5 | - |
| 1C-165 | 5 | 3 | 5 | 5 | - |
| 1C-166 | - | - | - | - | - |
| 1C-167 | 5 | - | 5 | 5 | - |
| 1C-168 | 5 | - | 5 | 5 | - |
| 1C-169 | 5 | - | 5 | 5 | - |
| 1C-170 | 5 | 5 | - | 5 | 5 |
| 1C-171 | 5 | 3 | - | 5 | 5 |
| 1C-172 | 5 | 2 | - | 5 | 5 |
| 1C-173 | - | - | - | 4 | - |
| 1C-174 | 5 | 4 | - | 5 | - |
| 1C-175 | 5 | 5 | - | 5 | - |
| 1C-176 | 5 | 5 | - | 5 | - |
| 1C-177 | - | - | - | - | - |
| 1C-178 | 5 | - | - | 4 | - |
| 1C-179 | 5 | 5 | - | 5 | - |
| 1C-180 | 5 | 5 | - | 5 | - |
| 1C-181 | 5 | - | - | 4 | - |
| 1C-182 | 5 | - | - | 3 | - |
| 1C-183 | 5 | 3 | - | 5 | - |
| 1C-184 | - | - | - | - | - |
| 1C-185 | 5 | 5 | - | 5 | - |

**[Table 8]**

| No. | ECHDG #1 | ECHDG (2-2.5L) #2 | MONVA #1 | SCIJU #1 |
|---|---|---|---|---|
| 1B-1 | 5 | 4 | 3 | 4 |
| 1B-2 | 5 | 5 | 5 | 5 |
| 1B-3 | 5 | 5 | 5 | 5 |
| 1B-4 | 5 | 5 | - | 5 |
| 1B-5 | 5 | 5 | - | 5 |
| 1B-6 | 5 | 5 | - | 5 |
| 1B-7 | 5 | - | 5 | 5 |
| 1B-8 | 5 | 5 | 5 | 5 |
| 1B-9 | - | - | - | - |
| 1B-10 | 5 | 5 | 5 | 5 |
| 1B-11 | 5 | 5 | - | 5 |
| 1B-12 | 5 | 5 | 5 | 5 |
| 1B-13 | 5 | 2 | 5 | 5 |
| 1B-14 | 5 | - | 5 | 5 |
| 1B-15 | 3 | - | 5 | 5 |
| 1B-16 | 5 | 3 | 5 | 5 |
| 1B-17 | 5 | 5 | 3 | 5 |
| 1B-18 | 5 | 5 | - | 5 |
| 1B-19 | 5 | 5 | 5 | 5 |
| 1B-20 | 5 | 5 | 5 | 5 |
| 1B-21 | 5 | 5 | - | 5 |
| 1B-22 | - | 5 | - | 5 |
| 1B-23 | 5 | 4 | - | - |
| 1B-24 | 5 | 5 | 5 | 5 |
| 1B-25 | 5 | 5 | - | 5 |
| 1B-26 | 5 | 4 | 5 | 5 |
| 1B-27 | - | - | - | - |
| 1B-28 | 5 | - | - | 4 |
| 1B-29 | 5 | 5 | - | 5 |

### Test Example 2

The herbicidal activity of the following comparative compounds A and B (the compounds disclosed Japanese Unexamined Patent Publication No. 2003-096059) were evaluated in a manner pursuant to Test Example 1 (#2).

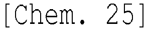

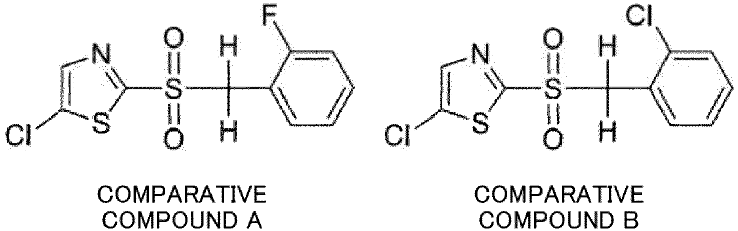

### <Test Results>

The results show that the comparative compound A had a growth inhibition effect of equal to or greater than 10% and less than 30%, and the comparative compound B had a growth inhibition effect of equal to or greater than 30% and less than 50%.

By contrast, the compounds of the present invention selected from Tables 7-1 to 7-5, i.e. the compounds 1C-6, 1C-16, 1C-19, 1C-20, 1C-21, 1C-33, 1C-38, 1C-39, 1C-41, 1C-42, 1C-48, 1C-49, 1C-50, 1C-51, 1C-52, 1C-54, 1C-55, 1C-56, 1C-71, 1C-91, 1C-131, 1C-132, 1C-133, 1C-134, 1C-135, 1C-140, 1C-151, 1C-152, 1C-153, 1C-154, 1C-158, 1C-159, 1C-160, 1C-162, 1C-163, 1C-175, 1C-176, 1C-179, 1C-180, and 1C-185 had a growth inhibition effect of equal to or greater than 90%, or achieved complete withering.

### Test Example 3

(#3) Test for Herbicidal Effect in Soil Treatment: Styrol cups each having an opening diameter of 8 cm (about 52 cm²) were respectively filled with soil (45 ml) which had been passed through a sieve (2 mm). Water (about 115 ml) was added. Thereafter, Digitaria ciliaris was seeded. Each of the compounds of the present invention was dissolved in acetone, and the obtained product was then diluted with water containing Tween 80®. Each of the resultant chemical solutions was dropped on the entire surface of an associated one of treatment sections, so that each section was treated with 1 ml of the respective chemical solution (a dose of 2 kg/ha). After the treatment, the plants were grown in a glasshouse. On day 21, a survey was conducted on the herbicidal activity by visual observation, and evaluation was made according to the following criteria.

The herbicidal activity of the following comparative compounds A and B (the compounds disclosed Japanese Unexamined Patent Publication No. 2003-096059) were evaluated.

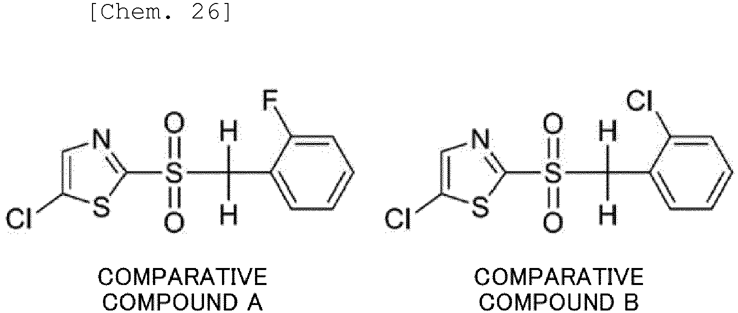

### <Test Results>

The results show that the comparative compounds A and B had a growth inhibition effect of equal to or greater than 30% and less than 50%.

By contrast, the following compounds of the present invention had a growth inhibition effect of equal to or greater than 50%: the compounds 1C-2, 1C-5, 1C-8, 1C-9, 1C-12, 1C-14, 1C-15, 1C-19, 1C-20, 1C-21, 1C-27, 1C-29, 1C-31, 1C-33, 1C-36, 1C-37, 1C-38, 1C-39, 1C-41, 1C-42, 1C-43, 1C-44, 1C-46, 1C-48, 1C-49, 1C-50, 1C-51, 1C-52, 1C-53, 1C-54, 1C-55, 1C-56, 1C-57, 1C-58, 1C-59, 1C-60, 1C-61, 1C-62, 1C-63, 1C-64, 1C-71, 1C-75, 1C-76, 1C-81, 1C-82, 1C-83, 1C-95, 1C-99, 1C-101, 1C-102, 1C-103, 1C-104, 1C-108, 1C-119, 1C-127, 1C-129, 1C-138, 1C-140, 1C-141, 1C-142, 1C-143, 1C-145, 1C-146, 1C-147, 1C-148, 1C-149, 1C-150, 1C-152, 1C-153, 1C-160, 1C-161, 1C-162, 1C-164, 1C-165, 1C-170, 1C-171, 1C-172, 1C-175, 1C-178, 1C-179, 1C-180, 1C-185, 1A-49, 1A-50, 1A-57, 1A-58, 1A-59, 1A-117, 1A-140, 1A-147, 1B-3, 1B-5, 1B-7, 1B-10, 1B-11, 1B-12, 1B-13, 1B-14, 1B-16 and 1B-29.

Among them, the following compounds had a growth inhibition effect of equal to or greater than 90%, or achieved complete withering: 1C-2, 1C-5, 1C-8, 1C-12, 1C-14, 1C-19, 1C-20, 1C-21, 1C-27, 1C-31, 1C-33, 1C-36, 1C-37, 1C-38, 1C-41, 1C-42, 1C-43, 1C-44, 1C-46, 1C-48, 1C-49, 1C-50, 1C-51, 1C-52, 1C-53, 1C-54, 1C-55, 1C-56, 1C-57, 1C-60, 1C-61, 1C-81, 1C-83, 1C-102, 1C-108, 1C-140, 1C-141, 1C-142, 1C-143, 1C-146, 1C-148, 1C-149, 1C-152, 1C-160, 1C-162, 1C-170, 1C-175, 1C-179, 1A-49, 1A-57, 1A-117, 1A-140, 1A-147, 1B-3, 1B-5, 1B-7, 1B-10, 1B-11, 1B-12, 1B-13, 1B-14, 1B-16 and 1B-29.

### CROSS-REFERENCE TO RELATED APPLICATION

This application claims priority to Japanese Patent Application No. 2017-034231 filed on February 26, 2017, the entire disclosure of which is incorporated by reference herein.

## Claims

1. A thiazole compound represented by general formula (1) : 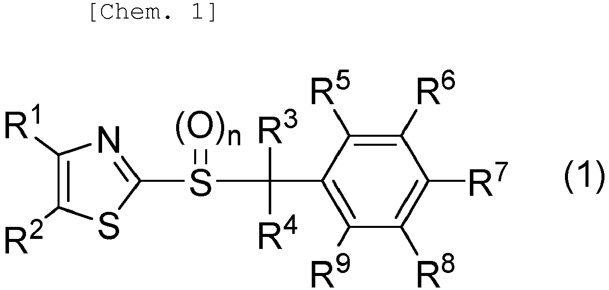 or a salt thereof,
wherein R¹ and R² are identical or different and each represent a hydrogen atom, a halogen atom, or an alkyl group which may have a substituent,
R³ and R⁴ are identical or different and each represent a hydrogen atom, a deuterium atom, a halogen atom, an alkyl group which may have a substituent, a COR¹⁰ group, or a COOR¹¹ group,
R⁵, R⁶, R⁷, R⁸ and R⁹ are identical or different and each represent a hydrogen atom, a halogen atom, a hydroxyl group, an OCOR¹² group, a COR¹³ group, a silyl group which may have a substituent, a silyloxy group which may have a substituent, a nitro group, a cyano group, an amino group which may have a substituent, an alkyl group which may have a substituent, an alkoxy group which may have a substituent, an aryloxy group which may have a substituent, a substituted sulfide group, a substituted sulfinyl group, a substituted sulfonyl group, or an OSO₂R¹⁴ group,
at least one of R⁵, R⁶, R⁷, R⁸ or R⁹ represents a hydroxyl group, an OCOR¹² group, a COR¹³ group, a silyl group which may have a substituent, a silyloxy group which may have a substituent, a cyano group, an amino group which may have a substituent, an alkoxy group which may have a substituent, an aryloxy group which may have a substituent, a substituted sulfide group, a substituted sulfinyl group, a substituted sulfonyl group, or an OSO₂R¹⁴ group,
R¹⁰, R¹¹, R¹², R¹³, and R¹⁴ are identical or different and each represent a hydrogen atom, an alkyl group which may have a substituent, or an aryl group which may have a substituent, and
n represents 0, 1, or 2.

2. The thiazole compound or a salt thereof according to claim 1, wherein
R¹ is a hydrogen atom, a halogen atom, or an unsubstituted alkyl group.

3. The thiazole compound or a salt thereof according to claim 1 or 2, wherein
R² is a halogen atom or a haloalkyl group.

4. The thiazole compound or a salt thereof according to any one of claims 1 to 3, wherein
R³ and R⁴ are identical or different and are a hydrogen atom, a deuterium atom, a halogen atom, an unsubstituted alkyl group, a COCH₃ group, or a COOCH₃ group.

5. The thiazole compound or a salt thereof according to any one of claims 1 to 4, wherein
out of R⁵, R⁶, R⁷, R⁸ and R⁹, at least one of R⁵ or R⁹ is a hydroxyl group, an OCOR¹² group, a silyl group which may have a substituent, a silyloxy group which may have a substituent, a cyano group, an amino group which may have a substituent, an alkoxy group which may have a substituent, an aryloxy group which may have a substituent, a substituted sulfonyl group, or an OSO₂R¹⁴ group.

6. The thiazole compound or a salt thereof according to any one of claims 1 to 5, wherein
at least one of R⁵ or R⁹ is an unsubstituted alkoxy group or a haloalkoxy group.

7. The thiazole compound or a salt thereof according to any one of claims 1 to 6, wherein
R⁷ is a hydrogen atom, an unsubstituted alkoxy group, or a haloalkoxy group.

8. The thiazole compound or a salt thereof according to any one of claims 1 to 7, wherein
R⁷ is an unsubstituted alkoxy group or a haloalkoxy group.

9. The thiazole compound or a salt thereof according to any one of claims 1 to 7, wherein
R⁷ is a hydrogen atom.

10. A herbicide containing the thiazole compound or a salt thereof according to any one of claims 1 to 9.
